# EUROPEAN PATENT APPLICATION

(11) **EP 4 847 020 A1**
(43) Date of publication of application: **02.12.2026**
(21) Application number: 25744565.0
(22) Date of filing: 21.01.2025
(51) Int. Cl.: A61K 31/216, A61K 31/194, A61K 31/23, A61K 31/423, A61K 31/5377, A61K 45/00, A61P 9/04

(54) **USE OF SMALL MOLECULE COMPOUND IN TREATMENT OF CARDIOVASCULAR DISEASES**

(30) Priority: 25.01.2024 CN 202410109124
(71) Applicant: Beijing Baheal Wisart Medical Research Co., Ltd, Beijing 102300 (CN)
(72) Inventor: WANG, Li, Beijing 100037 (CN); LI, Zheng, Beijing 100037 (CN); CHEN, Ying, Beijing 100037 (CN); HUANG, Yingbo, Beijing 100037 (CN); LI, Dandan, Beijing 100037 (CN)
(74) Representative: Ghirardi, Valeria
(86) International application number: PCT/CN2025/073563
(87) International publication number: WO 2025/157124

(57) **Abstract**

The present invention relates to the use of a small molecule compound in the treatment of cardiovascular diseases. Specifically, provided is the use of a compound as shown in formula I or a stereoisomer, prodrug, crystal form, pharmaceutically acceptable salt, pharmaceutically acceptable ester or pharmaceutically acceptable solvate thereof in the preparation of a drug for treating and/or preventing heart failure with preserved ejection fraction,

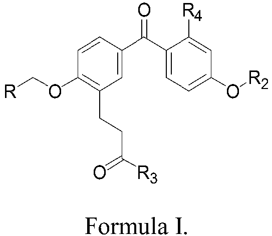

Figure 6 is designated as the abstract drawing.

## Description

### Technical Field

The present invention relates to the field of medicine, specifically to the use of small molecule compounds in the treatment of cardiovascular diseases.

### Background Art

Cardiovascular disease represents one of the significant challenges in medicine. Heart failure (HF) is a major cause of death in patients with cardiovascular disease and a significant clinical challenge. Heart failure includes heart failure with reduced ejection fraction (HFrEF) and heart failure with preserved ejection fraction (HFpEF).

Currently, HFpEF accounts for approximately 50% of all heart failure patients, and its prevalence is growing at an astonishing rate. It is also a leading cause of the rising mortality rate of cardiovascular diseases. HFpEF is a complex condition involving multi-organ dysfunction, where the heart, lungs, kidneys, skeleton, immune system, inflammation, metabolism, and other components collectively contribute to the symptoms and outcomes. It is often accompanied by conditions such as obesity, hypertension, cardiac hypertrophy, diabetes, or atrial fibrillation. HFpEF is a syndrome with high morbidity and mortality. Clinical statistics show a mortality rate of 35% due to HF, with HFpEF accounting for 57% of these deaths. However, to date, very few drug therapies or medical devices have been proven to alter the disease progression and prognosis of HFpEF patients. There is an urgent need in the art to develop effective drugs and/or treatment methods for HFpEF.

*Jun* is a transcription factor, a member of the AP1 family, possessing chromatin-binding activity and transcription cis-regulatory region-binding activity. It is involved in processes including animal organ development, protein phosphorylation, and cell proliferation. A *Jun* inhibitor refers to an inhibitor that can suppress the expression of the *Jun* gene, reduce the binding activity of *Jun* to DNA, reduce the level of the *Jun* gene expression product, or prevent or block the signal transduction of *Jun.* Studies have shown that *Jun* inhibitors have therapeutic effects in animal models of endometriosis, breast cancer, sepsis, etc.

### Contents of the Invention

The inventors of the present invention have discovered for the first time that using a *Jun* inhibitor to inhibit the high expression of *Jun* can prevent and treat HFpEF. Based on this, further research by the inventors revealed that the compounds of the present invention have the activity of inhibiting *Jun* binding to DNA and inhibiting *Jun* transcription, can prevent and treat HFpEF, and have broad application prospects.

Therefore, in a first aspect, the present invention provides the use of a compound represented by Formula I, or a stereoisomer, prodrug, crystal form, pharmaceutically acceptable salt, pharmaceutically acceptable ester, or pharmaceutically acceptable solvate thereof in the manufacture of a medicament for treating and/or preventing heart failure with preserved ejection fraction,
or, provides a compound represented by Formula I, or a stereoisomer, prodrug, crystal form, pharmaceutically acceptable salt, pharmaceutically acceptable ester, or pharmaceutically acceptable solvate thereof, for use in treating and/or preventing heart failure with preserved ejection fraction,
or, provides a method for treating and/or preventing heart failure with preserved ejection fraction, comprising: administering to a subject in need thereof an effective amount of a compound represented by Formula I, or a stereoisomer, prodrug, crystal form, pharmaceutically acceptable salt, pharmaceutically acceptable ester, or pharmaceutically acceptable solvate thereof, 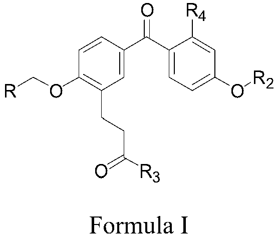
wherein:
   R is selected from the group consisting of C6-C10 aryl, and 6- to 10-membered heteroaryl, wherein the C6-C10 aryl, and 6- to 10-membered heteroaryl are each independently optionally substituted with R¹ and/or R⁵;
   preferably, R is selected from the group consisting of C6-C10 aryl, and 6- to 10-membered heteroaryl, wherein the C6-C10 aryl, and 6- to 10-membered heteroaryl are each independently optionally substituted with R¹ or R¹ and R⁵;
   more preferably, R is selected from the group consisting of C6-C10 aryl, and 6- to 10-membered heteroaryl, wherein the C6-C10 aryl, and 6- to 10-membered heteroaryl are each independently optionally substituted with R¹;
   R¹ is selected from the group consisting of hydrogen, C1-C6 alkyl, -COOH, -C(=O)O-C1-C6 alkyl, -(CH₂)ₘ-O-C1-C6 alkyl, and -NR^{c}R^{d};
      R^{c}, and R^{d} are each independently selected from the group consisting of hydrogen, C1-C6 alkyl, and -(CH₂)ₘ-O-C1-C6 alkyl;
      m is selected from the group consisting of 0, 1, 2, 3, 4, 5, and 6;
      preferably, m is selected from the group consisting of 0, and 1;
      more preferably, m is 1;
   R² is selected from the group consisting of -O-(CH₂)ₙ-R^{2'};
      n is selected from the group consisting of 0, 1, 2, 3, 4, 5, and 6;
      preferably, n is selected from the group consisting of 0, and 1;
      R^{2'} is selected from the group consisting of C1-C6 alkyl, C3-C8 cycloalkyl, C6-C10 aryl, and 5- to 6-membered heteroaryl;
   R³ is selected from the group consisting of hydroxy, and -O-C1-C6 alkyl, wherein the C1-C6 alkyl is optionally substituted with 5- to 6-membered heterocyclyl;
   preferably, R³ is selected from the group consisting of hydroxy, and -O-C1-C6 alkyl, wherein the C1-C6 alkyl is optionally substituted with 6-membered heterocyclyl;
   more preferably, R³ is selected from the group consisting of hydroxy, and -O-C1-C6 alkyl, wherein the C1-C6 alkyl is optionally substituted with morpholinyl;
   most preferably, R³ is selected from the group consisting of hydroxy, and -O-C1-C6 alkyl;
   R⁴ is selected from the group consisting of hydroxy, cyano, nitro, carboxyl, mercapto, halogen, C1-C6 alkyl, -NR^{a}R^{b}, -CONR^{a}R^{b}, C6-C10 aryl, -O-C6-C10 aryl, -C(=O)-C1-C6 alkyl, -CH₂-C6-C10 aryl, C1-C6 alkoxy, C2-C6 alkenyl, C3-C8 cycloalkyl, -C(=O)O-C1-C6 alkyl, -C(=O)O-C6-C10 aryl, C1-C6 alkylthio, -S(=O)-C1-C6 alkyl, -S(=O)₂-C1-C6 alkyl, and 5- to 6-membered heteroaryl;
      R^{a}, and R^{d} are each independently selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
   R⁵ is selected from the group consisting of hydrogen, C1-C6 alkyl, C1-C6 alkoxy, and C1-C6 alkylthio;
   and, the compound represented by Formula I does not include

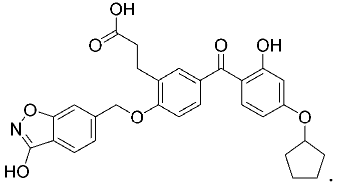

In some embodiments, R is selected from the group consisting of phenyl, benzimidazolyl, and 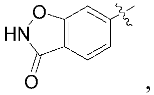 wherein the phenyl, benzimidazolyl, and 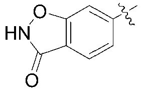 are each independently optionally substituted with R¹ and/or R⁵;
preferably, R is selected from the group consisting of phenyl, benzimidazolyl, and 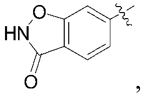 wherein the phenyl, benzimidazolyl, and 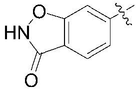 are each independently optionally substituted with R¹ or R¹ and R⁵;
more preferably, R is selected from the group consisting of phenyl, benzimidazolyl, and 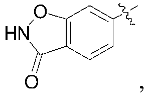 wherein the phenyl, benzimidazolyl, and 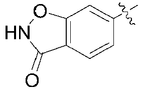 are each independently optionally substituted with R¹;
or preferably, R is selected from the group consisting of phenyl, 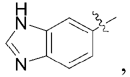 and 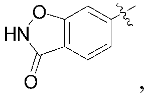 wherein the phenyl, 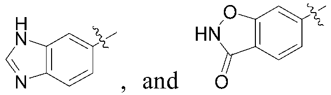 are each independently optionally substituted with R¹ and/or R⁵;
or more preferably, R is selected from the group consisting of phenyl, 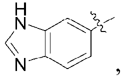 and 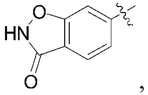 wherein the phenyl, 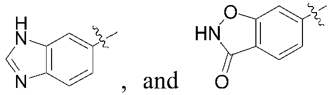 are each independently optionally substituted with R¹ or R¹ and R⁵;
or further preferably, R is selected from the group consisting of phenyl, 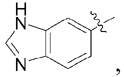 and 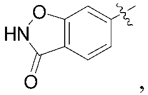 wherein the phenyl, 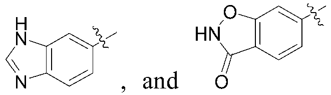 are each independently optionally substituted with R¹;
or preferably, R is selected from the group consisting of 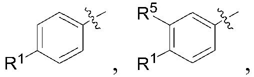 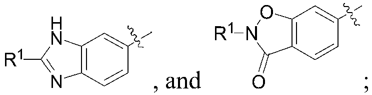
or more preferably, R is selected from the group consisting of 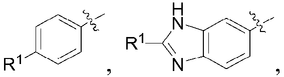 and 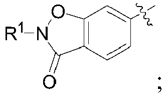
preferably, R¹ is selected from the group consisting of hydrogen, -COOH, -C(=O)O-C1-C6 alkyl, -(CH₂)ₘ-O-C1-C6 alkyl, and -NR^{c}R^{d};
more preferably, R¹ is selected from the group consisting of hydrogen, -COOH, -C(=O)O-C1-C6 alkyl, and -(CH₂)ₘ-O-C1-C6 alkyl;
   preferably, R^{c}, and R^{d} are each independently selected from the group consisting of hydrogen, and -(CH₂)ₘ-O-C1-C6 alkyl;
   more preferably, one of R^{c} and R^{d} is hydrogen, and the other is selected from the group consisting of -(CH₂)ₘ-O-C1-C6 alkyl;
further preferably, R¹ is selected from the group consisting of hydrogen, -COOH, -COOCH₃, -COOCH₂CH₃, -COO(CH₂)₂CH₃, -COO(CH₂)₃CH₃, -COO(CH₂)₄CH₃, -COO(CH₂)₅CH₃, and 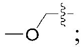
still further preferably, R¹ is selected from the group consisting of hydrogen, -COOH, -COOCH₃, -COOCH₂CH₃, and 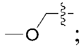
most preferably, R¹ is selected from the group consisting of hydrogen, -COOH, -COOCH₂CH₃, and 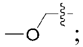
preferably, R⁵ is selected from the group consisting of hydrogen, C1-C6 alkyl, and C1-C6 alkoxy;
more preferably, R⁵ is selected from the group consisting of C1-C6 alkoxy;
most preferably, R⁵ is methoxy.

In some embodiments, R^{2'} is selected from the group consisting of C1-C6 alkyl, C3-C8 cycloalkyl, phenyl, pyrazinyl (e.g., 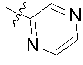 ), and furanyl (e.g., 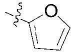 );
preferably, R^{2'} is selected from the group consisting of cyclopropyl, cyclobutyl, cyclopentyl, cyclohexyl, cycloheptyl, 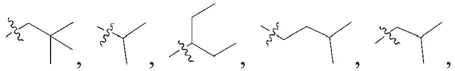 phenyl, pyrazinyl (e.g., 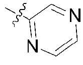 ), and furanyl (e.g., 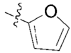 );
or, R^{2'} is selected from the group consisting of C1-C6 alkyl, C3-C8 cycloalkyl, and C6-C10 aryl;
or preferably, R^{2'} is selected from the group consisting of C1-C6 alkyl, C3-C8 cycloalkyl, and phenyl;
or more preferably, R^{2'} is selected from the group consisting of cyclopentyl, cyclohexyl, 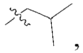 and phenyl;
or more preferably, R^{2'} is selected from the group consisting of cyclopentyl, 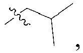 and phenyl;
preferably, R² is selected from the group consisting of -O-C1-C6 alkyl, -O-C3-C8 cycloalkyl, -O-CH₂-C6-C10 aryl, and -O-CH₂-5- to 6-membered heteroaryl;
more preferably, R² is selected from the group consisting of -O-C1-C6 alkyl, -O-C3-C8 cycloalkyl, -O-CH₂-phenyl, and -O-CH₂-5- to 6-membered heteroaryl, wherein the 5- to 6-membered heteroaryl is selected from the group consisting of pyrazinyl (e.g., 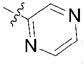 ), and furanyl (e.g., 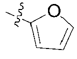 );
most preferably, R² is selected from the group consisting of 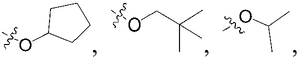 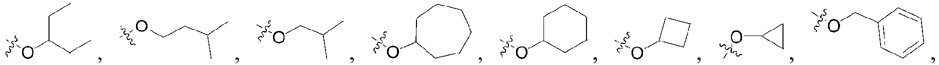 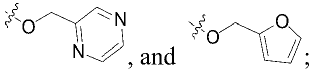
or preferably, R² is selected from the group consisting of -O-C1-C6 alkyl, -O-C3-C8 cycloalkyl, and -O-CH₂-C6-C10 aryl;
or more preferably, R² is selected from the group consisting of -O-C1-C6 alkyl, -O-C3-C8 cycloalkyl, and -O-CH₂-phenyl;
or further preferably, R² is selected from the group consisting of 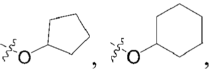 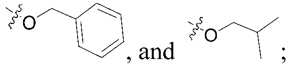
or most preferably, R² is selected from the group consisting of 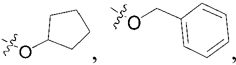 and

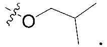

In some embodiments, R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, -O(CH₂)₅CH₃, and 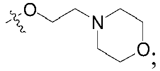
preferably, R³ is selected from the group consisting of hydroxy, -OCH₃, -OCH₂CH₃, -OCH(CH₃)CH₃, -O(CH₂)₂CH₃, and 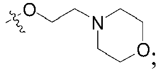
or preferably, R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, and -O(CH₂)₅CH₃;
or more preferably, R³ is selected from the group consisting of hydroxy, -OCH₃, -OCH₂CH₃, -O(CH₂)₂CH₃, -O(CH₂)₃CH₃, -O(CH₂)₄CH₃, and -O(CH₂)₅CH₃;
or further preferably, R³ is selected from the group consisting of hydroxy, -OCH₃, -OCH₂CH₃, -OCH(CH₃)CH₃, and -O(CH₂)₂CH₃;
or most preferably, R³ is selected from the group consisting of hydroxy, -OCH₃, and -OCH₂CH₃.

In some embodiments, R⁴ is selected from the group consisting of hydroxy, cyano, nitro, carboxyl, mercapto, halogen, C1-C6 alkyl, -NR^{a}R^{b}, -CONR^{a}R^{b}, phenyl, phenoxy, -C(=O)-C1-C6 alkyl, benzyl, C1-C6 alkoxy, C2-C6 alkenyl, C3-C8 cycloalkyl, -C(=O)O-C1-C6 alkyl, -C(=O)O-phenyl, C1-C6 alkylthio, -S(=O)-C1-C6 alkyl, -S(=O)₂-C1-C6 alkyl, and thienyl (e.g., 2-thienyl);
preferably, R^{a}, and R^{b} are each independently selected from the group consisting of hydrogen, methyl, 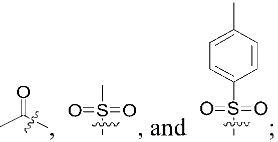
or preferably, one of R^{a} and R^{b} is selected from the group consisting of hydrogen, and C1-C6 alkyl, and the other is selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
or more preferably, one of R^{a} and R^{b} is selected from the group consisting of hydrogen, and methyl, and the other is selected from the group consisting of hydrogen, methyl,

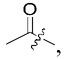

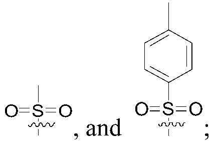

preferably, R⁴ is selected from the group consisting of hydroxy, cyano, nitro, carboxyl, -NH₂, -SH, -CONH₂, methyl, phenyl, benzyl, methoxy, phenoxy, acetyl, -F, -Cl, -Br, -CH=CH₂, cyclopentyl, -COOMe, -COOPh, -SMe, -SOMe, -SO₂Me, -NMe₂, -NHAc, -NHMs, -NHTs, and 2-thienyl;
more preferably, R⁴ is hydroxy.

In some embodiments, the structural formula of the compound is as shown in Formula I-1 or Formula I-1', preferably, the structural formula of the compound is as shown in Formula I-1, 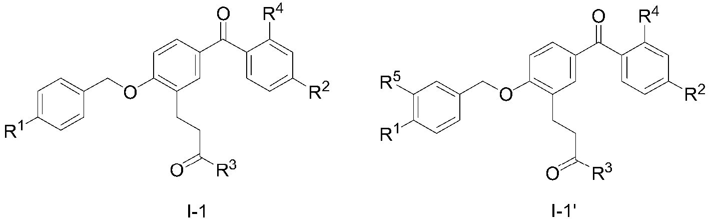 wherein:
R¹ is selected from the group consisting of -COOH, and -C(=O)O-C1-C6 alkyl;
preferably, R¹ is selected from the group consisting of -COOH, -COOCH₃, -COOCH₂CH₃, -COO(CH₂)₂CH₃, -COO(CH₂)₃CH₃, -COO(CH₂)₄CH₃, and -COO(CH₂)₅CH₃;
more preferably, R¹ is selected from the group consisting of -COOH, -COOCH₃, and -COOCH₂CH₃;

most preferably, R¹ is selected from the group consisting of -COOH, and -COOCH₂CH₃;
R² is selected from the group consisting of -O-(CH₂)ₙ-R^{2'};
   n is selected from the group consisting of 0, 1, 2, 3, 4, 5, and 6;
   preferably, n is selected from the group consisting of 0, and 1;
   R^{2'} is selected from the group consisting of C1-C6 alkyl, C3-C8 cycloalkyl, C6-C10 aryl, and 5- to 6-membered heteroaryl;
   preferably, R^{2'} is selected from the group consisting of C1-C6 alkyl, C3-C8 cycloalkyl, phenyl, pyrazinyl (e.g., 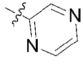 ), and furanyl (e.g., 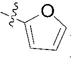 );
   more preferably, R^{2'} is selected from the group consisting of cyclopropyl, cyclobutyl, cyclopentyl, cyclohexyl, cycloheptyl, 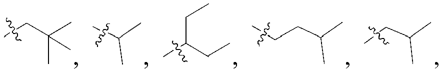 phenyl, pyrazinyl (e.g., 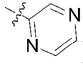 ), and furanyl (e.g., 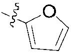 );
   or preferably, R^{2'} is selected from the group consisting of C1-C6 alkyl, C3-C8 cycloalkyl, and C6-C10 aryl;
   or more preferably, R^{2'} is selected from the group consisting of C1-C6 alkyl, C3-C8 cycloalkyl, and phenyl;
   or most preferably, R^{2'} is selected from the group consisting of cyclopentyl, 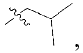 and phenyl;
preferably, R² is selected from the group consisting of -O-C1-C6 alkyl, -O-C3-C8 cycloalkyl, -O-CH₂-C6-C10 aryl, and -O-CH₂-5- to 6-membered heteroaryl;
more preferably, R² is selected from the group consisting of -O-C1-C6 alkyl, -O-C3-C8 cycloalkyl, -O-CH₂-phenyl, and -O-CH₂-5- to 6-membered heteroaryl, wherein the 5- to 6-membered heteroaryl is selected from the group consisting of pyrazinyl (e.g., 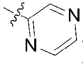 ), and furanyl (e.g., 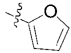 );
most preferably, R² is selected from the group consisting of 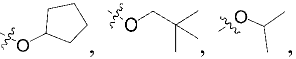 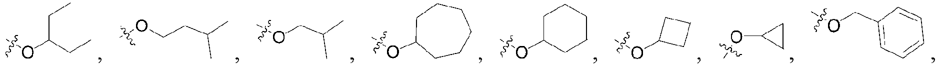 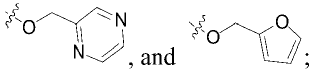
or preferably, R² is selected from the group consisting of -O-C1-C6 alkyl, -O-C3-C8 cycloalkyl, and -O-CH₂-C6-C10 aryl;
or more preferably, R² is selected from the group consisting of -O-C1-C6 alkyl, -O-C3-C8 cycloalkyl, and -O-CH₂-phenyl;
or most preferably, R² is selected from the group consisting of 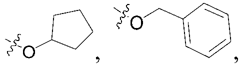 and 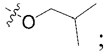
R³ is selected from the group consisting of hydroxy, and -O-C1-C6 alkyl;
preferably, R³ is selected from the group consisting of hydroxy, -OCH₃, -OCH₂CH₃, and -O(CH₂)₂CH₃;
more preferably, R³ is hydroxy;
R⁴ is selected from the group consisting of hydroxy, cyano, nitro, carboxyl, mercapto, halogen, C1-C6 alkyl, -NR^{a}R^{b}, -CONR^{a}R^{b}, C6-C10 aryl, -O-C6-C10 aryl, -C(=O)-C1-C6 alkyl, -CH₂-C6-C10 aryl, C1-C6 alkoxy, C2-C6 alkenyl, C3-C8 cycloalkyl, -C(=O)O-C1-C6 alkyl, -C(=O)O-C6-C10 aryl, C1-C6 alkylthio, -S(=O)-C1-C6 alkyl, -S(=O)₂-C1-C6 alkyl, and 5- to 6-membered heteroaryl;
preferably, R⁴ is selected from the group consisting of hydroxy, cyano, nitro, carboxyl, mercapto, halogen, C1-C6 alkyl, -NR^{a}R^{b}, -CONR^{a}R^{b}, phenyl, phenoxy, -C(=O)-C1-C6 alkyl, benzyl, C1-C6 alkoxy, C2-C6 alkenyl, C3-C8 cycloalkyl, -C(=O)O-C1-C6 alkyl, -C(=O)O-phenyl, C1-C6 alkylthio, -S(=O)-C1-C6 alkyl, -S(=O)₂-C1-C6 alkyl, and thienyl (e.g., 2-thienyl);
   R^{a}, and R^{b} are each independently selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
   preferably, R^{a}, and R^{b} are each independently selected from the group consisting of hydrogen, methyl, 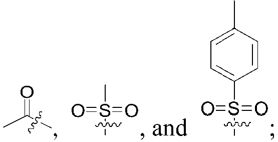
   or preferably, one of R^{a} and R^{b} is selected from the group consisting of hydrogen, and C1-C6 alkyl, and the other is selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
   or more preferably, one of R^{a} and R^{b} is selected from the group consisting of hydrogen, and methyl, and the other is selected from the group consisting of hydrogen, methyl, 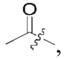 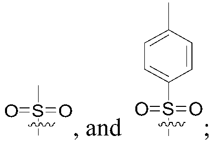
more preferably, R⁴ is selected from the group consisting of hydroxy, cyano, nitro, carboxyl, -NH₂, -SH, -CONH₂, methyl, phenyl, benzyl, methoxy, phenoxy, acetyl, -F, -Cl, -Br, -CH=CH₂, cyclopentyl, -COOMe, -COOPh, -SMe, -SOMe, -SO₂Me, -NMe₂, -NHAc, -NHMs, -NHTs, and 2-thienyl;
most preferably, R⁴ is hydroxy;
R⁵ is selected from the group consisting of hydrogen, C1-C6 alkyl, C1-C6 alkoxy, and C1-C6 alkylthio;
preferably, R⁵ is selected from the group consisting of hydrogen, C1-C6 alkyl, and C1-C6 alkoxy;
more preferably, R⁵ is selected from the group consisting of C1-C6 alkoxy;
most preferably, R⁵ is methoxy.

In some embodiments, the structural formula of the compound is as shown in Formula I-2, 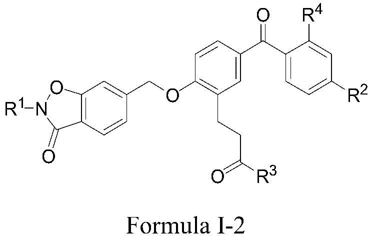 wherein:
R¹ is selected from the group consisting of hydrogen, -(CH₂)ₘ-O-C1-C6 alkyl, and -NR^{c}R^{d};
preferably, R¹ is selected from the group consisting of hydrogen, and -(CH₂)ₘ-O-C1-C6 alkyl;
   R^{c}, and R^{d} are each independently selected from the group consisting of hydrogen, C1-C6 alkyl, and -(CH₂)ₘ-O-C1-C6 alkyl;
   preferably, R^{c}, and R^{d} are each independently selected from the group consisting of hydrogen, and -(CH₂)ₘ-O-C1-C6 alkyl;
   more preferably, one of R^{c} and R^{d} is hydrogen, and the other is selected from the group consisting of -(CH₂)ₘ-O-C1-C6 alkyl;
   m is selected from the group consisting of 0, 1, 2, 3, 4, 5, and 6;
   preferably, m is selected from the group consisting of 0, and 1;
   more preferably, m is 1;
more preferably, R¹ is selected from the group consisting of hydrogen, and 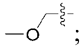
R² is selected from the group consisting of -O-(CH₂)ₙ-R^{2'};
   n is selected from the group consisting of 0, 1, 2, 3, 4, 5, and 6;
   preferably, n is selected from the group consisting of 0, and 1;
   R^{2'} is selected from the group consisting of C1-C6 alkyl, C3-C8 cycloalkyl, C6-C10 aryl, and 5- to 6-membered heteroaryl;
   preferably, R^{2'} is selected from the group consisting of C1-C6 alkyl, C3-C8 cycloalkyl, phenyl, pyrazinyl (e.g., 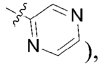 and furanyl (e.g., 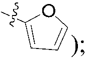
   more preferably, R^{2'} is selected from the group consisting of cyclopropyl, cyclobutyl, cyclopentyl, cyclohexyl, cycloheptyl, 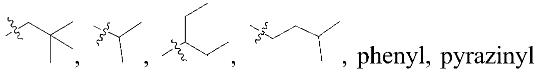
   (e.g., 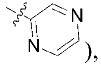 and furanyl (e.g., 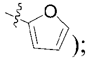
   or preferably, R^{2'} is selected from the group consisting of C3-C8 cycloalkyl;
   or more preferably, R^{2'} is selected from the group consisting of cyclopentyl, and cyclohexyl;
   or most preferably, R^{2'} is cyclopentyl;
preferably, R² is selected from the group consisting of -O-C1-C6 alkyl, -O-C3-C8 cycloalkyl, -O-CH₂-C6-C10 aryl, and -O-CH₂-5- to 6-membered heteroaryl;
more preferably, R² is selected from the group consisting of -O-C1-C6 alkyl, -O-C3-C8 cycloalkyl, -O-CH₂-phenyl, and -O-CH₂-5- to 6-membered heteroaryl, wherein the 5- to 6-membered heteroaryl is selected from the group consisting of pyrazinyl (e.g., 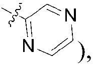 and furanyl (e.g., 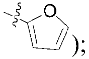
most preferably, R² is selected from the group consisting of 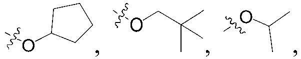 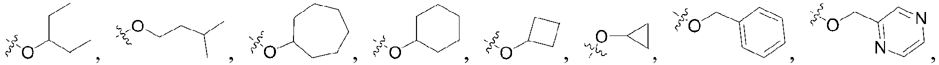 and 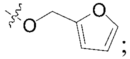
or preferably, R² is selected from the group consisting of -O-C3-C8 cycloalkyl;
or more preferably, R² is selected from the group consisting of 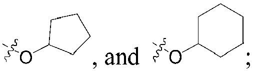
or most preferably, R² is 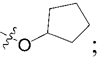
R³ is selected from the group consisting of hydroxy, and -O-C1-C6 alkyl, wherein the C1-C6 alkyl is optionally substituted with 5- to 6-membered heterocyclyl;
preferably, R³ is selected from the group consisting of hydroxy, and -O-C1-C6 alkyl, wherein the C1-C6 alkyl is optionally substituted with 6-membered heterocyclyl;
more preferably, R³ is selected from the group consisting of hydroxy, and -O-C1-C6 alkyl, wherein the C1-C6 alkyl is optionally substituted with morpholinyl;
further preferably, R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, -O(CH₂)₅CH₃, and 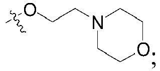
still further preferably, R³ is selected from the group consisting of -OCH₃, -OCH₂CH₃, -OCH(CH₃)CH₃, -O(CH₂)₂CH₃, and 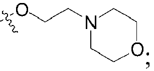
or preferably, R³ is selected from the group consisting of hydroxy, and -O-C1-C6 alkyl;
or more preferably, R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, and -O(CH₂)₅CH₃;
or further preferably, R³ is selected from the group consisting of hydroxy, -OCH₃, -OCH₂CH₃, -O(CH₂)₂CH₃, -O(CH₂)₃CH₃, -O(CH₂)₄CH₃, and -O(CH₂)₅CH₃;
or preferably, R³ is selected from the group consisting of -O-C1-C6 alkyl;
or more preferably, R³ is selected from the group consisting of -OCH₃, -OCH₂CH₃, -OCH(CH₃)CH₃, and -O(CH₂)₂CH₃;
or most preferably, R³ is selected from the group consisting of -OCH₃, and -OCH₂CH₃;
R⁴ is selected from the group consisting of hydroxy, cyano, nitro, carboxyl, mercapto, halogen, C1-C6 alkyl, -NR^{a}R^{b}, -CONR^{a}R^{b}, C6-C10 aryl, -O-C6-C10 aryl, -C(=O)-C1-C6 alkyl, -CH₂-C6-C10 aryl, C1-C6 alkoxy, C2-C6 alkenyl, C3-C8 cycloalkyl, -C(=O)O-C1-C6 alkyl, -C(=O)O-C6-C10 aryl, C1-C6 alkylthio, -S(=O)-C1-C6 alkyl, -S(=O)₂-C1-C6 alkyl, and 5- to 6-membered heteroaryl;
preferably, R⁴ is selected from the group consisting of hydroxy, cyano, nitro, carboxyl, mercapto, halogen, C1-C6 alkyl, -NR^{a}R^{b}, -CONR^{a}R^{b}, phenyl, phenoxy, -C(=O)-C1-C6 alkyl, benzyl, C1-C6 alkoxy, C2-C6 alkenyl, C3-C8 cycloalkyl, -C(=O)O-C1-C6 alkyl, -C(=O)O-phenyl, C1-C6 alkylthio, -S(=O)-C1-C6 alkyl, -S(=O)₂-C1-C6 alkyl, and thienyl (e.g., 2-thienyl);
   R^{a}, and R^{b} are each independently selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
   preferably, R^{a}, and R^{b} are each independently selected from the group consisting of hydrogen, methyl, 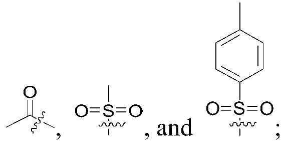
   or preferably, one of R^{a} and R^{b} is selected from the group consisting of hydrogen, and C1-C6 alkyl, and the other is selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
   or more preferably, one of R^{a} and R^{b} is selected from the group consisting of hydrogen, and methyl, and the other is selected from the group consisting of hydrogen, methyl, 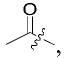 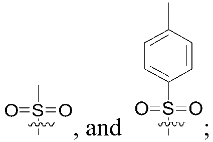
more preferably, R⁴ is selected from the group consisting of hydroxy, cyano, nitro, carboxyl, -NH₂, -SH, -CONH₂, methyl, phenyl, benzyl, methoxy, phenoxy, acetyl, -F, -Cl, -Br, -CH=CH₂, cyclopentyl, -COOMe, -COOPh, -SMe, -SOMe, -SO₂Me, -NMe₂, -NHAc, -NHMs, -NHTs, and 2-thienyl;
most preferably, R⁴ is hydroxy;
and, the compound represented by Formula I-2 does not include

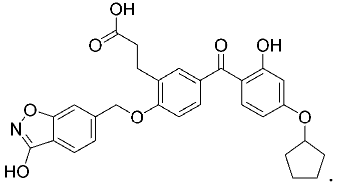

In some embodiments, the structural formula of the compound is as shown in Formula I-3, 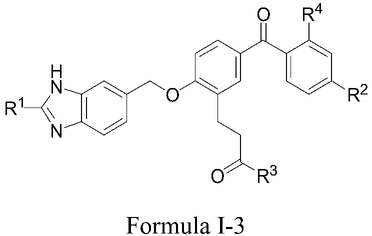 wherein:
R¹ is selected from the group consisting of hydrogen, and C1-C6 alkyl;
preferably, R¹ is hydrogen;
R² is selected from the group consisting of -O-C3-C8 cycloalkyl;
preferably, R² is 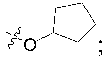
R³ is selected from the group consisting of -O-C 1-C6 alkyl;
preferably, R³ is -OCH₂CH₃;
R⁴ is hydroxy.

In some embodiments, the compound is selected from the group consisting of:

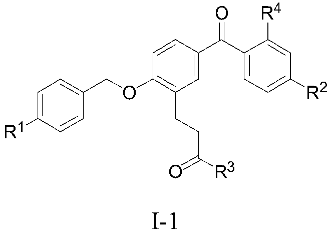

| | R¹ | R² | R³ | R⁴ |
|---|---|---|---|---|
| T3 | -COOH | 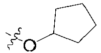 | -OH | -OH |
| | -COOCH₃ | 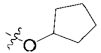 | -OH | -OH |
| | -COOCH₂CH₃ | 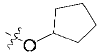 | -OH | -OH |
| | -COO(CH₂)₂CH₃ | 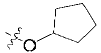 | -OH | -OH |
| | -COO(CH₂)₃CH₃ | 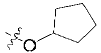 | -OH | -OH |
| | -COO(CH₂)₄CH₃ | 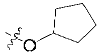 | -OH | -OH |
| | -COO(CH₂)₅CH₃ | 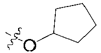 | -OH | -OH |
| | -COOH | 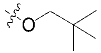 | -OH | -OH |
| | -COOH | 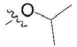 | -OH | -OH |
| | -COOH | 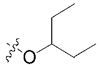 | -OH | -OH |
| | -COOH | 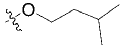 | -OH | -OH |
| T1 | -COOH | 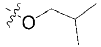 | -OH | -OH |
| | -COOH | 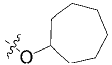 | -OH | -OH |
| | -COOH | 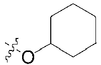 | -OH | -OH |
| | -COOH | 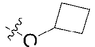 | -OH | -OH |
| | -COOH | 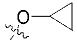 | -OH | -OH |
| T4 | -COOH | 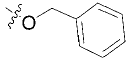 | -OH | -OH |
| | -COOH | 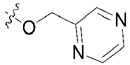 | -OH | -OH |
| | -COOH | 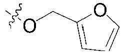 | -OH | -OH |
| | -COOH | 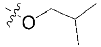 | -OCH₃ | -OH |
| | -COOH | 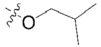 | -OCH₂CH₃ | -OH |
| | -COOH | 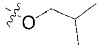 | -O(CH₂)₂CH₃ | -OH |
| | -COOH | 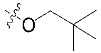 | -OCH₃ | -OH |
| | -COOH | 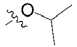 | -OCH₃ | -OH |
| | -COOH | 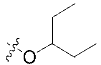 | -OCH₃ | -OH |
| | -COOH | 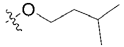 | -OCH₃ | -OH |
| | -COOH | 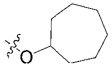 | -OCH₃ | -OH |
| | -COOH | 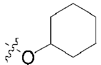 | -OCH₃ | -OH |
| T12 | -COOH | 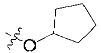 | -OCH₃ | -OH |
| | -COOH | 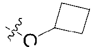 | -OCH₃ | -OH |
| | -COOH | 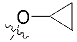 | -OCH₃ | -OH |
| | -COOH | 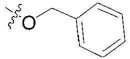 | -OCH₃ | -OH |
| | -COOH | 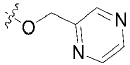 | -OCH₃ | -OH |
| | -COOH | 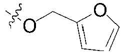 | -OCH₃ | -OH |
| | -COOH | 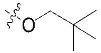 | -OCH₂CH₃ | -OH |
| | -COOH | 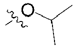 | -OCH₂CH₃ | -OH |
| | -COOH | 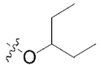 | -OCH₂CH₃ | -OH |
| | -COOH | 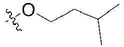 | -OCH₂CH₃ | -OH |
| | -COOH | 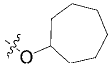 | -OCH₂CH₃ | -OH |
| | -COOH | 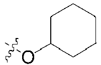 | -OCH₂CH₃ | -OH |
| | -COOH | 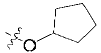 | -OCH₂CH₃ | -OH |
| | -COOH | 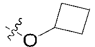 | -OCH₂CH₃ | -OH |
| | -COOH | 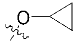 | -OCH₂CH₃ | -OH |
| | -COOH | 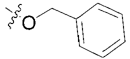 | -OCH₂CH₃ | -OH |
| | -COOH | 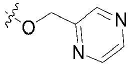 | -OCH₂CH₃ | -OH |
| | -COOH | 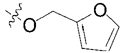 | -OCH₂CH₃ | -OH |
| | -COOCH₃ | 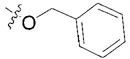 | -OH | -OH |
| | -COOCH₂CH₃ | 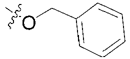 | -OH | -OH |
| | -COO(CH₂)₂CH₃ | 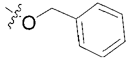 | -OH | -OH |
| | -COO(CH₂)₃CH₃ | 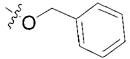 | -OH | -OH |
| | -COO(CH₂)₄CH₃ | 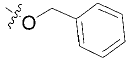 | -OH | -OH |
| | -COO(CH₂)₅CH₃ | 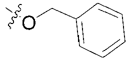 | -OH | -OH |
| | -COOCH₃ | 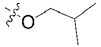 | -OH | -OH |
| T2 | -COOCH₂CH₃ | 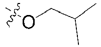 | -OH | -OH |
| | -COO(CH₂)₂CH₃ | 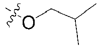 | -OH | -OH |
| | -COO(CH₂)₃CH₃ | 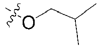 | -OH | -OH |
| | -COO(CH₂)₄CH₃ | 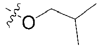 | -OH | -OH |
| | -COO(CH₂)₅CH₃ | 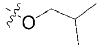 | -OH | -OH |
| | -COOH | 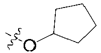 | -OCH₃ | -OH |
| T13 | -COOCH₃ | 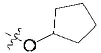 | -OCH₃ | -OH |
| | -COOCH₂CH₃ | 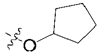 | -OCH₃ | -OH |
| | -COO(CH₂)₂CH₃ | 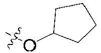 | -OCH₃ | -OH |
| | -COO(CH₂)₃CH₃ | 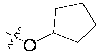 | -OCH₃ | -OH |
| | -COO(CH₂)₄CH₃ | 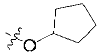 | -OCH₃ | -OH |
| | -COO(CH₂)₅CH₃ | 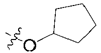 | -OCH₃ | -OH |
| | -COOH | 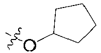 | -OCH₂CH₃ | -OH |
| | -COOCH₃ | 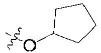 | -OCH₂CH₃ | -OH |
| | -COOCH₂CH₃ | 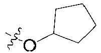 | -OCH₂CH₃ | -OH |
| | -COO(CH₂)₂CH₃ | 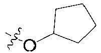 | -OCH₂CH₃ | -OH |
| | -COO(CH₂)₃CH₃ | 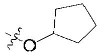 | -OCH₂CH₃ | -OH |
| | -COO(CH₂)₄CH₃ | 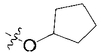 | -OCH₂CH₃ | -OH |
| | -COO(CH₂)₅CH₃ | 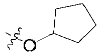 | -OCH₂CH₃ | -OH |
| | -COOH | 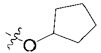 | -O(CH₂)₂CH₃ | -OH |
| | -COOCH₃ | 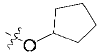 | -O(CH₂)₂CH₃ | -OH |
| | -COOCH₂CH₃ | 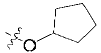 | -O(CH₂)₂CH₃ | -OH |
| | -COO(CH₂)₂CH₃ | 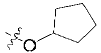 | -O(CH₂)₂CH₃ | -OH |
| | -COO(CH₂)₃CH₃ | 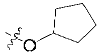 | -O(CH₂)₂CH₃ | -OH |
| | -COO(CH₂)₄CH₃ | 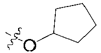 | -O(CH₂)₂CH₃ | -OH |
| | -COO(CH₂)₅CH₃ | 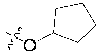 | -O(CH₂)₂CH₃ | -OH |
| | -COOH | 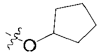 | -OH | -CN |
| | -COOH | 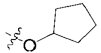 | -OH | -NO₂ |
| | -COOH | 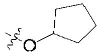 | -OH | -COOH |
| | -COOH | 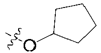 | -OH | -NH₂ |
| | -COOH | 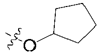 | -OH | -SH |
| | -COOH | 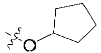 | -OH | -CONH₂ |
| | -COOH | 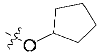 | -OH | -Me |
| | -COOH | 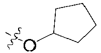 | -OH | -Ph |
| | -COOH | 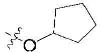 | -OH | -Bn |
| | -COOH | 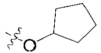 | -OH | -OMe |
| | -COOH | 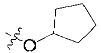 | -OH | -OPh |
| | -COOH | 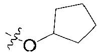 | -OH | -Ac |
| | -COOH | 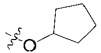 | -OH | -F |
| | -COOH | 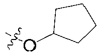 | -OH | -Cl |
| | -COOH | 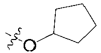 | -OH | -Br |
| | -COOH | 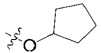 | -OH | -CH=CH2 |
| | -COOH | 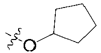 | -OH | 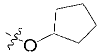 |
| | -COOH | 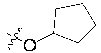 | -OH | -COOMe |
| | -COOH | 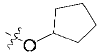 | -OH | -COOPh |
| | -COOH | 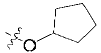 | -OH | -SMe |
| | -COOH | 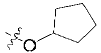 | -OH | -SOMe |
| | -COOH | 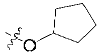 | -OH | -SO₂Me |
| | -COOH | 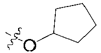 | -OH | -NMe₂ |
| | -COOH | 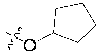 | -OH | -NHAc |
| | -COOH | 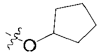 | -OH | -NHMs |
| | -COOH | 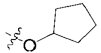 | -OH | -NHTs |
| | -COOH | 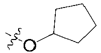 | -OH | 2-thienyl |

or,

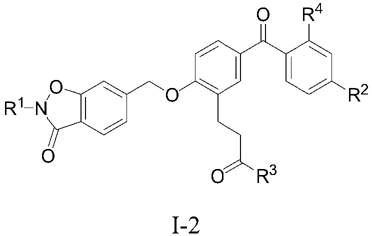

| | R¹ | R² | R³ | R⁴ |
|---|---|---|---|---|
| T8 | H | 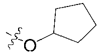 | -OCH₃ | -OH |
| T5 | H | 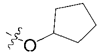 | -OCH₂CH₃ | -OH |
| T9 | H | 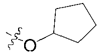 | -OCH(CH₃)CH₃ | -OH |
| T16 | H | 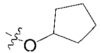 | -O(CH₂)₂CH₃ | -OH |
| | H | 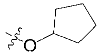 | -O(CH₂)₃CH₃ | -OH |
| | H | 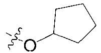 | -O(CH₂)₄CH₃ | -OH |
| | H | 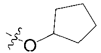 | -O(CH₂)₅CH₃ | -OH |
| | H | 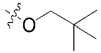 | -OH | -OH |
| | H | 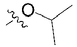 | -OH | -OH |
| | H | 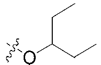 | -OH | -OH |
| | H | 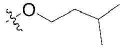 | -OH | -OH |
| | H | 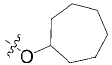 | -OH | -OH |
| | H | 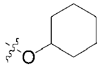 | -OH | -OH |
| | H | 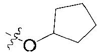 | -OH | -OH |
| | H | 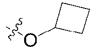 | -OH | -OH |
| | H | 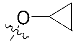 | -OH | -OH |
| | H | 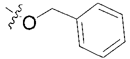 | -OH | -OH |
| | H | 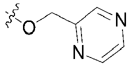 | -OH | -OH |
| | H | 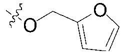 | -OH | -OH |
| | H | 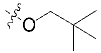 | -OCH₃ | -OH |
| | H | 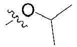 | -OCH₃ | -OH |
| | H | 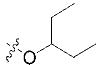 | -OCH₃ | -OH |
| | H | 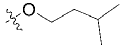 | -OCH₃ | -OH |
| | H | 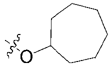 | -OCH₃ | -OH |
| T10 | H | 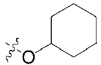 | -OCH₃ | -OH |
| | H | 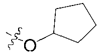 | -OCH₃ | -OH |
| | H | 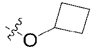 | -OCH₃ | -OH |
| | H | 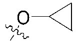 | -OCH₃ | -OH |
| | H | 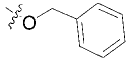 | -OCH₃ | -OH |
| | H | 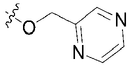 | -OCH₃ | -OH |
| | H | 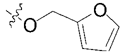 | -OCH₃ | -OH |
| | H | 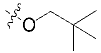 | -OCH₂CH₃ | -OH |
| | H | 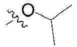 | -OCH₂CH₃ | -OH |
| | H | 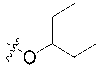 | -OCH₂CH₃ | -OH |
| | H | 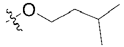 | -OCH₂CH₃ | -OH |
| | H | 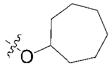 | -OCH₂CH₃ | -OH |
| T11 | H | 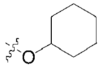 | -OCH₂CH₃ | -OH |
| | H | 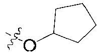 | -OCH₂CH₃ | -OH |
| | H | 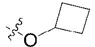 | -OCH₂CH₃ | -OH |
| | H | 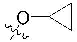 | -OCH₂CH₃ | -OH |
| | H | 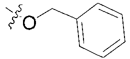 | -OCH₂CH₃ | -OH |
| | H | 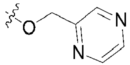 | -OCH₂CH₃ | -OH |
| | H | 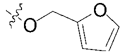 | -OCH₂CH₃ | -OH |
| | | 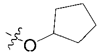 | -OH | -OH |
| T6 | | 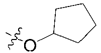 | -OCH₃ | -OH |
| | | 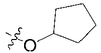 | -OCH₂CH₃ | -OH |
| | H | 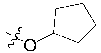 | -OH | -CN |
| | H | 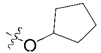 | -OH | -NO₂ |
| | H | 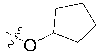 | -OH | -COOH |
| | H | 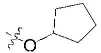 | -OH | -NH₂ |
| | H | 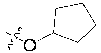 | -OH | -SH |
| | H | 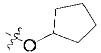 | -OH | -CONH₂ |
| | H | 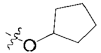 | -OH | -Me |
| | H | 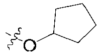 | -OH | -Ph |
| | H | 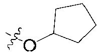 | -OH | -Bn |
| | H | 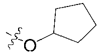 | -OH | -OMe |
| | H | 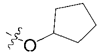 | -OH | -OPh |
| | H | 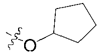 | -OH | -Ac |
| | H | 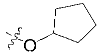 | -OH | -F |
| | H | 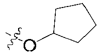 | -OH | -Cl |
| | H | 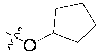 | -OH | -Br |
| | H | 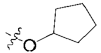 | -OH | -CH=CH₂ |
| | H | 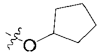 | -OH | 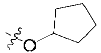 |
| | H | 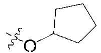 | -OH | -COOMe |
| | H | 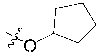 | -OH | -COOPh |
| | H | 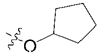 | -OH | -SMe |
| | H | 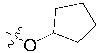 | -OH | -SOMe |
| | H | 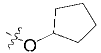 | -OH | -SO₂Me |
| | H | 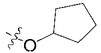 | -OH | -NMe₂ |
| | H | 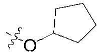 | -OH | -NHAc |
| | H | 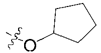 | -OH | -NHMs |
| | H | 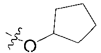 | -OH | -NHTs |
| | H | 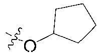 | -OH | 2-thienyl |
| T14 | H | 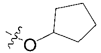 | 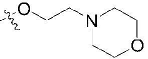 | -OH |

or,

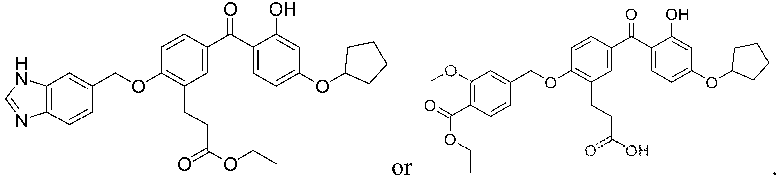

In a second aspect, the present invention provides the use of a pharmaceutical composition in the manufacture of a medicament for treating and/or preventing heart failure with preserved ejection fraction, wherein the pharmaceutical composition comprises a compound represented by Formula I, or a stereoisomer, prodrug, crystal form, pharmaceutically acceptable salt, pharmaceutically acceptable ester, or pharmaceutically acceptable solvate thereof,
or, provides a pharmaceutical composition for use in treating and/or preventing heart failure with preserved ejection fraction, wherein the pharmaceutical composition comprises a compound represented by Formula I, or a stereoisomer, prodrug, crystal form, pharmaceutically acceptable salt, pharmaceutically acceptable ester, or pharmaceutically acceptable solvate thereof,
or, provides a method for treating and/or preventing heart failure with preserved ejection fraction, comprising: administering to a subject in need thereof an effective amount of a pharmaceutical composition, wherein the pharmaceutical composition comprises a compound represented by Formula I, or a stereoisomer, prodrug, crystal form, pharmaceutically acceptable salt, pharmaceutically acceptable ester, or pharmaceutically acceptable solvate thereof, 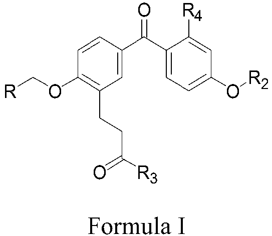
wherein, R, R², R³, R⁴ are each independently as defined in any technical solution of the first aspect;
preferably, the structural formula of the compound is as shown in Formula I-1, Formula I-1', Formula I-2 or Formula I-3, wherein the compound represented by Formula I-1 or Formula I-1' is as described in the first aspect, the compound represented by Formula I-2 is as described in the first aspect, and the compound represented by Formula I-3 is as described in the first aspect;
more preferably, the structural formula of the compound is as shown in Formula I-1, Formula I-2 or Formula I-3, wherein the compound represented by Formula I-1 is as described in the first aspect, the compound represented by Formula I-2 is as described in the first aspect, and the compound represented by Formula I-3 is as described in the first aspect;
more preferably, the structural formula of the compound is as shown in Formula I-1, wherein the compound represented by Formula I-1 is as described in the first aspect;
more preferably, the structural formula of the compound is as shown in Formula I-1', wherein the compound represented by Formula I-1' is as described in the first aspect;
more preferably, the structural formula of the compound is as shown in Formula I-2, wherein the compound represented by Formula I-2 is as described in the first aspect;
more preferably, the structural formula of the compound is as shown in Formula I-3, wherein the compound represented by Formula I-3 is as described in the first aspect;
most preferably, the compound is as described above.

### Brief Description of the Drawings

Figure 1 shows the construction of HFpEF model. A: Schematic diagram of the experimental procedure; B: Results of systolic function test in mice after 5 weeks of feeding; C: Results of diastolic function test in mice after 5 weeks of feeding.
Figure 2 shows the relative expression levels of *Jun* in cardiomyocytes after 15 weeks of HFD+L-NAME feeding, indicating high expression of *Jun* in HFpEF model mice.
Figure 3 shows that T-5224 effectively alleviated the occurrence and development of HFpEF. A: Results of systolic function test in mice at different time points; B: Results of diastolic function test in mice at different time points; C: Changes in body weight of mice under different treatments; D: Changes in *Jun* expression levels in mouse cardiomyocytes under different treatments.
Figure 4 shows the results of the inhibitory effects of compounds at different concentrations on the binding activity of *Jun* to DNA at the molecular level.
Figure 5 shows the results of the inhibitory effects of compounds at different concentrations on cellular *Jun* transcriptional activity.
Figures 6 to 9 show that the compounds of the present invention effectively inhibit the occurrence and development of HFpEF.
Figures 10 to 18 show the NMR spectra of the compounds of the present invention.

### Specific Models for Carrying Out the Invention

It should be understood that the terms used herein are for the purpose of describing specific embodiments only and is not intended to be limiting. Furthermore, although any methods, devices, and materials similar or equivalent to those described herein can be used in the practice or testing of the present invention, the preferred methods, devices, and materials are now described.

In the present invention, unless otherwise specified, the description "each independently selected from" used throughout the text can indicate that the specific options expressed by the same or different symbols in different groups do not affect each other, and can also indicate that the specific options expressed by the same or different symbols in the same group do not affect each other.

The substituents of the compounds of the present invention are disclosed according to group types or ranges. It is specifically noted that the present invention includes each individual sub-combination of the members of these group types and ranges. For example, the term "C1-C6 alkyl" specifically refers to independently disclosed methyl, ethyl, C3 alkyl, C4 alkyl, C5 alkyl, and C6 alkyl.

The term "C1-C6 alkyl" refers to an alkyl group having 1 to 6 carbon atoms, preferably "C1-C4 alkyl", more preferably "C1-C3 alkyl", most preferably "C1-C2 alkyl". Examples of "C1-C6 alkyl" include, but are not limited to, methyl, ethyl, propyl (e.g., n-propyl, isopropyl), butyl (e.g., n-butyl, isobutyl, tert-butyl), pentyl (e.g., n-pentyl, isopentyl, neopentyl), etc. Examples of "C1-C4 alkyl" include, but are not limited to, methyl, ethyl, propyl (e.g., n-propyl, isopropyl), butyl (e.g., n-butyl, isobutyl, tert-butyl), etc. Examples of "C1-C3 alkyl" include methyl, ethyl, propyl (e.g., n-propyl, isopropyl), etc. Examples of "C1-C2 alkyl" include methyl, ethyl.

The term "C1-C6 alkoxy" refers to any of the above C1-C6 alkyl groups attached to the rest of the molecule via an oxygen atom (-O-), and its examples include methoxy, ethoxy, isopropoxy, etc.

The term "C1-C6 alkylthio" refers to a group obtained by replacing the oxygen atom in any of the above C1-C6 alkoxy groups with a sulfur atom, and its examples include C1-C6 alkylthio, C1-C4 alkylthio, C1-C3 alkylthio, etc.

The term "C3-C8 cycloalkyl" refers to a saturated monocyclic or polycyclic hydrocarbon substituent containing 3 to 8 carbon atoms, and its non-limiting examples include cyclopropyl, cyclobutyl, cyclopentyl, cyclohexyl, cycloheptyl, cyclooctyl, etc., preferably "C3-C6 cycloalkyl".

The term "C2-C6 alkenyl" refers to a straight or branched chain aliphatic hydrocarbon group having 2 to 6 carbon atoms and containing one or more carbon-carbon double bonds, and its examples include vinyl, allyl, etc.

The term "aryl" refers to a group of a carbocyclic aromatic system. For example, "C6-C10 aryl" refers to a group of a carbocyclic aromatic system having 6 to 10 carbon atoms, and its non-limiting examples include, but are not limited to, phenyl, naphthyl, etc.

The term "heteroaryl" refers to an aromatic monocyclic, bicyclic, tricyclic, or more polycyclic group having at least one heteroatom (N, O, or S) in at least one ring, wherein the heteroatom-containing ring optionally further has 1, 2, or 3 heteroatoms selected from the group consisting of N, O, and S, and for bicyclic, tricyclic, or more polycyclic heteroaryl, each ring in the bicyclic, tricyclic, or polycyclic system must constitute an aromatic system. For example, "5-to 6-membered heteroaryl" refers to a 5- or 6-membered aromatic monocyclic, bicyclic, tricyclic, or more polycyclic group having at least one heteroatom (N, O, or S) in at least one ring, and its non-limiting examples include, for example, pyridyl, pyrazinyl, pyrimidinyl, pyridazinyl, imidazolyl, thiazolyl, isothiazolyl, thioxazolyl, pyrrolyl, furanyl, oxazolyl, isoxazolyl, pyrazolyl, thienyl. For another example, the term "6- to 10-membered heteroaryl" refers to a 6-, 7-, 8-, 9-, or 10-membered aromatic monocyclic, bicyclic, tricyclic, or more polycyclic group having at least one heteroatom (N, O, or S) in at least one ring, and its non-limiting examples include, for example, pyridyl, pyrazinyl, pyrimidinyl, pyridazinyl, indolyl, imidazolyl, phenyl-pyrrolyl, phenyl-furanyl, benzofuranyl, benzothienyl, benzimidazolyl, indazolyl, quinolinyl, isoquinolinyl, etc.

The term "heterocyclyl" refers to a 3-, 4-, 5-, 6-, 7-, 8-, 9-, or 10-membered (preferably 3- to 7-membered or 3- to 6-membered) saturated or partially unsaturated carbocycle, wherein one or more carbon atoms are replaced by heteroatoms such as nitrogen, oxygen, and sulfur. Non-limiting examples of heterocyclyl include, for example, pyran, pyrrolidine, pyrroline, imidazoline, imidazolidine, pyrazolidine, pyrazoline, thiazoline, thiazolidine, dihydrofuran, tetrahydrofuran, 1,3-dioxolane, piperidine, piperazine, morpholine, morpholinyl, tetrahydropyrrolyl, thiomorpholinyl, etc. For example, "6-membered heterocyclyl" refers to a 6-membered saturated or partially unsaturated carbocycle, wherein one or more carbon atoms are replaced by heteroatoms such as nitrogen, oxygen, and sulfur. Non-limiting examples of 6-membered heterocyclyl include, for example, pyran, piperidine, piperazine, morpholine, morpholinyl, thiomorpholinyl, etc. For another example, "5-membered heterocyclyl" refers to a 5-membered saturated or partially unsaturated carbocycle, wherein one or more carbon atoms are replaced by heteroatoms such as nitrogen, oxygen, and sulfur. Non-limiting examples of 5-membered heterocyclyl include, for example, pyrrolidine, pyrroline, imidazoline, imidazolidine, pyrazolidine, pyrazoline, thiazoline, thiazolidine, 1,3-dioxolane, etc.

The term "heteroatom" refers to N, O, or S.

The term "substituted" means that any hydrogen on a designated atom or group is selectively replaced by a designated group, provided that the normal valence of the designated atom is not exceeded.

In the present invention, the term "treatment" generally refers to obtaining a desired pharmacological and/or physiological effect. The effect may be prophylactic in terms of completely or partially preventing a disease or its symptoms; and/or therapeutic in terms of partially or completely stabilizing or curing a disease and/or side effects resulting from the disease. "Treatment" as used herein covers any treatment of a disease in a patient, including: (a) preventing the disease or symptoms in a patient who may be predisposed to the disease or symptom but has not yet been diagnosed as having it; (b) inhibiting the symptom of the disease, i.e., arresting its development; or (c) relieving the symptom of the disease, i.e., causing regression of the disease or symptom.

In the present invention, "subject" refers to a vertebrate. In certain embodiments, the vertebrate refers to a mammal. Mammals include, but are not limited to, livestock (such as cattle), pets (such as cats, dogs, and horses), primates, mice, and rats. In certain embodiments, the mammal refers to a human.

In the present invention, "effective amount" refers to an amount effective to achieve the desired therapeutic effect at the required dosage and time. A "therapeutically effective amount" of a substance/molecule of the present invention may vary depending on factors such as the individual's disease state, age, sex, and weight, and the ability of the substance/molecule to elicit a desired response in the individual. A therapeutically effective amount also encompasses an amount where any toxic or detrimental effects of the substance/molecule are outweighed by the therapeutically beneficial effects.

The pharmaceutical composition according to the present invention may comprise a pharmaceutically acceptable adjuvant, including but not limited to: ion exchanger, alumina, aluminum stearate, lecithin, serum protein such as human serum albumin, buffer substance such as phosphate, glycerin, sorbic acid, potassium sorbate, partial glyceride mixture of saturated vegetable fatty acids, water, salt or electrolyte such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin, and the like.

The pharmaceutical composition of the present invention can be prepared in various forms according to different routes of administration. For example, the pharmaceutical composition can be administered in any of the following ways: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal, and intracranial injection or infusion, or administration using an external reservoir. Among them, oral administration or intravenous administration is preferred.

The compound of the present invention may optionally be used in combination with one or more other active ingredients, wherein the respective amounts and ratios thereof may be adjusted by those skilled in the art according to the specific disease condition, the particular circumstances of the patient, clinical needs, and the like.

As used herein, unless otherwise specified, the term "prodrug" refers to a derivative of a compound of the present invention that can be hydrolyzed, oxidized, or undergo other reactions under biological conditions (in vitro or in vivo) to provide the compound of the present invention. Prodrugs only become active compounds after such reactions under biological conditions, or they have no or only low activity in their unreacted form. Prodrugs can generally be prepared using known methods, such as those described in Burger's Medicinal Chemistry and Drug Discovery (1995) 172-178, 949-982 (edited by Manfred E. Wolff, 5th edition).

For stereoisomers in the compounds described herein, when specifically designated as an (R)- or (S)-isomer by chemical name, it should be understood as being primarily in the (R)-isomer or (S)-isomer configuration, respectively. Any asymmetric carbon atom may be present in the *(R)-,* (S)-, or (R,S)-configuration, preferably in the (R)- or (S)-configuration.

Tautomerism refers to the phenomenon where a functional group in a compound changes its structure to another functional isomer, and the two isomers can rapidly interconvert. This rapid and reversible conversion process allows the two isomers to coexist in a certain ratio under given conditions, forming a dynamic equilibrium state. Taking compounds A and B below as examples, those skilled in the art will understand that compounds A and B below are tautomers of each other (as indicated by the structural difference shown in the box), and the two isomers can rapidly interconvert to reach a certain equilibrium. Therefore, for those skilled in the art, compounds A and B below represent the same compound, only expressed slightly differently. Similarly, for other compounds with similar structures in the present application, those skilled in the art will also understand that different tautomers essentially represent the same compound, only expressed slightly differently.

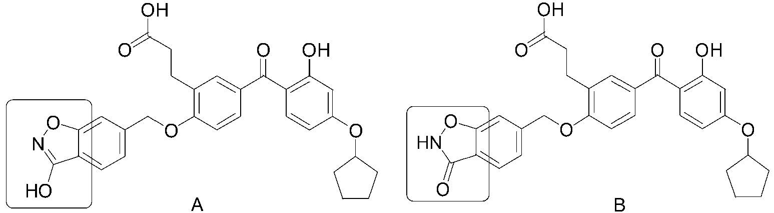

"Solvate" and "solvation compound" can be used interchangeably and refers to a compound that exists in combination with solvent molecules. This combination can include a stoichiometric amount of a certain solvent, such as a monohydrate or dihydrate, or can include any amount of water; for another example, methanol or ethanol can form "alcohol solvate", which can also be stoichiometric or non-stoichiometric. The term "solvate" as used herein refers to a solid form; thus, a compound in a solvent solution, although it may be solvated, is not a solvate as the term is used herein.

As used herein, the term "pharmaceutically acceptable salt" refers to: (i) a salt formed by the acidic functional group (e.g., -COOH) present in the compound provided by the present invention with an appropriate inorganic or organic cation (base), and includes, but is not limited to, alkali metal salt such as sodium salt, potassium salt, lithium salt, etc.; alkaline earth metal salt such as calcium salt, magnesium salt, etc.; other metal salt such as aluminum salt, iron salt, zinc salt, copper salt, nickel salt, cobalt salt, etc.; inorganic base salt such as ammonium salt; organic base salt such as tert-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt, tris(hydroxymethyl)aminomethane salt; and (ii) a salt formed by the basic functional group (e.g., -NH₂) present in the compound provided by the present invention with an appropriate inorganic or organic anion (acid), and includes, but is not limited to, hydrohalide such as hydrofluoride, hydrochloride, hydrobromide, hydroiodide, etc.; inorganic acid salt such as nitrate, perchlorate, sulfate, phosphate, etc.; lower alkylsulfonate such as methanesulfonate, trifluoromethanesulfonate, ethanesulfonate, etc.; arylsulfonate such as benzenesulfonate, p-toluenesulfonate, etc.; organic acid salt such as acetate, malate, fumarate, succinate, citrate, tartrate, oxalate, maleate, etc.; amino acid salt such as glycinate, trimethylglycinate, arginine salt, ornithine salt, glutamate, aspartate, etc.

As used herein, the term "pharmaceutically acceptable ester" refers to an ester formed by -COOH present in the compound provided by the present invention with an appropriate alcohol, or an ester formed by -OH present in the compound provided by the present invention with an appropriate acid (e.g., a carboxylic acid or an oxygen-containing inorganic acid). Suitable ester groups include, but are not limited to, formate, acetate, propionate, butyrate, acrylate, ethylsuccinate, stearate, or palmitate. The ester can be hydrolyzed in the presence of an acid or base to generate the corresponding acid or alcohol.

As used herein, the term "crystal form" refers to the crystal structure of a substance. During crystallization, a substance may be affected by various factors, causing changes in intramolecular or intermolecular bonding modes, thereby resulting in different arrangements of molecules or atoms in the crystal lattice, leading to different crystal structures. The compounds of the present invention can exist in one crystal structure or multiple crystal structures, i.e., have "polymorphism". The compound of the present invention can exist in different crystal forms.

The present invention is further explained and illustrated below with reference to specific examples. Unless otherwise specified, all reagents and raw materials were commercially available or could be prepared according to conventional techniques in the art based on the prior art and common knowledge, and all instruments were those routinely used by those skilled in the art.

### Example 1

### 1. Materials and reagents

In this example, C57BL/6N wild-type mice were purchased from Beijing Vital River. The sources of reagents were shown in the following table.

| Reagent | Cat. No. | Supplier |
|---|---|---|
| Collagenase type II | 17101015 | Gibico |
| T-5224 | S8966 | Selleck |
| Polyvinylpyrrolidone (PVP) | S3596 | Selleck |
| GeneJet RNA purification kit | K0732 | Thermo Scientific |
| iScript TM cDNA synthesis kit | 1708890 | Bio-Rad |
| iTaqUniversl SYBR Green supermix | 1725121 | Bio-Rad |

In addition to the above, other materials and reagents used in this example were also commercially available products.

### 2. Animal experiment guideline

In this example, all animal studies were conducted under the guidance of the Laboratory Animal Center of the Institutional Animal Care and Use Committee of Fuwai Hospital, National Center for Cardiovascular Diseases, China. All mice were propagated and raised in the same environment, and the mice were randomly divided into groups during the experiment. Echocardiographic analysis was performed by an independent researcher who was unaware of the study objectives.

### 3. Induction of model of heart failure with preserved ejection fraction

Male C57BL/6N wild-type mice aged 8 to 10 weeks were divided into three groups, namely, normal group (normal diet and drinking water), model control group (high-fat diet combined with administration of N-nitro-L-arginine methyl ester) and model treatment group (high-fat diet combined with administration of N-nitro-L-arginine methyl ester and treatment with T-5224). Among them, the model control group and the model treatment group were modeled by the following literature: Gabriele G. Schiattarella et al., Nitrosative stress drives heart failure with preserved ejection fraction, https://doi.org/10.1038/s41586-019-1100-z. Specifically, heart failure with preserved ejection fraction was induced by a high-fat diet (HFD) (60% kcal, from fat (lard)) and N-nitro-L-arginine methyl ester (abbreviated as L-NAME, 0.5 g/L in drinking water) to obtain the HFpEF animal model.

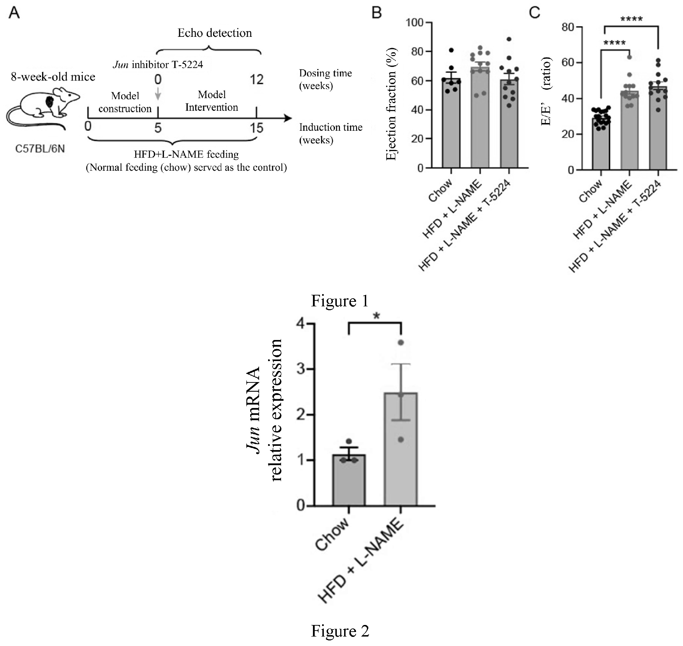

In the fifth week of model induction, the systolic function parameter LVEF of mice did not change, while the diastolic function parameter (E/E') increased significantly in the 5^{th} week of model induction, indicating that the model of heart failure with preserved ejection fraction recorded in the above literature had been successfully obtained. At the same time, there was no significant difference in the diastolic function parameter (E/E') between the model control group and the model treatment group in the 5^{th} week. Subsequent drug administration was carried out under the same baseline, as shown in Figure 1.

### 4. Jun expression was correlated with HFpEF

In the 15^{th} week of model induction, the cardiomyocytes of normal group and the model control group were isolated by perfusion method, and quantitative RCR detection was performed. The specific operations were as follows:

### 4.1. Isolation of adult mouse cardiomyocytes:

In order to isolate cardiomyocytes from the heart of adult mice, we used the classic perfusion method to isolate cardiomyocytes. Specifically, mice were injected with 100 µl of sodium heparin (1000 units in 50 ml) 20 minutes before being killed to prevent heart coagulation during the operation, which increased the difficulty of digestion. After that, the mice were anesthetized and killed, and the hearts were removed and transferred to a calcium-free solution for washing. Then, the Langendorff method was used for digestion. The heart was perfused with a calcium-free solution for 5 minutes using a Langendorff apparatus, and then digested with digestive enzyme solution (0.7 mg/ml type II collagenase and 0.7 mg/ml bovine serum albumin in a calcium-free solution) for about 30 minutes. The heart was constantly touched at about 20 minutes. When the heart became soft and slippery, which indicated that the digestion was basically completed, the tissues from the ventricles were then collected, cut into pieces, and gently pipetted to dissociate into single cells. The cells were allowed to settle, and the supernatant was taken to remove the undigested and adherent tissues, and centrifuged at 100 g and 4°C for 2 minutes to obtain cardiomyocyte pellets, and the supernatant was mostly non-cardiomyocytes. Cardiomyocytes were resuspended in a calcium-free solution containing 10% FBS for subsequent experiments. Non-cardiomyocytes could be resuspended with culture medium or PBS for subsequent experiments. If purer cardiomyocytes and non-cardiomyocytes were required, the cell suspension could be centrifuged (100g, 2 minutes at room temperature) three times to separate cardiomyocytes from non-cardiomyocytes. Cardiomyocytes were collected for further experiments.

### 4.2. Quantitative PCR detection:

Total RNA was extracted from cells using GeneJet RNA purification kit (Thermo Scientific, K0732), and 0.1µg of total RNA was reverse transcribed using iScript TM cDNA synthesis kit (Bio-Rad, 1708890) to generate cDNA. qPCR was performed using iTaqUniversl SYBR Green supermix (1725121, Bio-Rad) on ABI Vii7 real-time system (Life Technologies, Q6), and b-Actin was used for standardized quantitative analysis. As shown in Figure 2, compared with normal mice, high expression of *Jun* was significantly observed in the mouse HFpEF animal model. This indicated a correlation between *Jun* expression levels and HFpEF in mice, with *Jun* being highly expressed in HFpEF.

### 5. T-5224 administration method

After the animal model was obtained, that was, from the 5^{th} week of inducing the HFpEF model, the model treatment group was treated with T-5224, the model control group was treated with a solvent without drug, and the mice with normal diet and drinking water during the whole induction process were used as negative control. The mice were administrated with drugs when they were raised for 5 weeks. For the treatment group, T-5224 was administered at 250 mg/kg according to the body weight of mice. Specifically, the administration was performed every other day, 0.8 mg of T-5224 was dissolved in 200 uL of 1% PVP solution each time. The administration started from the 5^{th} week and ended at the 13^{th} week (a total of 15 administrations), with a total dose of 250 mg/kg. The control group was administrated with an equal volume of 1% PVP solution, and other treatments were the same.

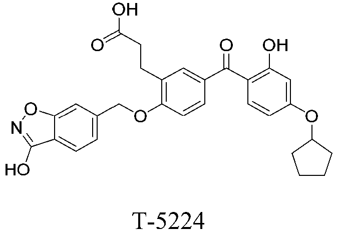

### 6. Conventional echocardiography and Doppler imaging

All the mice were raised under different conditions for five weeks and then began to undergo conventional echocardiography and Doppler imaging, which was performed every two weeks until the end of the 15^{th} week. Specifically, transthoracic echocardiography was performed using the VisualSonics Vevo 2100 system equipped with an MS400 transducer (Visual Sonics). Left ventricular ejection fraction (LVEF) and other systolic function indices were obtained from the short-axis M-mode scans at the midventricular level, as indicated by the presence of papillary muscles, in conscious, gently restrained mice. Cardiac apical four-chamber views were obtained in the anesthetized mice, and used for diastolic function measurements by pulsed waves and tissue Doppler imaging at the level of mitral valve. Anesthesia was induced by 2.5% isoflurane and confirmed by lack of response to firm pressure on one of the hind paws. During echocardiographic acquisition (under temperature-controlled conditions), isoflurane was reduced to 1.0% to 1.5% and adjusted to maintain heart rate in the range of 500 beats per minute. The parameters collected included: heart rate, left ventricular end-diastolic diameter, left ventricular end-systolic diameter, end-diastolic interventricular septal wall thickness, left ventricular end-diastolic posterior wall, left ventricular fractional shortening, LVEF, peak Doppler blood inflow velocity across mitral valve during early diastole, peak Doppler blood inflow velocity across mitral valve during late diastole, isovolumetric relaxation time, peak tissue Doppler of myocardial relaxation velocity at the mitral valve annulus during early diastole and early filling deceleration time. At the end of the procedure, all the mice recovered from anesthesia without any abnormalities. All parameters were measured at least 3 times, and the mean values were provided. The echocardiography and Doppler imaging included systolic function and diastolic function detection.

### 7. Experimental results and conclusions

Firstly, the detection of systolic function and diastolic function of mice in the 5^{th} week showed that there was no significant change in systolic function, but the diastolic function parameter E/E' increased significantly, proving that there was a disorder in diastolic function, indicating that the model described in the above literature was successfully obtained. At the same time, taking the 5^{th} week as the starting point of drug administration, there was no significant difference in cardiac diastolic function between the model control group and the model treatment group before drug administration (Figure 1A-C), and the drug administration was carried out on this basis.

Secondly, compared with the normal group, the expression of *Jun* in the model control group was upregulated, indicating that the expression of *Jun* was correlated with HFpEF. In the HFpEF mouse model, *Jun* was highly expressed (Figure 2). Based on this correlation, it could be judged that *Jun* inhibitor could be used for the prevention and treatment of HFpEF.

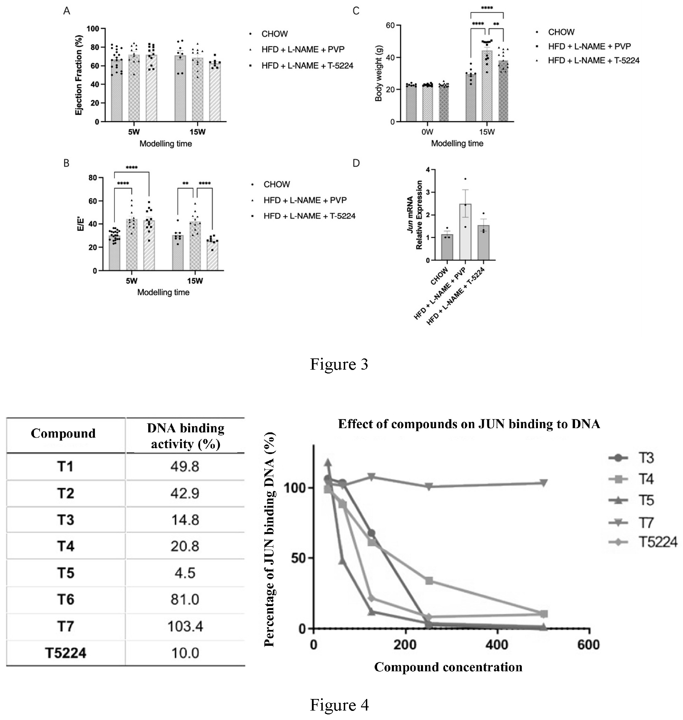

Further, T-5224 was used to verify the effectiveness of *Jun* inhibitor in preventing and treating HFpEF. After confirming that the model construction was successful and that the baselines of the model control group and the model treatment group were consistent, the model treatment group was treated with T-5224. The cardiac function detection results showed that in the model treatment group (after T-5224 administration), the occurrence and development of HFpEF had been well suppressed. Specifically, after treatment with T-5224, the diastolic function in the mice treated with high-fat diet combined with L-NAME (HFD + 0.5g/L L-NAME) was significantly improved and could be maintained until the 15^{th} week. However, in the model control mice that were not treated with T-5224, continued deterioration of diastolic function was observed (Figure 3A-B). At the same time, in the model treatment group, the expression of *Jun* was down-regulated compared with the model control group (Figure 3D), and the mouse obesity was improved (Figure 3C). This indicated that T-5224, as a *Jun* inhibitor, could bring about preventive and therapeutic effects on HFpEF in the mouse HFpEF model.

### Example 2

Based on the experimental results of Example 1, the inventors further explored the therapeutic efficacy of other compounds on HFpEF using T-5224 as a positive control.

### 1. Synthesis of Compounds

### 1.1 Synthesis of Compound Target 1 (abbreviated as T1)

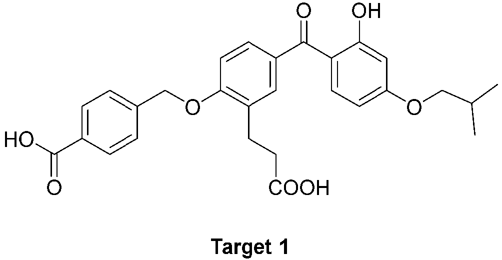

### 1) Synthesis of Compound 2

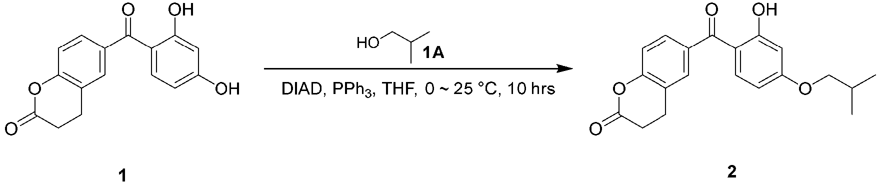

Under nitrogen protection, a solution of Compound 1 (10.0 g, 34.9 mmol, 1.00 *eq),* Compound 1A (2.85 g, 38.4 mmol, 3.55 mL, 1.10 *eq),* and triphenylphosphine (11.0 g, 41.9 mmol, 1.20 *eq)* in tetrahydrofuran (100 mL) was cooled to 0 °C. Diisopropyl azodicarboxylate (8.48 g, 41.9 mmol, 8.14 mL, 1.20 *eq)* was added dropwise, and the mixture was stirred at 25 °C for 10 hours. When LCMS (EC20183-4-P1A1) monitoring showed that Compound 1 was completely consumed and that a single major peak corresponding to the target molecular weight appeared (Rt = 0.36 min, MS cal.: 340.13, MS observed: [M+H]⁺ = 341.0), the reaction was quenched by adding water (300 mL) at 25 °C, and the reaction mixture was extracted with ethyl acetate (200 mL * 3). All organic phases were combined, washed with brine (300 mL), dried over anhydrous sodium sulfate, filtered, and concentrated under reduced pressure to obtain a residue. The crude product was purified by reversed-phase high-performance liquid chromatography (neutral conditions) to obtain Compound 2 as a pale yellow solid (4.68 g, 13.3 mmol, 38.1% yield, 97.1% purity).

LCMS: EC20183-4-P1A1, Rₜ = 0.36 min, MS cal.: 340.13, MS observed: [M+H]⁺ = 341.0.

LCMS: EC20183-4-P1A3, Rt = 0.39 min, MS cal.: 340.13, MS observed: [M+H]⁺ = 341.1.

HPLC: EC20183-4-P1A4, Rt = 2.34 min, 97.1% purity.

¹H NMR: EC20183-4-P1B, 400 MHz, DMSO-*d₆*
*δ*: 11.84 (s, 1H), 7.63 (s, 1H), 7.57 (d, *J =* 8.4 Hz, 1H), 7.44 (d, *J =* 9.6 Hz, 1H), 7.19 (d, *J =* 8.4 Hz, 1H), 6.54 - 6.53 (m, 2H), 3.83 (d, *J =* 8.8 Hz, 2H), 3.07 (t, *J =* 6.8 Hz, 2H), 2.84 (t, *J =* 8.0 Hz, 2H), 2.07 - 1.98 (m, 1H), 0.97 (d, *J =* 6.4 Hz, 6H).

### 2) Synthesis of Compound 3

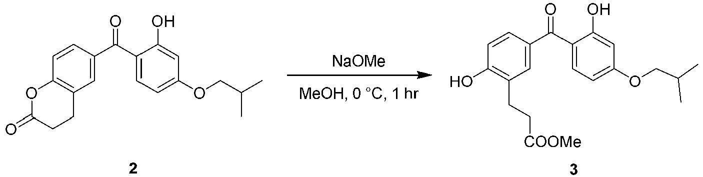

Compound 2 (4.68 g, 13.3 mmol, 1.00 *eq)* was dissolved in methanol (20.0 mL), then a solution of sodium methoxide (1.73 g, 32.0 mmol, 2.40 *eq)* in methanol (30.0 mL) was added. The mixture was stirred at 0 °C for 1 hour. When LCMS (EC20183-6-P1A) monitoring showed that Compound 2 was completely consumed and that a single major peak corresponding to the target molecular weight appeared (Rₜ = 0.53 min, MS cal.: 372.16, MS observed: [M+H]⁺ = 373.2), the reaction mixture was poured into 1 N hydrochloric acid (50.0 mL), diluted with water (200 mL), and extracted with ethyl acetate (100 mL * 3). All organic phases were combined, washed with brine (200 mL), dried over anhydrous sodium sulfate, filtered, and concentrated under reduced pressure to obtain Compound 3 as a pale yellow solid (4.97 g, 13.0 mmol, 97.4% yield, 97.5% purity).

LCMS: EC20183-6-P1A, Rt = 0.53 min, MS cal.: 372.16, MS observed: [M+H]⁺ = 373.2.

LCMS: EC20183-6-P1A1, Rₜ = 0.53 min, MS cal.: 372.16, MS observed: [M+H]⁺ = 373.2.

HPLC: EC20183-6-P1A2, Rt = 3.60 min, 97.5% purity.

### 3) Synthesis of Compound 4 - Notebook Page: EC20183-7

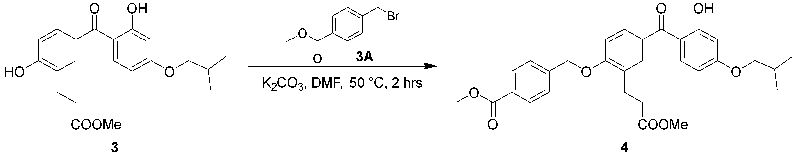

A solution of Compound 3 (2.48 g, 6.49 mmol, 1.00 *eq),* Compound 3A (1.86 g, 8.12 mmol, *1.25 eq),* and potassium carbonate (1.79 g, 12.9 mmol, 2.00 *eq)* in N,Ndimethylformamide (15.0 mL) was stirred at 50 °C for 2 hours. When LCMS (EC20183-7-P1A1) monitoring showed that Compound 3 was completely consumed and that a single major peak corresponding to the target molecular weight appeared (Rₜ = 0.47 min, MS cal.: 520.21, MS observed: [M+H]⁺ = 521.3), the reaction mixture was poured into 1 N HCl (50.0 mL) at 0 °C, then diluted with water (150 mL), and extracted with ethyl acetate (100 mL * 3). All organic phases were combined, washed with water (200 mL * 2) and brine (200 mL), dried over anhydrous sodium sulfate, filtered, and concentrated under reduced pressure to obtain Compound 4 as a pale yellow solid (3.47 g, crude product).

LCMS: EC20183-7-P1A1, Rₜ = 0.47 min, MS cal.: 520.21, MS observed: [M+H]⁺ = 521.3.

LCMS: EC20183-7-P1A2, Rt = 0.47 min, MS cal.: 520.21, MS observed: [M+H]⁺ = 521.3.

### 4) Synthesis of Compound T1

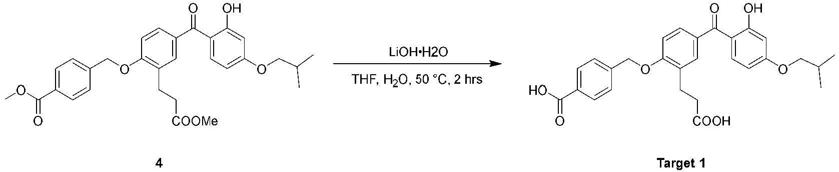

A solution of lithium hydroxide monohydrate (1.12 g, 26.6 mmol, 4.00 *eq)* in water (7.00 mL) was added to a solution of Compound 4 (3.47 g, 6.67 mmol, 1.00 *eq)* in tetrahydrofuran (7.00 mL). The mixture was stirred at 50 °C for 2 hours. When LCMS (EC20183-8-P1A) monitoring showed that Compound 4 was completely consumed and that a single major peak corresponding to the target molecular weight appeared (Rt = 0.39 min, MS cal.: 492.18, MS observed: [M+H]⁺= 493.2), the reaction mixture was poured into 1N HCl (50.0 mL), diluted with 150 mL of water, and extracted with ethyl acetate (100 mL * 3). All organic phases were combined, washed with brine (100 mL), dried over anhydrous sodium sulfate, filtered, and concentrated under reduced pressure to obtain a residue. TLC (dichloromethane:methanol = 10:1) showed four spots (R_{f} = 0.25, 0.20, 0.10, 0.00). The residue was purified by column chromatography (silica gel, dichloromethane:methanol = 100:1 to 1:1, R_{f} = 0.20) to obtain Compound T1 as a white solid (0.90 g, 1.82 mmol, 27.3% yield, 99.6% purity).

LCMS: EC20183-8-P1A, Rₜ = 0.39 min, MS cal.: 492.18, MS observed: [M+H]⁺ = 493.2.

HRMS: EC20183-8-P1F4.

HPLC: EC20183-8-P1F, Rt = 3.62 min, 99.6% purity.

¹H NMR: EC20183-8-P1B, 400 MHz, DMSO-*d₆*
*δ*: 12.57 (br s, 2H), 12.00 (s, 1H), 7.98 (d, *J =* 8.0 Hz, 2H), 7.60 (d, *J =* 8.0 Hz, 2H), 7.55 - 7.54 (m, 2H), 7.46 (d, *J=* 9.6 Hz, 1H), 7.17 (br d, *J=* 8.8 Hz, 1H), 6.53 - 6.53 (m, 2H), 5.34 (s, 2H), 3.82 (d, *J =* 6.4 Hz, 2H), 2.91 (t, *J =* 7.2 Hz, 2H), 2.57 (t, *J =* 7.6 Hz, 2H), 2.07 - 1.97 (m, 1H), 0.97 (d, *J =* 6.4 Hz, 6H).

### 1.2 Synthesis of Compound Target 2 (abbreviated as T2)

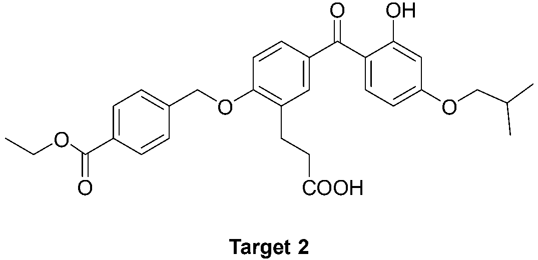

### 1) Synthesis of Compound 2

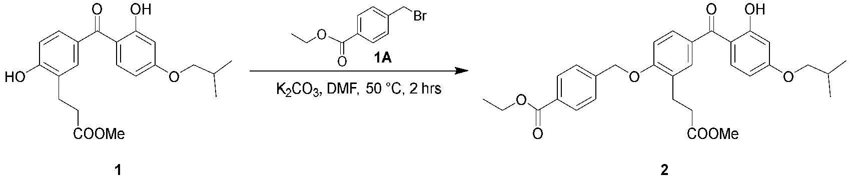

A solution of Compound 1 (2.49 g, 6.52 mmol, 1.00 *eq),* Compound 1A (1.98 g, 8.15 mmol, *1.25 eq),* and potassium carbonate (1.80 g, 13.0 mmol, 2.00 *eq)* in N,Ndimethylformamide (15.0 mL) was stirred at 50 °C for 2 hours. When LCMS (EC20184-4-P1A) monitoring showed that Compound 1 was completely consumed and that a single major peak corresponding to the target molecular weight appeared (Rₜ = 0.49 min, MS cal.: 534.23, MS observed: [M+H]⁺ = 535.3), the reaction mixture was poured into 50.0 mL of 1N hydrochloric acid at 0 °C, diluted with 150 mL of water, and extracted with ethyl acetate (100 mL * 3). All organic phases were combined, washed with water (200 mL * 2) and brine (200 mL), dried over anhydrous sodium sulfate, filtered, and concentrated under reduced pressure to obtain Compound 2 as a pale yellow solid (3.39 g, crude product).

LCMS: EC20184-4-P1A, Rt = 0.49 min, MS cal.: 534.23, MS observed: [M+H]⁺ = 535.3.

LCMS: EC20184-4-P1A1, Rt = 0.49 min, MS cal.: 534.23, MS observed: [M+H]⁺ = 535.3.

### 2) Synthesis of Compound T2

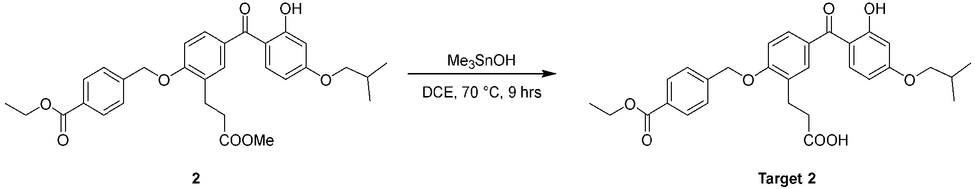

Trimethyltin hydroxide (2.58 g, 14.3 mmol, 2.25 *eq)* was added to a solution of Compound 2 (3.39 g, 6.34 mmol, 1.00 *eq)* in 1,2-dichloroethane (30.0 mL). The mixture was stirred at 70 °C for 9 hours. When LCMS (EC20184-5-P1A) monitoring showed that Compound 2 was not completely consumed and that the peak corresponding to the target molecular weight was the major peak (Rₜ = 0.44 min, MS cal.: 520.21, MS observed: [M+H]⁺ = 521.3), the reaction mixture was adjusted with 1N hydrochloric acid at 25 °C to reach a pH of 6, then diluted with 150 mL of water, and extracted with ethyl acetate (100 mL * 3). All organic phases were combined, washed with brine (100 mL), dried over anhydrous sodium sulfate, filtered, and concentrated under reduced pressure to obtain a residue. TLC (dichloromethane:methanol = 20:1) showed four spots (R_{f} = 0.35, 0.30, 0.25, 0.00). The residue was purified by silica gel column chromatography (dichloromethane:methanol = 100:1 to 1:1, R_{f} = 0.30), then the crude product was purified by preparative HPLC (TFA conditions) to obtain T2 as a white solid (0.55 g, 1.06 mmol, 16.6% yield, and 100% purity).

LCMS: EC20184-5-P1A, Rt = 0.44 min, MS cal.: 520.21, MS observed: [M+H]⁺ = 521.3.

HRMS: EC20184-5-P1E4.

HPLC: EC20184-5-P1E3, Rt = 2.85 min, 100% purity.

¹H NMR: EC20184-5-P1B, 400 MHz, DMSO-*d₆*
*δ*: 12.14 (br s, 1H), 11.99 (s, 1H), 8.00 (d, *J =* 8.4 Hz, 2H), 7.63 (d, *J =* 8.4 Hz, 2H), 7.55 - 7.53 (m, 2H), 7.47 - 7.44 (m, 1H), 7.17 (br d, *J =* 9.2 Hz, 1H), 6.54 - 6.51 (m, 2H), 5.36 (s, 2H), 4.32 (q, *J =* 7.2 Hz, 2H), 3.83 (d, *J =* 6.8 Hz, 2H), 2.91 (t, *J =* 7.6 Hz, 2H), 2.57 (t, *J =* 7.2 Hz, 2H), 2.07 - 1.98 (m, 1H), 1.32 (t, *J =* 6.8 Hz, 3H), 0.98 (d, *J =* 6.4 Hz, 6H).

### 1.3 Synthesis of Compound Target 3 (abbreviated as T3)

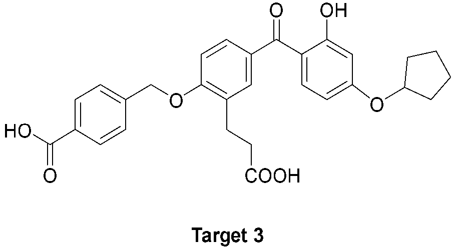

### 1) Synthesis of Compound 2B

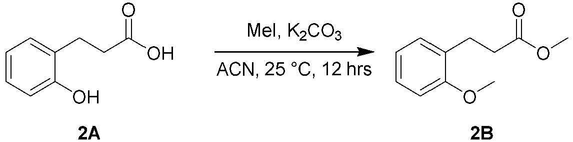

Under nitrogen protection, potassium carbonate (311 g, 2.26 mol, 3.00 *eq)* was added to a solution of Compound 2A (125 g, 752 mmol, 1.00 *eq)* in acetonitrile (1.25 L), followed by the addition of iodomethane (266 g, 1.88 mol, 117 mL, 2.50 *eq)* into the mixture. The mixture was stirred under nitrogen at 25 °C for 12 hours. When TLC (petroleum ether:ethyl acetate = 5:1) monitoring showed that Compound 2A (R_{f} = 0.15) was completely consumed and two new spots appeared (R_{f} = 0.40, 0.60), the reaction mixture was diluted with water (3.00 L) and extracted with ethyl acetate (1.00 L * 3). All organic phases were combined, washed successively with water (1.00 L * 3) and brine (1.00 L * 2), dried over anhydrous sodium sulfate, filtered, and concentrated under reduced pressure to obtain Compound 2B as a brown liquid (138 g, 710 mmol, 94.4% yield).

¹H NMR: EC6536-710-P1A1, 400 MHz, CDCl₃
*δ*: 7.26 - 7.13 (m, 2H), 6.98 - 6.81 (m, 2H), 3.91 - 3.83 (m, 3H), 3.76 - 3.67 (m, 3H), 3.00 (t, *J = 7.6* Hz, 2H), 2.67 (t, *J* = 7.6 Hz, 2H).

### 2) Synthesis of Compound 2

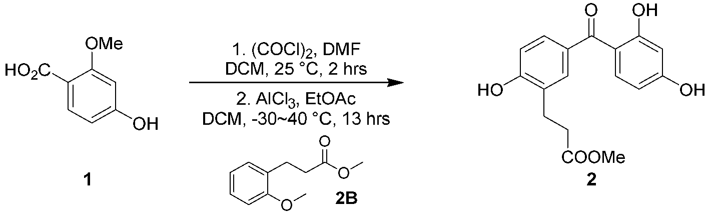

Oxalyl chloride (33.4 g, 263 mmol, 23.0 mL, 1.20 *eq)* was added dropwise to a mixed solution of Compound 1 (40.0 g, 219 mmol, 1.00 *eq)* in N,N-dimethylformamide (160 mg, 2.20 mmol, 168 µL, 0.01 *eq)* and dichloromethane (400 mL). The mixture was stirred at 25 °C for 2 hours, then concentrated under reduced pressure to obtain a residue. After dissolving the residue in dichloromethane (400 mL), a solution of aluminum chloride AlCl₃ (73.1 g, 548 mmol, 30.0 mL, 2.50 *eq)* and Compound 2B (51.1 g, 263 mmol, 1.20 *eq)* in dichloromethane (50.0 mL) was added at -30 °C. The mixture was stirred at 0 °C for 1 hour, then ethyl acetate (61.2 g, 694 mmol, 68.0 mL, 3.16 *eq)* was added dropwise, followed by the addition of aluminum chloride (161 g, 1.21 mol, 66.0 mL, 5.50 *eq)* at 0 °C. The reaction mixture was stirred at 40 °C for 12 hours (two parallel batches). When LCMS (EC6536-741-P1A1) monitoring showed that Compound 1 was completely consumed and that a single major peak corresponding to the target molecular weight appeared (Rt = 0.37 min, MS cal.: 316.09, MS observed: [M+H]⁺ = 317.0), the reaction mixture was cooled to 25 °C, poured into ice-cold 6 M hydrochloric acid, and the aqueous phase was extracted with ethyl acetate (1.00 L * 3). All organic phases were combined, washed with brine (1.00 L * 2), dried over anhydrous sodium sulfate, filtered, and concentrated under reduced pressure to obtain a residue. The crude product was purified by column chromatography (silica gel, petroleum ether:ethyl acetate = 100:1 to 0:1). TLC (petroleum ether:ethyl acetate = 1:1, R_{f} = 0.30) monitoring showed that Compound 2 was obtained as a pale yellow oil (121 g, 382 mmol, 87.1% yield).

LCMS 1: EC6536-741-P1A1, Rt = 0.37 min, MS cal.: 316.09, MS observed: [M+H]⁺ = 317.0.

LCMS 2: EC6536-741-P1A2, Rt = 0.37 min, MS cal.: 316.09, MS observed: [M+H]⁺ = 317.0.

¹H NMR: EC6536-741-P1A, 400 MHz, DMSO-*d₆*.

*δ*: 12.27 (s, 1H), 10.58 (br s, 1H), 10.37 (br s, 1H), 7.49 - 7.35 (m, 3H), 6.93 (d, *J =* 8.4 Hz, 1H), 6.45 - 6.35 (m, 2H), 3.59 (s, 3H), 2.90 - 2.83 (m, 2H), 2.61 (t, *J =* 7.6 Hz, 2H).

### 3) Synthesis of Compound Int A

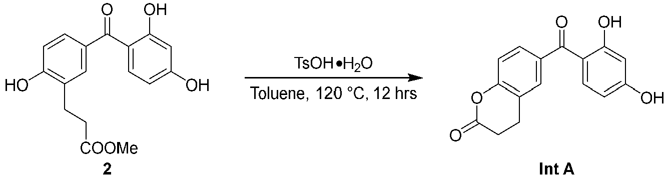

p-Toluenesulfonic acid monohydrate (3.04 g, 15.9 mmol, 0.05 *eq)* was added to a solution of Compound 2 (101 g, 319 mmol, 1.00 *eq)* in toluene (1.00 L). The mixture was stirred at 120 °C for 12 hours. When LCMS (EC6536-760-P1A) monitoring showed that Compound 2 was completely consumed and that a single major peak corresponding to the target molecular weight appeared (Rt = 0.56 min, MS cal.: 284.07, MS observed: [M+H]⁺ = 285.0), ethyl acetate (1.50 L) and saturated sodium bicarbonate solution (1.50 L) were added. The aqueous phase was extracted with ethyl acetate (1.00 L * 3). All organic phases were combined, washed with brine (1.50 L * 2), dried over anhydrous sodium sulfate, filtered, and concentrated under reduced pressure to obtain a residue. The crude product was triturated with petroleum ether/ethyl acetate (3:1, 100 mL) at 25 °C for 30 minutes, filtered, and the filter cake was concentrated under reduced pressure to obtain Compound Int A as a light brown solid (63.1 g, 220 mmol, 69.1% yield, 99.4% purity).

LCMS 1: EC6536-760-P1A, Rt = 0.56 min, MS cal.: 284.07, MS observed: [M+H]⁺ = 285.0.

LCMS 2: EC6536-760-P1A1, Rt = 0.56 min, MS cal.: 284.07, MS observed: [M+H]⁺ = 285.0, 99.4% purity.

¹H NMR: EC6536-760-P1A, 400 MHz, DMSO-*d₆*
*δ*: 12.05 (br s, 1H), 11.13 - 10.42 (m, 1H), 7.72 - 7.47 (m, 2H), 7.40 (br d, *J =* 8.4 Hz, 1H), 7.18 (br d, *J =* 8.4 Hz, 1H), 6.50 - 6.28 (m, 2H), 3.07 (br t, *J =* 7.2 Hz, 2H), 2.84 (br t, *J =* 7.2 Hz, 2H).

### 4) Synthesis of Compound Int A_3

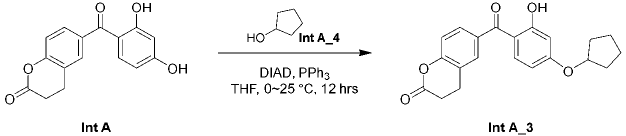

A solution of Compound Int A (25.1 g, 87.9 mmol, 1.00 *eq)* in tetrahydrofuran was cooled to 0 °C. Diisopropyl azodicarboxylate (21.3 g, 105 mmol, 20.4 mL, 1.20 *eq),* **Int-A_4** (7.95 g, 92.3 mmol, 8.38 mL, 1.05 *eq),* and triphenylphosphine (27.6 g, 105 mmol, 1.20 *eq)* were added dropwise, and the mixture was stirred at 25 °C for 12 hours. When LCMS (EC6536-768-P1A) monitoring showed that Compound Int A was completely consumed and that a single major peak corresponding to the target molecular weight appeared (Rt = 0.51 min, MS cal.: 352.13, MS observed: [M+H]⁺= 353.0), the reaction mixture was poured into a mixture of ethyl acetate (300 mL) and water (300 mL). The aqueous phase was extracted with ethyl acetate (200 mL * 3). All organic phases were combined, washed with brine (300 mL * 2), dried over anhydrous sodium sulfate, filtered, and concentrated under reduced pressure to obtain a residue. The crude product was purified by reversed-phase HPLC (neutral conditions) to obtain Compound Int **A_3** as a pale yellow solid (15.2 g, 43.1 mmol, 49.0% yield, 100% purity).

LCMS 1: EC6536-768-P1A, Rt = 0.51 min, MS cal.: 352.13, MS observed: [M+H]⁺= 353.0.

LCMS 2: EC6536-768-P1B, Rt = 0.51 min, MS cal.: 352.13, MS observed: [M+H]⁺ = 353.1, 100% purity.

¹H NMR: EC6536-768-P1A1, 400 MHz, DMSO-*d₆*
*δ*: 11.90 (s, 1H), 7.63 (s, 1H), 7.57 (dd, *J =* 2.0, 8.4 Hz, 1H), 7.43 (d, *J =* 8.8 Hz, 1H), 7.19 (d, *J =* 8.4 Hz, 1H), 6.54 - 6.44 (m, 2H), 4.91 (br t, *J =* 6.0 Hz, 1H), 3.12 - 3.05 (m, 2H), 2.89 - 2.82 (m, 2H), 2.01 - 1.88 (m, 2H), 1.78 - 1.52 (m, 6H).

### 5) Synthesis of Compound Int D

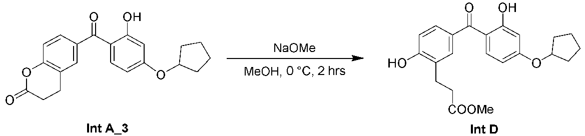

A solution of Compound **Int-A_3** (5.00 g, 14.1 mmol, 1.00 *eq)* in methanol (50.0 mL) was cooled to 0 °C, then a solution of sodium methoxide (1.84 g, 34.0 mmol, 2.40 *eq)* in methanol (10.0 mL) was added. The mixture was stirred at 0 °C for 2 hours. When LCMS (EC6536-769-P1A1) monitoring showed that Compound **Int-A_3** was completely consumed and that a single major peak corresponding to the target molecular weight appeared (Rt = 0.49 min, MS cal.: 384.16, MS observed: [M+H]⁺ = 385.1), the reaction mixture was poured into water (200 mL), and the pH was adjusted to 5 with 1M hydrochloric acid. It was then extracted with ethyl acetate (200 mL * 3). All organic phases were combined, washed with brine (200 mL * 2), dried over anhydrous sodium sulfate, filtered, and concentrated under reduced pressure to obtain Compound **Int-D** as a pale yellow oil (4.70 g, 12.2 mmol, 86.1% yield, crude product).

LCMS 1: EC6536-769-P1A1, Rt = 0.49 min, MS cal.: 384.16, MS observed: [M+H]⁺ = 385.1.

LCMS 2: EC6536-769-P1A4, Rt = 0.50 min, MS cal.: 384.16, MS observed: [M+H]⁺ = 385.0.

### 6) Synthesis of Compound 4

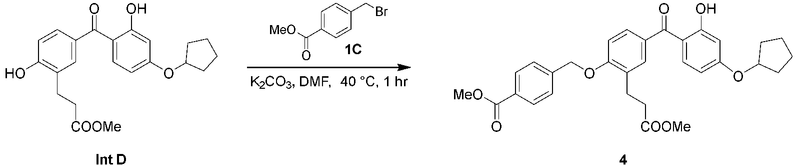

Potassium carbonate (1.44 g, 10.4 mmol, 2.00 *eq)* was added to a solution of Compound Int D (2.00 g, 5.20 mmol, 1.00 *eq)* and Compound 1C (1.19 g, 5.20 mmol, 1.00 *eq)* in N,N-dimethylformamide (20.0 mL). The mixture was stirred at 40 °C for 1 hour. When TLC (petroleum ether:ethyl acetate = 2:1) monitoring showed that Compound Int D was completely consumed and a new spot appeared, the reaction mixture was poured into water (50.0 mL), adjusted to pH 2 with 1M hydrochloric acid, and extracted with ethyl acetate (30.0 mL * 3). All organic phases were combined, washed with brine (30.0 mL * 2), dried over anhydrous sodium sulfate, filtered, and concentrated under reduced pressure to obtain Compound 4 as a pale yellow oil (1.70 g, 3.14 mmol, 60.3% yield).

¹H NMR: EC6536-773-P1A, 400 MHz, DMSO-*d₆*
*δ*: 12.01 (s, 1H), 8.05 - 7.87 (m, 2H), 7.69 - 7.49 (m, 4H), 7.43 (br d, *J =* 8.8 Hz, 1H), 7.21 - 7.08 (m, 1H), 6.72 - 6.40 (m, 2H), 5.42 - 5.26 (m, 2H), 4.90 (br s, 1H), 4.44 - 4.26 (m, 2H), 3.62 - 3.47 (m, 3H), 3.03 - 2.82 (m, 2H), 2.77 - 2.56 (m, 2H), 1.96 - 1.87 (m, 2H), 1.72 (br s, 2H), 1.60 (br s, 2H), 1.37 - 1.29 (m, 3H).

### 7) Synthesis of Compound T3

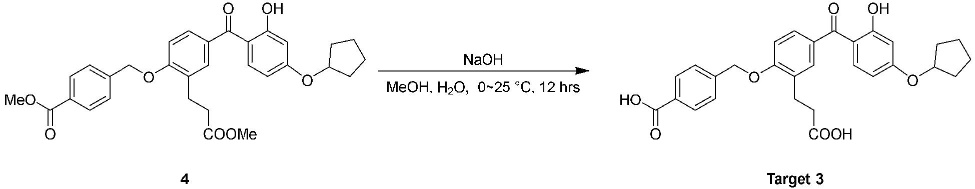

A solution of Compound 4 (1.70 g, 3.14 mmol, 1.00 *eq)* in methanol (20.0 mL) was cooled to 0 °C, then a solution of sodium hydroxide (627 mg, 15.6 mmol, 5.00 *eq)* in water (5.00 mL) was added. The mixture was stirred at 25 °C for 12 hours. When LCMS (EC6536-776-P1A3) monitoring showed that Compound 4 was completely consumed and that a single major peak corresponding to the target molecular weight appeared (Rt = 0.51 min, MS cal.: 504.18, MS observed: [M+H]⁺ = 505.3), the reaction mixture was poured into water (50.0 mL), adjusted to pH 5 with 1M hydrochloric acid, and extracted with ethyl acetate (30.0 mL * 3). All organic phases were combined, washed with brine, dried over anhydrous sodium sulfate, filtered, and concentrated under reduced pressure to obtain a residue. The residue was purified by column chromatography (silica gel, dichloromethane:methanol = 1:0 to 10:1). TLC monitoring (petroleum ether:ethyl acetate = 1:2, R_{f} = 0.65) showed that a crude product was obtained. The crude product was triturated with dichloromethane (20.0 mL) at 25 °C for 30 minutes, filtered, and the filter cake was concentrated under reduced pressure to obtain Compound **Target** 3 as a white solid (510 mg, 1.01 mmol, 32.1% yield, 100% purity).

LCMS: EC6536-776-P1A3, Rt = 0.51 min, MS cal.: 504.18, MS observed: [M+H]⁺ = 505.3.

HRMS: EC20403-1.

HPLC: EC20403-1-P1A2, Rt = 3.52 min, 100% purity.

¹H NMR: EC20403-1-P1A7, 400 MHz, DMSO-*d₆*
*δ*: 13.79 - 12.12 (m, 2H), 12.04 (br s, 1H), 7.98 (d, *J =* 8.4 Hz, 2H), 7.60 (br d, *J =* 8.0 Hz, 2H), 7.57 - 7.50 (m, 2H), 7.45 (br d, *J =* 8.8 Hz, 1H), 7.17 (br d, *J =* 9.2 Hz, 1H), 6.54 - 6.44 (m, 2H), 5.35 (s, 2H), 4.91 (br t, *J =* 5.2 Hz, 1H), 2.92 (br t, *J =* 7.2 Hz, 2H), 2.57 (br t, *J =* 7.2 Hz, 2H), 2.01 - 1.88 (m, 2H), 1.79 - 1.65 (m, 4H), 1.59 (br d, *J =* 2.4 Hz, 2H).

### 1.4 Synthesis of Compound Target 4 (abbreviated as T4)

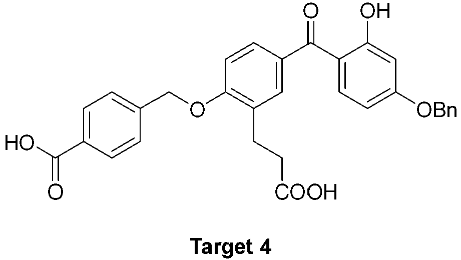

### 1) Synthesis of Compound 2B

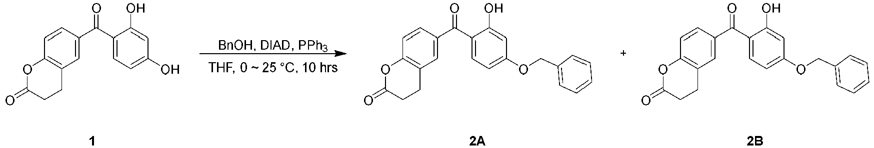

A solution of Compound 1 (5.00 g, 17.4 mmol, 1.00 *eq),* benzyl alcohol (2.08 g, 19.2 mmol, 1.99 mL, 1.10 *eq),* and triphenylphosphine (5.50 g, 20.9 mmol, 1.20 *eq)* in tetrahydrofuran (50.0 mL) was cooled to 0 °C. Diisopropyl azodicarboxylate (4.24 g, 20.9 mmol, 4.07 mL, 1.20 *eq)* was added dropwise under nitrogen protection, and the mixture was stirred at 25 °C for 10 hours. When LCMS (EC20185-4-P1A1) monitoring showed that Compound 1 was completely consumed and that a single major peak corresponding to the target molecular weight appeared (Rt = 0.35 min, MS cal.: 374.12, MS observed: [M+H]⁺ = 375.0), the reaction was quenched with water (200 mL) at 25 °C, and the mixture was extracted with ethyl acetate (150 mL * 3). All organic phases were combined, washed with brine (200 mL), dried over anhydrous sodium sulfate, filtered, and concentrated under reduced pressure to obtain a residue. The crude product was purified by reversed-phase HPLC (neutral conditions) to obtain Compound 2A as a pale yellow solid (2.10 g, 4.81 mmol, 27.4% yield, 85.7% purity) and Compound 2B as a pale yellow solid (2.89 g, 4.27 mmol, 24.4% yield, 55.3% purity).

LCMS: EC20185-4-P1A1, Rt = 0.35 min, MS cal.: 374.12, MS observed: [M+H]⁺ = 375.0.

LCMS: EC20185-4-P1C1, Rt = 0.51 min, MS cal.: 374.12, MS observed: [M+H]⁺ = 375.0.

LCMS: EC20185-4-P1D1, Rt = 0.50 min, MS cal.: 374.12, MS observed: [M+H]⁺ = 375.0.

HPLC: EC20185-4-P1C2, Rt = 4.31 min, 85.7% purity.

HPLC: EC20185-4-P1C3, Rt = 4.31 min, 55.3% purity.

### 2) Synthesis of Compounds 3B and 3C

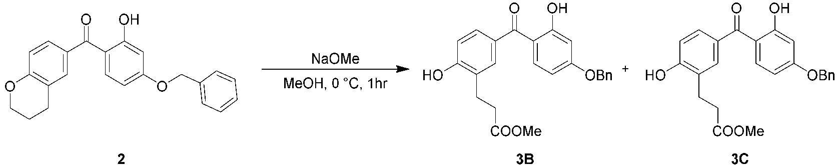

A solution of Compound 2 (2.89 g, 4.27 mmol, 1.00 *eq)* in methanol (15.0 mL) was cooled to 0 °C, then a solution of sodium methoxide (553 mg, 10.2 mmol, 2.40 *eq)* in methanol (20.0 mL) was added. The mixture was stirred at 0 °C for 1 hour. When TLC (petroleum ether:ethyl acetate = 3:1) monitoring showed that Compound 2 (R_{f} = 0.50) was completely consumed and two new spots appeared, the reaction mixture was poured into 1 M hydrochloric acid (50.0 mL), diluted with water (150 mL), and extracted with ethyl acetate (100 mL * 3). All organic phases were combined, washed with brine, dried over anhydrous sodium sulfate, filtered, and concentrated under reduced pressure to obtain a residue. The residue was purified by column chromatography (silica gel, petroleum ether:ethyl acetate = 100:1 to 1:1, R_{f} = 0.30) to obtain Compound 3B as a white solid (0.88 g, 2.05 mmol, 48.1% yield, 94.9% purity) and Compound 3C as a white solid (0.75 g, 1.55 mmol, 36.2% yield, 83.8% purity).

LCMS: EC20185-6-P1A3, Rt = 0.49 min, MS cal.: 406.14, MS observed: [M+H]⁺ = 407.1.

LCMS: EC20185-6-P1A5, Rₜ = 0.48 min, MS cal.: 406.14, MS observed: [M+H]⁺ = 407.2.

HPLC: EC20185-6-P1A4, Rt = 3.49 min, 94.9% purity.

HPLC: EC20185-6-P1A6, Rt = 3.50 min, 83.8% purity.

### 3) Synthesis of Compound 4

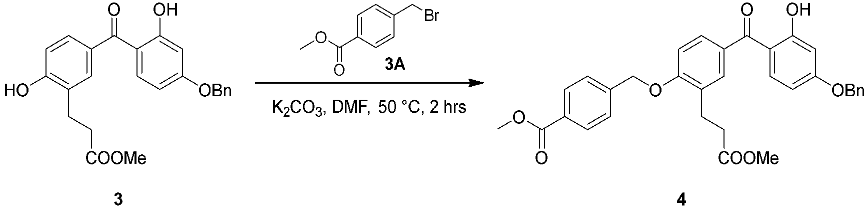

A solution of Compound 3 (1.63 g, 3.60 mmol, 1.00 eq), Compound 3A (1.03 g, 4.50 mmol, *1.25 eq),* and potassium carbonate (995 mg, 7.20 mmol, 2.00 *eq)* in N,N-dimethylformamide (5.00 mL) was heated to 50 °C and stirred for 2 hours. When LCMS (EC20185-12-P1A) monitoring showed that Compound 3 was completely consumed and a single major peak corresponding to the target molecular weight appeared (Rt = 0.43 min, MS cal.: 554.19, MS observed: [M+H]⁺ = 555.2), the reaction mixture was poured into 1 M HCl (20.0 mL) at 0 °C, then diluted with water (50.0 mL), and extracted with ethyl acetate (30.0 mL * 3). All organic phases were combined, washed successively with water (30.0 mL * 2) and brine (30.0 mL), dried over anhydrous sodium sulfate, filtered, and concentrated under reduced pressure to obtain a residue. TLC (petroleum ether:ethyl acetate = 2:1) monitoring showed four spots (R_{f} = 0.50, 0.30, 0.25, 0.00). The residue was purified by column chromatography (silica gel, petroleum ether:ethyl acetate = 100:1 to 1:1, R_{f} = 0.30). The crude product was purified by reversed-phase HPLC (neutral conditions) to obtain Compound 4 as a white solid (0.68 g, 1.23 mmol, 34.0% yield, 100% purity).

LCMS: EC20185-12-P1A, Rₜ = 0.43 min, MS cal.: 554.19, MS observed: [M+H]⁺ = 555.2.

LCMS: EC20185-12-P1E, Rt = 0.43 min, MS cal.: 554.19, MS observed: [M+H]⁺ = 555.2.

HPLC: EC20185-12-P1E1, Rt = 3.04 min, 100% purity.

¹H NMR: EC20185-12-P1B, 400 MHz, DMSO-*d₆*
*δ*: 11.90 (s, 1H), 8.00 (d, *J =* 8.0 Hz, 2H), 7.62 (d, *J =* 8.4 Hz, 2H), 7.56 - 7.54 (m, 2H), 7.47 - 7.33 (m, 6H), 7.17 (d, *J =* 8.4 Hz, 1H), 6.63 - 6.58 (m, 2H), 5.35 (s, 2H), 5.19 (s, 2H), 3.86 (s, 3H), 3.56 (s, 3H), 2.94 (t, *J =* 7.2 Hz, 2H), 2.65 (t, *J =* 7.6 Hz, 2H).

### 4) Synthesis of Compound T4

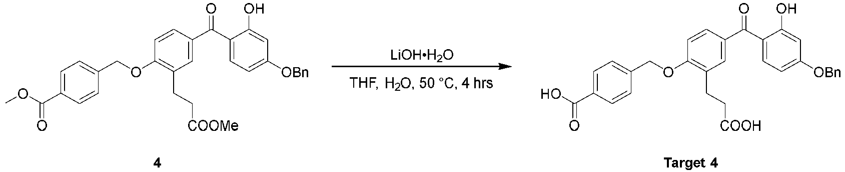

A solution of lithium hydroxide monohydrate (205 mg, 4.90 mmol, 4.00 *eq)* in water (5.00 mL) was added to a solution of Compound 4 (0.68 g, 1.23 mmol, 1.00 *eq)* in tetrahydrofuran (5.00 mL). The mixture was stirred at 50 °C for 4 hours. When LCMS (EC20185-15-P1A4) monitoring showed that Compound 4 was completely consumed and that a single major peak corresponding to the target molecular weight appeared (Rt = 0.27 min, MS cal.: 526.16, MS observed: [M+H]⁺ = 527.2), the reaction mixture was adjusted to pH 1 with 1M hydrochloric acid at 25 °C, and then stirred at this temperature for 1 hour. The reaction mixture was filtered, and the filter cake was collected, dispersed in deionized water, and then lyophilized to obtain Compound Target 4 as a white solid (0.51 g, 968 µmol, 79.0% yield, 100% purity).

LCMS: EC20185-15-P1A4, Rt = 0.27 min, MS cal.: 526.16, MS observed: [M+H]⁺ = 527.2.

HRMS: EC20185-15-P1E.

HPLC: EC20185-15-P1C3, Rₜ = 3.53 min, 100% purity.

¹H NMR: EC20185-15-P1B2, 400 MHz, DMSO-*d₆*
*δ*: 12.56 (br s, 2H), 11.95 (s, 1H), 7.98 (d, *J =* 8.4 Hz, 2H), 7.61 (d, *J =* 8.0 Hz, 2H), 7.56 - 7.54 (m, 2H), 7.49 - 7.45 (m, 3H), 7.41 (t, *J =* 6.8 Hz, 2H), 7.36 - 7.33 (m, 1H), 7.17 (d, *J =* 8.8 Hz, 1H), 6.64 - 6.59 (m, 2H), 5.34 (s, 2H), 5.19 (s, 2H), 2.91 (t, *J =* 6.8 Hz, 2H), 2.57 (t, *J =* 7.6 Hz, 2H).

### 1.5 Synthesis of Compound Target 5 (abbreviated as T5)

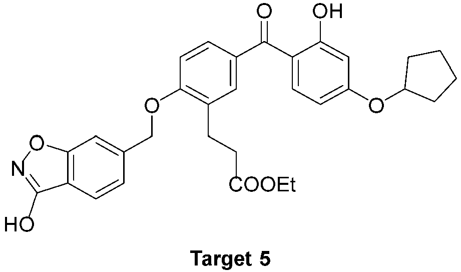

### 1) Synthesis of Compound 2

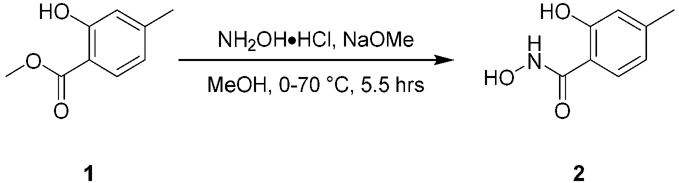

To a methanol solution of NH₂OH•HCl (31.3 g, 451 mmol, 3.00 *eq)* under nitrogen protection in an ice bath, a methanol solution of NaOMe (135 g, 752 mmol, 30.0% purity, 5.00 *eq)* was slowly added dropwise over at least 10 minutes. The reaction mixture was allowed to warm to room temperature and reacted for another 10 minutes. The reaction mixture was then cooled again in an ice bath, and a solution of Compound 1 (25.0 g, 150 mmol, 1.00 *eq)* in methanol (80 mL) was slowly added dropwise. The mixture was warmed to room temperature and reacted for 1 hour, then heated to 70 °C and continuously reacted for another 4 hours. LCMS 1 (EC6536-775-P1A1) showed that Compound 1 was completely consumed with a yield of 91.7% (Rt = 0.19 min, MS cal.: 167.0, MS observed: [M+H]⁺ = 168.0). The reaction mixture was poured into 300 mL of ice water, adjusted to pH 5 with 1M HCl, and filtered under suction. The filter cake was collected and washed successively with 500 mL of water, diisopropyl ether, and n-hexane. The filter cake was concentrated under reduced pressure to obtain Compound 2 as a white solid (21.7 g, 129 mmol, yield 85.7%, purity 99.4%), which was confirmed by LCMS (EC6536-775-P1A2) and ¹H NMR (EC6536-775-P1Al).

LCMS 1: EC6536-775-P1A1, Rₜ = 0.19 min, MS cal.: 167.0, MS observed: [M+H]⁺ = 168.0.

LCMS: EC6536-775-P1A2, Rt = 0.20 min, MS cal.: 167.0, MS observed: [M+H]⁺ = 168.2.

¹H NMR: EC6536-775-P1A1, 400 MHz, DMSO-*d₆*
*δ*: 14.03 - 10.20 (m, 2H), 10.09 - 8.59 (m, 1H), 7.56 (br d, *J =* 8.0 Hz, 1H), 6.94 - 6.41 (m, 2H), 2.25 (s, 3H).

### 2) Synthesis of Compound 3

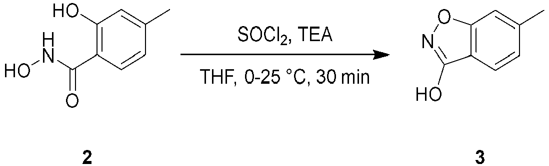

Compound 2 (21.7 g, 129 mmol, 1.00 *eq)* was dissolved in 400 mL of THF. Under an ice bath and nitrogen protection, TEA (78.3 g, 774 mmol, 107 mL, 6.00 *eq)* and SOCl₂ (23.0 g, 193 mmol, 14.0 mL, 1.50 *eq)* were slowly added dropwise. The reaction mixture was allowed to react at room temperature for 30 minutes. LCMS 1 (EC20395-3-P1A2) showed that Compound 2 was completely consumed, and the yield of Compound 3 was 93.2% (Rt = 0.29 min, MS cal.: 149.0, MS observed: [M+H]⁺ = 150.0). The reaction mixture was poured into 200 mL of ice water, and adjusted to pH 1 with 1M aqueous HCl. The precipitated yellow solid was Compound 3 (14.6 g, 97.6 mmol, yield 75.6%, purity 99.7%), and its structure was confirmed by LCMS (EC20395-3-P1B1) and ¹H NMR (EC20395-3-P1A1).

LCMS 1: EC20395-3-P1A2, Rt = 0.29 min, MS cal.: 149.0, MS observed: [M+H]⁺ = 150.0.

LCMS: EC20395-3-P1B1, Rt = 0.29 min, MS cal.: 149.0, MS observed: [M+H]⁺ = 150.2.

¹H NMR: EC20395-3-P1A1, 400 MHz, DMSO-*d₆*
*δ*: 7.60 (d, *J= 8.0* Hz, 1H), 7.34 (s, 1H), 7.13 (d, *J=* 8.0 Hz, 1H), 2.43 (s, 3H).

### 3) Synthesis of Compounds 4 and 4A

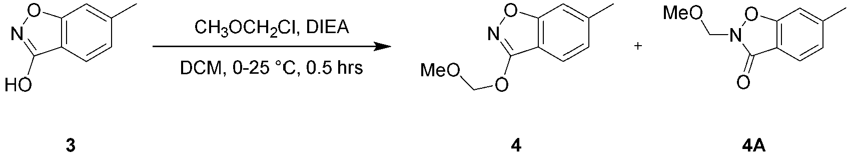

Compound 3 (9.60 g, 64.1 mmol, 1.00 *eq)* was dissolved in 100 mL of DCM. Under an ice bath and nitrogen protection, DIEA (12.4 g, 96.2 mmol, 16.7 mL, 1.50 *eq)* and CH₃OCH₂Cl (18.4 g, 228 mmol, 17.3 mL, 3.56 *eq)* were slowly added dropwise sequentially. The reaction mixture was stirred at room temperature for 30 minutes. LCMS (EC6536-790-P1A2) showed that Compound 3 was completely consumed, and the yield of the target compound was 41.5% (Rt = 0.67 min, MS cal.: 193.0, MS observed: [M+H]⁺ = 194.1). 200 mL of ice water was added to the reaction mixture. The organic phase was washed with 200 mL of water and 200 mL of saturated brine, and dried over anhydrous sodium sulfate. After filtration and evaporation to dryness, a solid was obtained. The solid was purified by reversed-phase preparative liquid chromatography (neutral conditions) to obtain Compound 4 as a yellow oil (4.14 g, 20.0 mmol, yield 31.2%, purity 93.7%), which was confirmed by ¹H NMR 1 (EC6536-790-P1A1) and LCMS 1 (EC6536-790-P1D1). Compound 4A was also obtained as a yellow oil (4.25 g, 21.6 mmol, yield 33.7%, purity 98.3%), and its structure was confirmed by LCMS 2 (EC6536-790-P2A1) and ¹H NMR 2 (EC6536-790-P1A2).

LCMS: EC6536-790-P1A2, Rt = 0.67 min, MS cal.: 193.0, MS observed: [M+H]⁺ = 194.1.

LCMS 1: EC6536-790-P1D1, Rt = 0.66 min, MS cal.: 193.0, MS observed: [M+H]⁺ = 194.0.

LCMS 2: EC6536-790-P2A1, Rt = 0.56 min, MS cal.: 193.0, MS observed: [M+H]⁺ = 194.0.

¹H NMR 1: EC6536-790-P1A1, 400 MHz, CDCl₃
*δ*: 7.54 (d, *J= 8.0* Hz, 1H), 7.25 (s, 1H), 7.11 (d, *J =* 8.0 Hz, 1H), 5.55 (s, 2H), 3.64 (s, 3H), 2.51 (s, 3H).

¹H NMR 2: EC6536-790-P1A2, 400 MHz, CDCl₃
*δ*: 7.72 (br d, *J =* 8.0 Hz, 1H), 7.10 (br d, *J =* 7.8 Hz, 1H), 7.04 (br s, 1H), 5.31 (s, 2H), 3.44 (s, 3H), 2.48 (s, 3H).

### 4) Synthesis of Compound 5

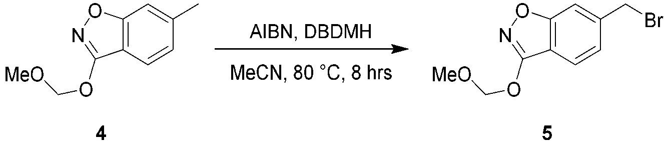

Compound **4** (2.07 g, 10.0 mmol, 1.00 *eq*) was dissolved in 30 mL of acetonitrile. AIBN (164 mg, 1.00 mmol, 0.10 *eq*) and DBDMH (3.73 g, 13.0 mmol, 1.30 *eq*) were added. The reaction mixture was reacted at 80 °C for 8 hours. LCMS (EC20395-14-P2C4) showed that Compound **4** was completely consumed, and the yield of the target compound was 60.5% (Rt = 0.69 min, MS cal.: 270.9, MS observed: [M+H]⁺ = 271.9). The reaction mixture was treated with 50 mL of ice water and separated. The organic phase was washed successively with aqueous solution of Na₂S₂O₃ (50.0 mL), aqueous solution of NaHCO₃ (50.0 mL), and saturated brine (50.0 mL), dried over anhydrous sodium sulfate, filtered, and evaporated to dryness under reduced pressure to obtain Compound 5 as a yellow oil (5.27 g, crude product).

LCMS: EC20395-14-P2C4, Rₜ = 0.69 min, MS cal.: 270.9, MS observed: [M+H]⁺ = 271.9.

### 5) Synthesis of Compound Int E

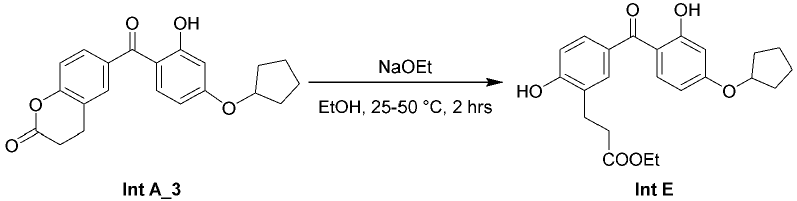

**Int A_3** (6.00 g, 17.0 mmol, 1.00 *eq*) was dissolved in 50 mL of ethanol. NaOEt (0.29 M, 146 mL, 2.50 *eq*) was added at room temperature. The reaction mixture was reacted at 50 °C for 2 hours. LCMS (EC6536-771-P1A1) showed that **Int A_3** was completely consumed, and the yield was 91.8% (Rt = 0.52 min, MS cal.: 398.1, MS observed: [M+H]⁺ = 399.2). The reaction mixture was poured into 200 mL of pH 5 aqueous hydrochloric acid and extracted with ethyl acetate (200 mL * 3). The organic phases were combined, washed twice with saturated brine, dried over anhydrous sodium sulfate, filtered, and concentrated under reduced pressure to obtain Compound **Int E** as a yellow oil (6.78 g, 16.6 mmol, yield 97.8%, purity 97.9%), which was confirmed by LCMS 1 (EC6536-771-P1A3).

LCMS: EC6536-771-P1A1, Rₜ = 0.52 min, MS cal.: 398.1, MS observed: [M+H]⁺ = 399.2.

LCMS 1: EC6536-771-P1A3, Rₜ = 0.53 min, MS cal.: 398.1, MS observed: [M+H]⁺ = 399.2.

### 6) Synthesis of Compound 6

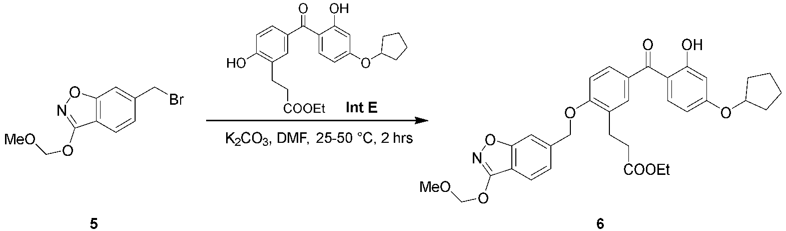

Compound **5** (5.27 g, 19.3 mmol, 1.43 *eq*) was dissolved in 50 mL of DMF and stirred at room temperature. Compound **Int E** (5.50 g, 13.5 mmol, 1.00 *eq*) and K₂CO₃ (3.74 g, 27.0 mmol, 2.00 *eq*) were added. The reaction mixture was heated to 50 °C and stirred for 2 hours. LCMS (EC20395-15-P1A3) showed that Compound **5** was completely consumed with a yield of 36.9% (Rt = 0.93 min, MS cal.: 589.2, MS observed: [M+H]⁺ = 590.4). The reaction mixture was treated with 100 mL of ethyl acetate and 100 mL of water. After separation, the organic phase was washed with 100 mL of water and 100 mL of saturated brine, and dried over anhydrous sodium sulfate. After filtration and evaporation to dryness under reduced pressure, a crude product was obtained, which was purified by reversed-phase HPLC to obtain Compound **6** as a yellow oil (3.17 g, 5.06 mmol, yield 37.4%, purity 94.2%), and it was confirmed by LCMS 1 (EC20395-15-P1D16) and HPLC (EC20395-15-P1A23).

LCMS: EC20395-15-P1A3, Rₜ = 0.93 min, MS cal.: 589.2, MS observed: [M+H]⁺ = 590.4.

LCMS 1: EC20395-15-P1D16, Rₜ = 2.10 min, MS cal.: 589.2, MS observed: [M+H]⁺ = 590.1.

HPLC: EC20395-15-P1A23, Rₜ = 4.01 min, purity: 94.2%.

### 7) Synthesis of Compound T5

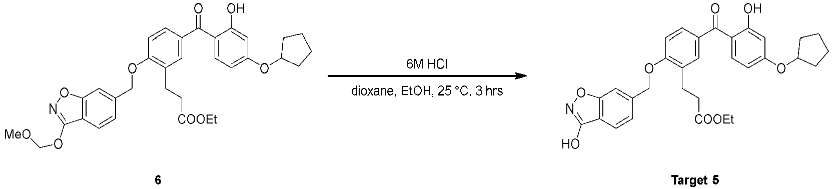

Compound **6** (2.00 g, 3.20 mmol, 1.00 *eq*) was dissolved in a mixed solution of 10.0 mL of dioxane and 10.0 mL of ethanol. 6 M HCl (14.5 mL, 27.3 *eq*) was added at room temperature, and the reaction mixture was stirred at room temperature for 3 hours. LCMS (EC20395-17-P1A4) showed that Compound **6** was completely consumed with a yield of 92.1% (Rt = 0.66 min, MS cal.: 545.2, MS observed: [M+H]⁺ = 546.4). The reaction mixture was treated with 100 mL of ethyl acetate and 100 mL of water. The liquid phases were separated. The organic phase was washed successively with 100 mL of water and 100 mL of saturated brine, dried over anhydrous sodium sulfate, filtered, and evaporated to dryness under reduced pressure. The crude product was purified by reversed-phase preparative liquid chromatography to obtain the target compound **T5** as a white solid (750 mg, 1.37 mmol, yield 67.9%, purity 99.4%). Its structure was confirmed by LCMS 1 (EC20395-18-P1A1), HPLC (EC20395-18-P1A2), HRMS (EC20395-18-P1A1), ¹H NMR (EC20395-18-P1A3), and ¹³C NMR (EC20395-18-P1A2).

LCMS: EC20395-17-P1A4, Rₜ = 0.66 min, MS cal.: 545.2, MS observed: [M+H]⁺ = 546.4.

LCMS 1: EC20395-18-P1A1, Rₜ = 0.67 min, MS cal.: 545.2, MS observed: [M+H]⁺ = 546.4.

HRMS: EC20395-18-P1A1.

HPLC: EC20395-18-P1A2, Rₜ = 3.82 min, purity: 99.4%.

¹H NMR: EC20395-18-P1A3, 400 MHz, CDCl₃.

¹³C NMR: EC20395-18-P1A2.

*δ*: 12.69 (br s, 1H), 7.83 (d, *J =* 8.0 Hz, 1H), 7.58 (d, *J =* 2.2 Hz, 1H), 7.57 - 7.53 (m, 2H), 7.51 (d, *J* = 9.0 Hz, 1H), 7.39 (d, *J* = 8.4 Hz, 1H), 6.96 (d, *J=* 8.4 Hz, 1H), 6.49 (d, *J* = 2.4 Hz, 1H), 6.38 (dd, *J =* 2.4, 8.9 Hz, 1H), 5.35 (s, 2H), 4.83 (tt, *J =* 2.8, 5.8 Hz, 1H), 4.15 (q, *J* = 7.2 Hz, 2H), 3.11 (t, *J* = 7.8 Hz, 2H), 2.70 (t, *J* = 7.6 Hz, 2H), 2.00 - 1.76 (m, 6H), 1.71 - 1.60 (m, 2H), 1.24 (t, *J =* 7.2 Hz, 3H).

### 1.6 Synthesis of Compound Target 6 (abbreviated as T6)

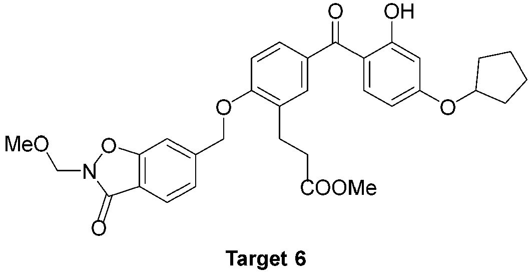

### 1) Synthesis of Compound 7

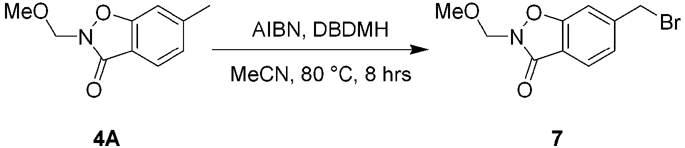

Compound **4A** (2.00 g, 10.3 mmol, 1.00 *eq*) was dissolved in 20 mL of acetonitrile and stirred at room temperature. AIBN (169 mg, 1.04 mmol, 0.10 *eq*) and DBDMH (3.85 g, 13.4 mmol, 1.30 *eq*) were added. The reaction mixture was reacted at 80 °C for 8 hours. LCMS (EC20419-5-P2A4) showed that **4A** was completely consumed with a yield of 32.6% (Rt = 0.58 min, MS cal.: 270.9, MS observed: [M+H]⁺ = 271.9). The reaction mixture was treated with 50 mL of ice water, and the phases were separated. The organic phase was washed with aqueous Na₂S₂O₃ (50.0 mL), aqueous NaHCO₃ (50.0 mL), and saturated brine (50.0 mL), and dried over anhydrous sodium sulfate. After filtration and evaporation to dryness under reduced pressure, 5.74 g of crude Compound **7** was obtained as a yellow oil.

LCMS: EC20419-5-P2A4, Rₜ = 0.58 min, MS cal.: 270.9, MS observed: [M+H]⁺ = 271.9.

### 2) Synthesis of Compound T6

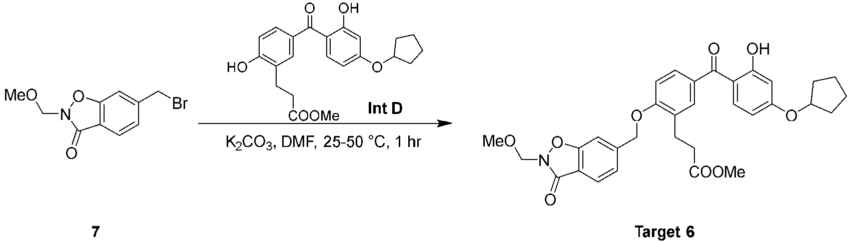

Compound **7** (5.74 g, 21.1 mmol, 1.35 *eq*) was dissolved in 50 mL of DMF and stirred at room temperature. **Int D** (6.41 g, 15.6 mmol, 1.00 *eq*) and K₂CO₃ (4.31 g, 31.2 mmol, 2.00 *eq*) were added. The reaction mixture was heated to 50 °C and reacted for 1 hour. LCMS (EC20419-6-P1A) showed that Compound **7** was completely consumed with a yield of 17.5% (Rt = 0.87 min, MS cal.: 575.2, MS observed: [M+H]⁺ = 576.2). The reaction mixture was diluted with 50 mL of water and extracted with ethyl acetate (50.0 mL * 3). The organic phases were combined, washed with water (50.0 mL * 5) and 100 mL of saturated brine, dried over anhydrous sodium sulfate, filtered, and concentrated under reduced pressure to obtain a solid, which was purified by reversed-phase preparative liquid chromatography to obtain the target compound **T6** as a yellow solid (683 mg, 1.18 mmol, yield 7.56%, purity 99.5%). Its structure was confirmed by HPLC (EC20419-6-P1D2), ¹H NMR (EC20419-6-P1A1), ¹³C NMR (EC20419-6-P1A2), and HRMS (EC20419-6-P1A1).

LCMS: EC20419-6-P1A, Rₜ = 0.87 min, MS cal.: 575.2, MS observed: [M+H]⁺ = 576.2.

HRMS: EC20419-6-P1A1.

HPLC: EC20419-6-P1D2, Rₜ = 4.19 min, purity: 99.5%.

¹³C NMR: EC20419-6-P1A2.

¹H NMR: EC20419-6-P1A1, 400 MHz, CDCl₃.

*δ*: 12.68 (s, 1H), 7.90 (d, *J =* 8.0 Hz, 1H), 7.57 (d, *J =* 2.0 Hz, 1H), 7.56 - 7.53 (m, 1H), 7.51 (d, *J =* 8.8 Hz, 1H), 7.40 (s, 1H), 7.35 (d, *J =* 8.0 Hz, 1H), 6.94 (d, *J =* 8.4 Hz, 1H), 6.49 (d, *J =* 2.4 Hz, 1H), 6.38 (dd, *J =* 2.4, 8.8 Hz, 1H), 5.36 (s, 2H), 5.31 (s, 2H), 4.83 (td, *J =* 2.8, 5.6 Hz, 1H), 3.69 (s, 3H), 3.47 (s, 3H), 3.10 (t, *J =* 7.6 Hz, 2H), 2.71 (t, *J =* 7.6 Hz, 2H), 2.01 - 1.76 (m, 6H), 1.71 - 1.64 (m, 2H).

### 1.7 Synthesis of Compound Target 7 (abbreviated as T7)

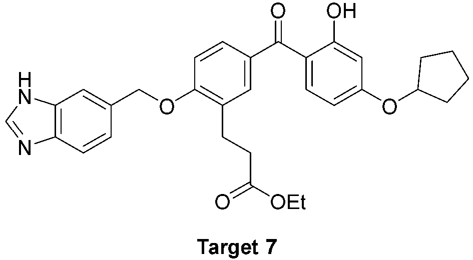

### Synthesis of Compound T7

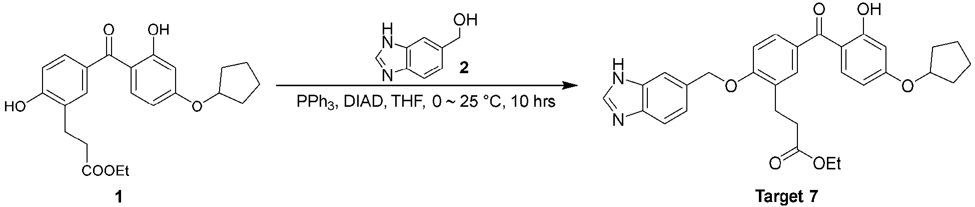

At 0 °C, Compound **2** (1.78 g, 12.0 mmol, 1.10 *eq*), Compound 1 (4.45 g, 10.9 mmol, 1.00 *eq),* and triphenylphosphine (3.44 g, 13.1 mmol, 1.20 *eq*) were dissolved in tetrahydrofuran (40.0 mL). Diisopropyl azodicarboxylate (2.65 g, 13.1 mmol, 2.54 mL, 1.20 *eq*) was added dropwise, and the mixture was stirred at 25 °C for 10 hours. When LCMS (EC19915-16-P1A) monitoring showed that Compound **1** (Rt = 0.400 min) was not completely consumed and that the peak corresponding to the target molecular weight was the major peak (Rt = 0.32 min, MS cal.: 528.23, MS observed: [M+H] + = 529.3), the reaction mixture was concentrated under reduced pressure to obtain a residue. TLC (dichloromethane:methanol = 15:1) showed 5 spots (R_{f} = 0.50, 0.30, 0.20, 0.15, 0.00). The residue was purified by column chromatography (silica gel, dichloromethane:methanol = 100:1 to 1:1, R_{f} = 0.20), and then the crude product was purified by preparative HPLC (trifluoroacetic acid conditions) to obtain Compound **Target 7** as a white solid (0.65 g, 1.23 mmol, yield 11.2%, purity 100%).

LCMS: EC19915-16-P1A, Rₜ = 0.32 min, MS cal.: 528.23, MS observed: [M+H]⁺ = 529.3.

HRMS: EC21255-1-P1A2.

HPLC: EC21255-1-P1A1, Rₜ = 3.03 min, 100% purity.

¹H NMR: EC21255-1-P1B, 400 MHz, DMSO*-d₆*

*δ*: 11.98 (s, 1H), 9.32 (s, 1H), 7.93 (s, 1H), 7.86 (d, *J =* 8.4 Hz, 1H), 7.64 (dd, J1 = 1.2 Hz, J2 = 8.4 Hz, 1H), 7.57 - 7.53 (m, 2H), 7.42 (d, *J =* 8.8 Hz, 1H), 7.23 (d, *J =* 8.8 Hz, 1H), 6.50 - 6.45 (m, 2H), 5.43 (s, 2H), 4.90 (t, *J =* 5.6 Hz, 1H), 4.00 (q, *J =* 7.2 Hz, 2H), 2.92 (t, *J =* 7.6 Hz, 2H), 2.62 (t, *J =* 7.2 Hz, 2H), 1.98 - 1.90 (m, 2H), 1.73 - 1.59 (m, 6H), 1.09 (t, *J =* 7.2 Hz, 3H).

### 1.8 Synthesis of Compound Target 8 (abbreviated as T8)

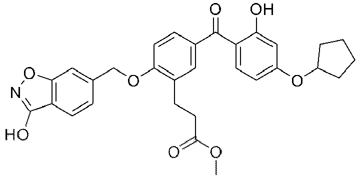

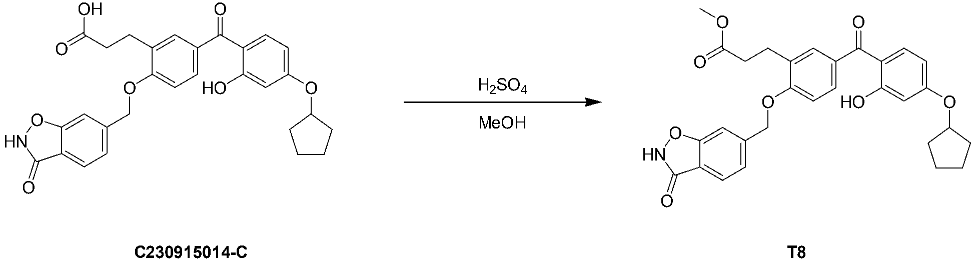

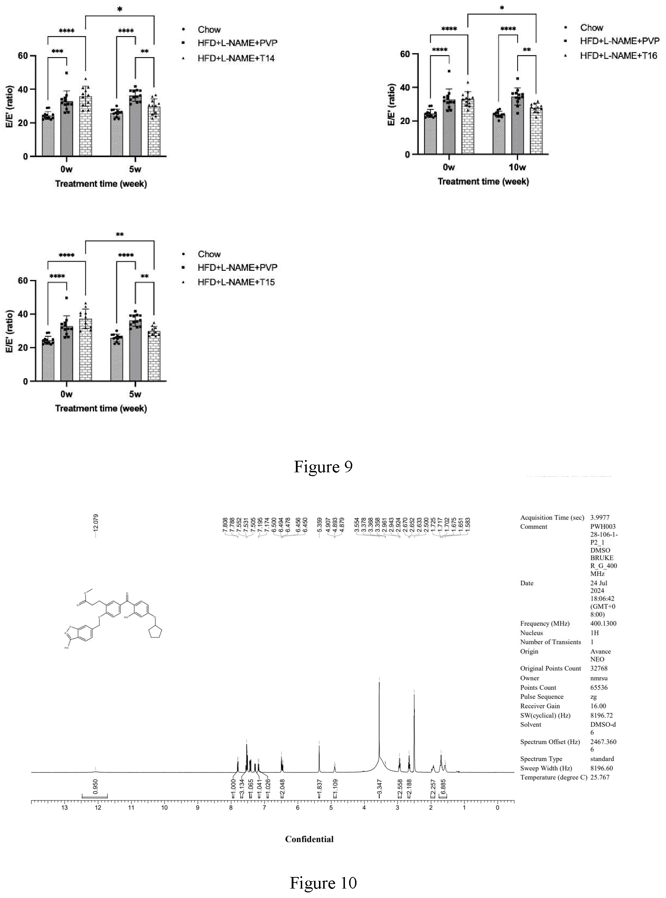

Compound **C230915014-C** (1 g) was added to anhydrous methanol (5 ml), and 10 µl of concentrated sulfuric acid was added dropwise. The reaction was carried out at 60 °C for 3 hours. TLC (Plate1: PE:EA=1:1, 254 nm) showed a small amount of starting material remaining. Saturated aqueous sodium bicarbonate solution was added to the system to adjust the pH to 8 to 9. The mixture was extracted with EA (20 ml * 3) three times, washed with 20 ml of saturated brine, and the organic phase was collected and concentrated to obtain 0.35 g of **T8.** The NMR spectrum of the product is shown in Figure 10.

### 1.9 Synthesis of Compound Target 9 (abbreviated as T9)

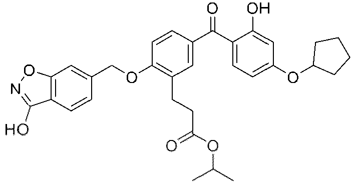

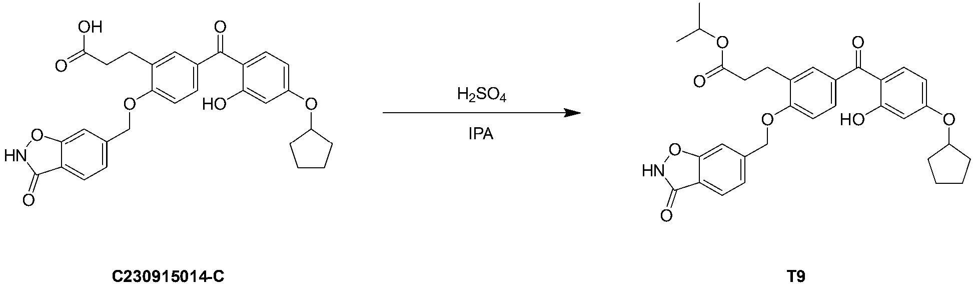

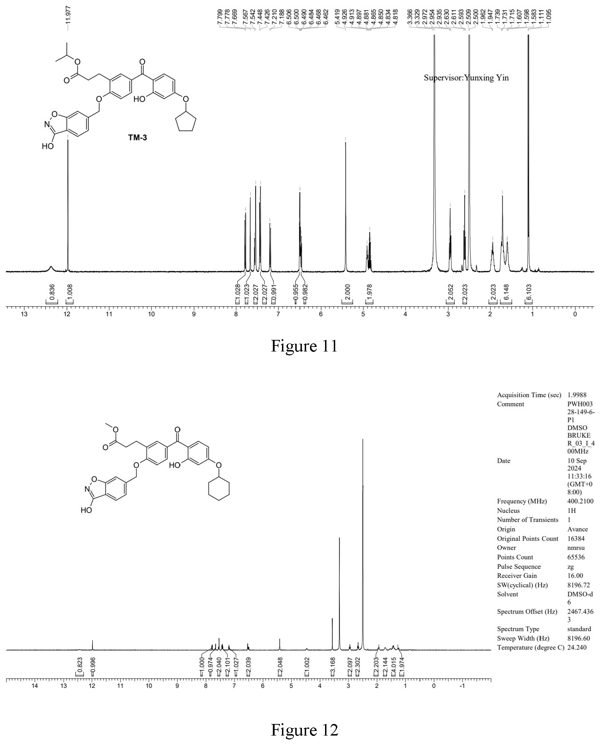

Compound **C230915014-C** (0.7 g) was added to isopropanol (30 ml), and 0.29 ml (4 *eq*) of concentrated sulfuric acid was added dropwise. The reaction was carried out at 85 °C for 2 hours. TLC (Plate1: PE:EA=1:1, 254 nm) showed the formation of the target product. The mixture was concentrated, extracted three times with 40 ml of EA and 30 ml of water, washed with 20 ml of saturated brine, and the organic phase was adsorbed onto silica gel. The product was isolated by column chromatography (PE:EA = 50:1 to 1:1). The target fraction was concentrated, and the organic phase was collected and concentrated to obtain 0.52 g of **T9.** The NMR spectrum of the product is shown in Figure 11.

### 1.10 Synthesis of Compound Target 10 (abbreviated as T10)

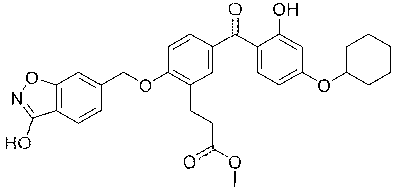

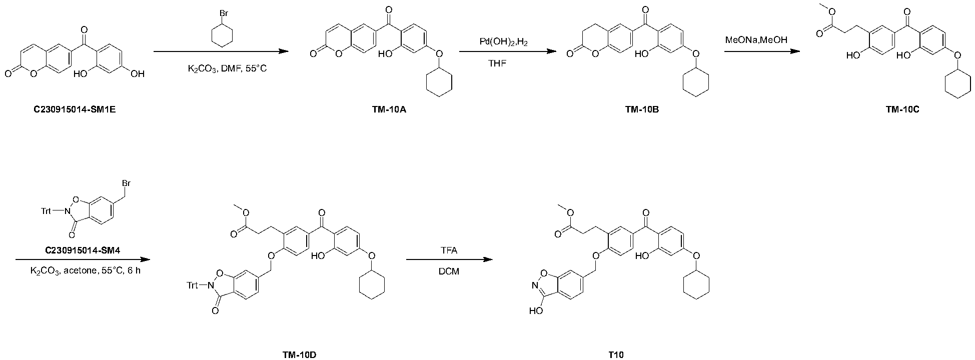

Compound **C230915014-SM1E** (8 g) was added to DMF (80 ml), followed by the addition of potassium carbonate (15.6 g, 4 *eq*) and bromocyclohexane (36.9 g, 8 *eq*). The reaction was carried out at 70 °C for 12 hours. LCMS showed the formation of the target product. The mixture was extracted with 240 ml of ethyl acetate and 300 ml of H₂O. The organic phase was washed with 200 ml of saturated aqueous sodium chloride solution and concentrated. The concentrate was filtered through a pad of silica gel (300 to 400 mesh) using ethyl acetate/n-heptane (1:4, 500 mL). The filtrate was further diluted with 500 ml of n-heptane and filtered again through a pad of 300 to 400 mesh silica gel to obtain the product fraction **TM-10A** (1.95 g).

Compound **TM-10A** (1.7 g) was added to THF (30 ml), followed by the addition of wet Pd(OH)₂ (0.5 g, 0.75 *eq*). The reaction was carried out under H₂ (15 psi) at 25 °C for 2 hours. HPLC showed the formation of the target product. The reaction mixture was concentrated to obtain **TM-10B** (1.6 g).

Compound **TM-10B** (0.8 g) was added to methanol (10 ml), followed by the addition of sodium methoxide (0.36 g, 3 *eq*). The reaction was carried out at 25 °C for 3 hours. LCMS showed the formation of the target product. The reaction mixture was concentrated to remove MeOH. 40 ml of EA and 40 ml of water were added to the system, and the mixture was extracted and separated. The organic phase was collected. The aqueous phase was extracted with 30 ml of EA and 20 ml of aqueous ammonium chloride solution, and the organic phase was collected. The organic phases were combined, washed with 60 ml of saturated brine, and dried over anhydrous sodium sulfate to obtain **TM-10C** (0.9 g).

Compound **TM-10C** (0.9 g) was added to acetone (20 ml), followed by the addition of potassium carbonate (0.3 g, 1 *eq*) and **C230915014-SM4** (1.06 g, 1 *eq*). The reaction was carried out at 55 °C for 16 hours. LCMS showed the formation of the target product. The reaction mixture was filtered and concentrated. 40 ml of EA and 30 ml of water were added to the reaction mixture, and the reaction mixture was extracted and separated. The organic phase was collected, washed with 30 ml of saturated brine, and dried over anhydrous sodium sulfate. The organic phase was purified by column chromatography (TLC: Plate 1, PE:EA=3:1, 254 nm; eluent: PE:EA=50:1 to 5:1). The fraction was concentrated to obtain 1.0 g of a crude product, which was lyophilized to obtain **TM-10D** (1 g).

Compound **TM-10D** (1 g) was added to DCM (20 ml), followed by the addition of TFA (10 ml). The reaction was carried out at 25 °C for 16 hours. LCMS showed the formation of the target product. 40 ml of water and 40 ml of DCM were added to the reaction mixture for extraction. The organic phase was collected, washed with 40 ml of saturated brine, and separated. The organic phase was collected and purified by column chromatography (TLC: Plate 1, PE:EA=1:1, 254 nm; eluent: PE:EA=25:1 to 2:1). The fraction was concentrated to obtain 0.1 g of **T10**. The NMR spectrum of the product is shown in Figure 12.

### 1.11 Synthesis of Compound Target 11 (abbreviated as T11)

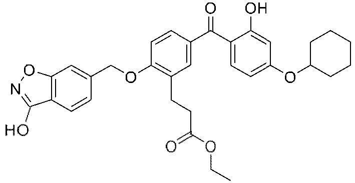

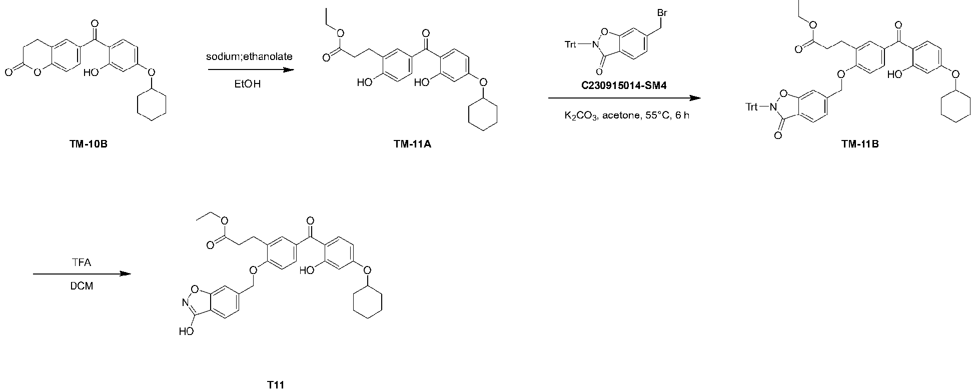

Compound **TM-10B** (1.6 g) was added to ethanol (20 ml), followed by the addition of sodium ethoxide (0.6 g, 2 *eq*). The reaction was carried out under H₂ (15 psi) at 25 °C for 12 hours. LCMS showed the formation of the target product. The reaction mixture was concentrated to remove EtOH. 40 ml of EA and 40 ml of water were added to the system, and the mixture was extracted and separated. The organic phase was collected. The aqueous phase was extracted with 30 ml of EA and 20 ml of aqueous ammonium chloride solution, and the organic phase was collected. The organic phases were combined, washed with 60 ml of saturated brine, and dried over anhydrous sodium sulfate to obtain **TM-11A** (1.8 g).

Compound **TM-11A** (1.8 g) was added to acetone (30 ml), followed by the addition of potassium carbonate (0.6 g, 1.1 *eq*) and **C230915014-SM4** (2.15 g, 1.05 *eq*). The reaction was carried out at 55 °C for 2 hours. LCMS showed the formation of the target product. The reaction mixture was filtered and concentrated. 40 ml of EA and 30 ml of water were added, and the mixture was extracted and separated. The organic phase was collected, washed with 30 ml of saturated brine, and dried over anhydrous sodium sulfate. The organic phase was purified by column chromatography (TLC: Plate 1, PE:EA=3:1, 254 nm; eluent: PE:EA=10:1 to 5:1, with 10% DCM). The fraction was concentrated to obtain **TM-11B** (2 g).

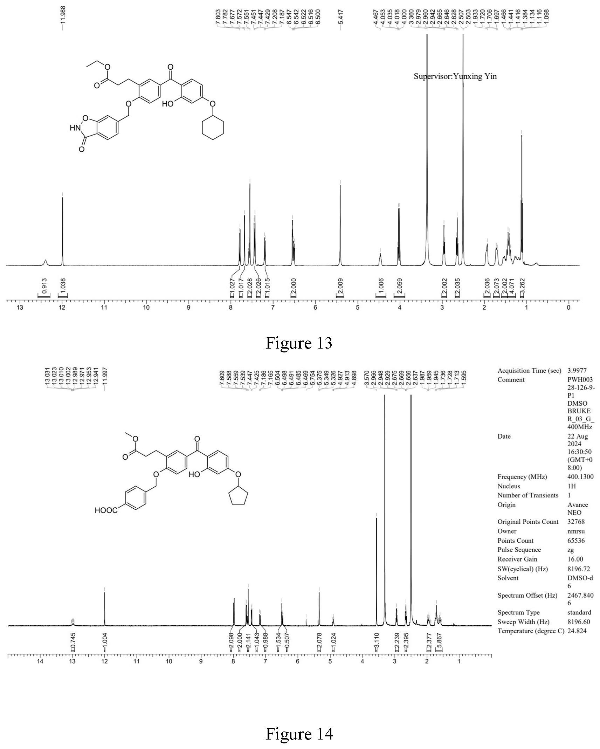

Compound **TM-11B** (0.6 g) was added to **DCM** (10 ml), followed by the addition of HCl/Dioxane (0.37 ml, 2M). The reaction was carried out at 25 °C for 12 hours. LCMS showed the formation of the target product. The reaction mixture was concentrated, extracted with 40 ml of ethyl acetate and 30 ml of H₂O, and the organic phase was washed with 20 ml of saturated aqueous sodium chloride solution. The organic phase was concentrated and triturated with 10 mL of ethanol to obtain 330 mg of T11. The NMR spectrum of the product is shown in Figure 13.

### 1.12 Synthesis of Compound Target 12 (abbreviated as T12)

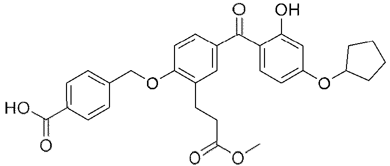

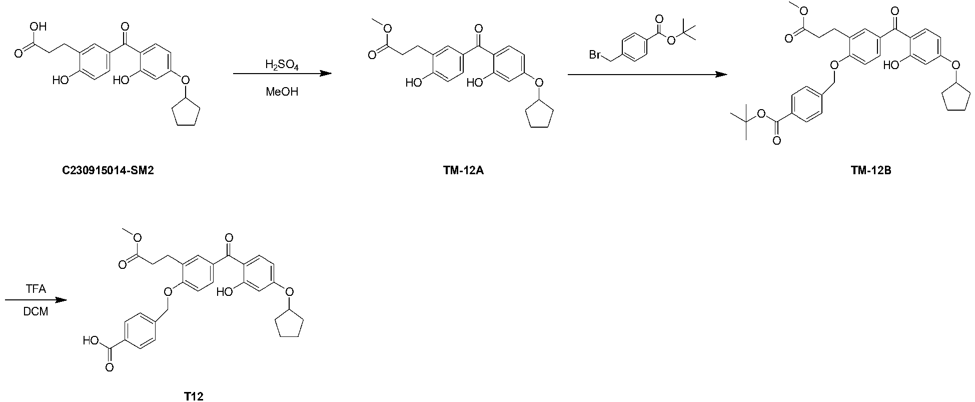

Compound **C230915014-SM2** (1 g) was added to anhydrous methanol (5 ml), and 0.03 ml (0.2 *eq*) of concentrated sulfuric acid was added dropwise. The reaction was carried out at 60 °C for 3 hours. LCMS showed the formation of the target product. Saturated sodium bicarbonate solution was added to the system to adjust the pH to 8 to 9. The mixture was extracted with EA (20 ml * 3). The organic phase was washed with saturated brine and dried over anhydrous sodium sulfate, then concentrated to obtain **TM-12A** (0.75 g).

Compound **TM-12A** (1 g) was added to acetone (20 ml), followed by the addition of potassium carbonate (0.4 g, 1.1 *eq*) and tert-butyl 4-(bromomethyl)benzoate (0.77 g, 1.1 *eq*). The reaction was carried out at 55 °C for 10 hours. LCMS showed the formation of the target product. The reaction mixture was filtered and concentrated. 20 mL of water and 20 mL of EA were added for extraction. The organic phase was concentrated and adsorbed onto silica gel, then purified by column chromatography (TLC: Plate 1, PE:EA=5:1, 254 nm; eluent: PE:EA=50:1 to 20:1). The fraction was concentrated to obtain **TM-12B** (1.2 g).

Compound **TM-12B** (1.2 g) was added to DCM (20 ml), followed by the addition of TFA (6 ml, 39 *eq*). The reaction was carried out at 25 °C for 3 hours. LCMS showed the formation of the target product. 40 ml of water and 20 ml of DCM were added to the reaction mixture for extraction. The organic phase was collected, washed with 30 ml of water, and separated. The organic phase was collected and concentrated to obtain 0.8 g of **T12.** The NMR spectrum of the product is shown in Figure 14.

### 1.13 Synthesis of Compound Target 13 (abbreviated as T13)

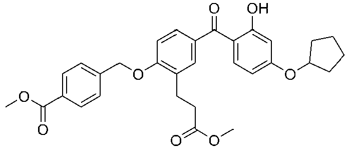

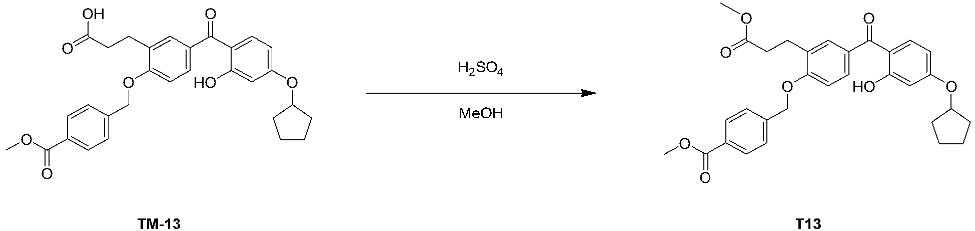

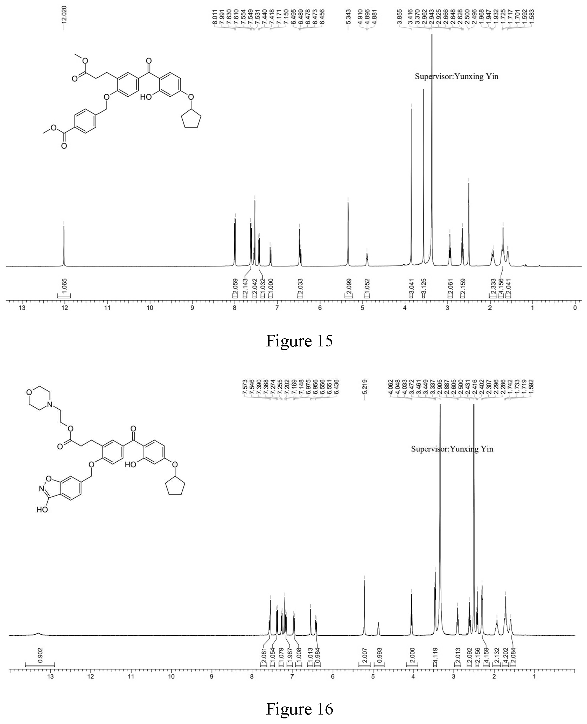

Compound **TM-13** (1.3 g) was added to methanol (20 ml), followed by the addition of sulfuric acid (0.37 g, 1.5 *eq*). The reaction was carried out at 80 °C for 0.5 hours. TLC (Plate 1: PE:EA=1:1, 254 nm) showed the formation of a new spot. The reaction mixture was concentrated, extracted with 40 ml of ethyl acetate and 30 ml of H₂O, and the organic phase was washed with 20 ml of saturated aqueous sodium chloride solution. The organic phase was concentrated and triturated with ethyl acetate/n-heptane (1:5) to obtain 1.0 g of **T13.** The NMR spectrum of the product is shown in Figure 15.

### 1.14 Synthesis of Compound Target 14 (abbreviated as T14)

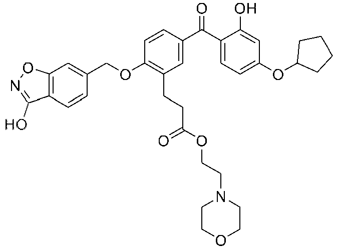

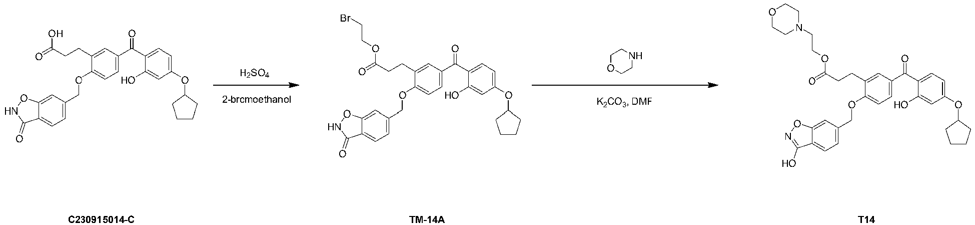

Compound **C230915014-C** (2 g) was added to 2-bromoethanol (30 ml), and 0.5 ml (2.5 *eq*) of concentrated sulfuric acid was added dropwise. The reaction was carried out at 85 °C for 2 hours. LCMS showed the formation of the target product. The mixture was concentrated, extracted three times with 40 ml of EA and 30 ml of water, washed with 20 ml of saturated brine, and the organic phase was concentrated and triturated with 50 ml of ACN to obtain 1.6 g of **TM-14A.**

Compound **TM-14A** (1.4 g) was added to DMF (14 ml), followed by the addition of potassium carbonate (0.25 g, 0.8 *eq*) and morpholine (0.2 ml, 1 *eq*). The reaction was carried out at 5 °C for 0.5 hours. LCMS showed the formation of the target product. 15 ml of H₂O was added to the reaction mixture, followed by filtration. The filtrate was subjected to preparative separation, and the fraction was lyophilized to obtain 0.28 g of **T14.** The NMR spectrum of the product is shown in Figure 16.

### 1.15 Synthesis of Compound Target 15 (abbreviated as T15)

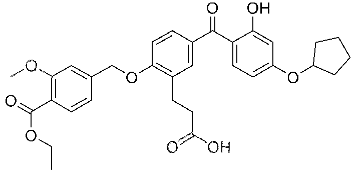

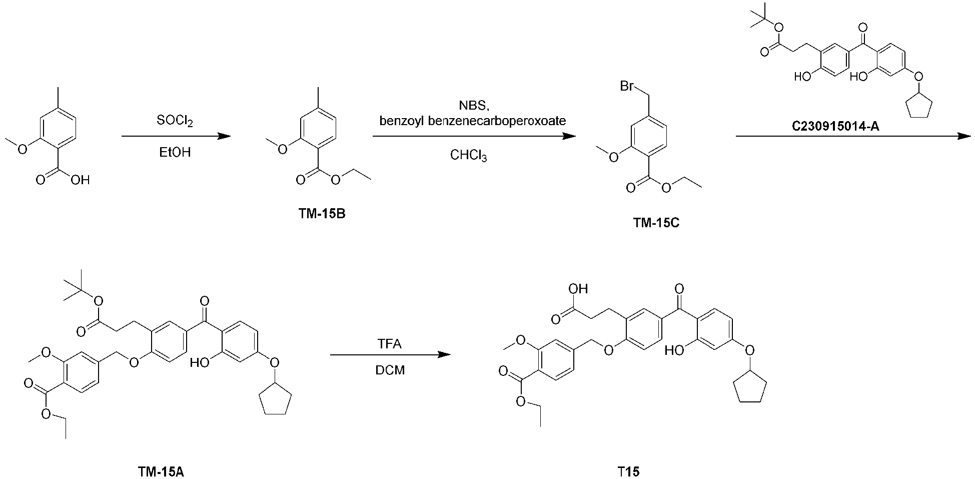

2-Methoxy-4-methylbenzoic acid (4 g) was added to ethanol (20 ml), and SOCl₂ (3.5 ml, 2 *eq*) was added dropwise. The reaction was carried out at 25 °C for 14 hours. TLC (Plate 1: PE:EA=3:1, 254 nm) showed the formation of the target product. 12 mL of water was slowly added dropwise to the reaction mixture, and the pH of the reaction solution was adjusted to 7 to 8 with saturated aqueous NaHCO₃ solution. The mixture was extracted three times with EA (40 mL * 3). The organic phases were combined. The organic phase was washed twice with saturated aqueous NaCl solution (20 mL * 2), dried over anhydrous Na₂SO₄, filtered, and the filtrate was concentrated under reduced pressure at 45 °C to obtain **TM-15B** (4 g).

Compound **TM-15B** (0.6 g) was added to chloroform (9 ml), followed by the addition of NBS (0.55 g, 1 *eq*) and benzoyl peroxide (0.04 g, 0.05 *eq*). The reaction was carried out at 35 °C for 1 hour. HPLC showed the formation of the target product. 20 mL of saturated aqueous NaHCO₃ solution was added to the reaction mixture, and the organic phase was collected. The aqueous phase was extracted twice with DCM (15 mL * 2), and the organic phases were combined. The organic phase was washed three times with water (20 mL * 3), dried over 10 g of anhydrous Na₂SO₄, filtered, and the filtrate was concentrated to obtain **TM-15C** (0.7 g).

Compound **TM-15C** (0.44 g) was added to acetone (7 ml), followed by the addition of potassium carbonate (0.22 g, 1 *eq*) and **C230915014-A** (0.7 g, 1 *eq*). The reaction was carried out at 65 °C for 14 hours. LCMS showed the formation of the target product. 21 mL of water was added to the reaction mixture, and the aqueous phase was extracted three times with EA (10 mL * 3). The organic phases were combined. The organic phase was washed twice with water (20 mL * 2), dried over 10 g of anhydrous Na₂SO₄, filtered, and the filtrate was concentrated to obtain **TM-15A** (1 g).

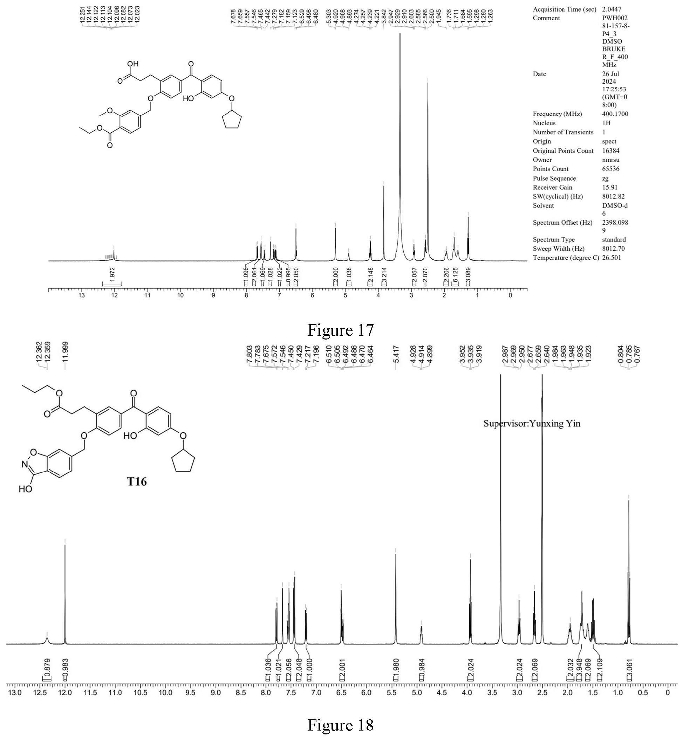

Compound **TM-15A** (1 g) was added to DCM (10 ml), followed by the addition of TFA (2.4 ml, 20 *eq*). The reaction was carried out at 25 °C for 2 hours. TLC (Plate 1: PE:EA=3:1, 254 nm) showed the reaction was complete. 30 ml of water and 20 ml of DCM were added to the reaction mixture for extraction. The organic phase was collected, washed with 30 ml of water, and separated. The organic phase was collected, concentrated, and then triturated with 6 mL of n-heptane/ethyl acetate at 15 °C for 20 minutes. After filtration, 0.53 g of **T15** was obtained. The NMR spectrum of the product is shown in Figure 17.

### 1.16 Synthesis of Compound Target 16 (abbreviated as T16)

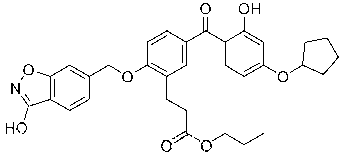

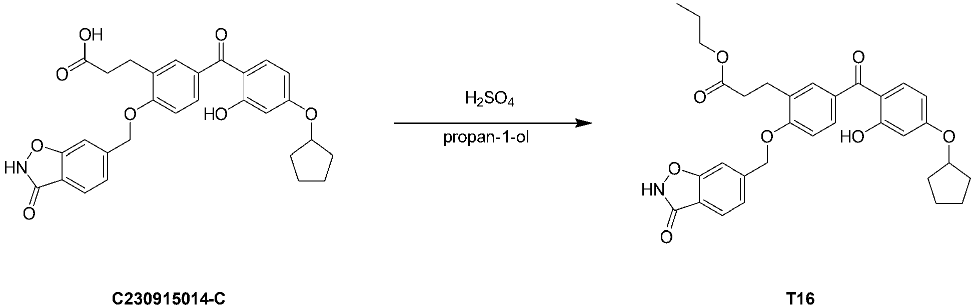

Compound **C230915014-C (0.7 g)** was added to n-propanol (30 ml), and 0.29 ml (4 *eq*) of concentrated sulfuric acid was added dropwise. The reaction was carried out at 85 °C for 2 hours. TLC (Plate 1: PE:EA=1:1, 254 nm) showed the formation of the target product. The mixture was concentrated, extracted three times with 40 ml of EA and 30 ml of water, washed with 20 ml of saturated brine, and the organic phase was adsorbed onto silica gel. The product was isolated by column chromatography (PE:EA = 50:1 to 1:1). The target fraction was concentrated, and the organic phase was collected and concentrated to obtain 0.6 g of **T16.** The NMR spectrum of the product is shown in Figure 18.

### 2. Test for Inhibition of DNA Binding Activity

### 2.1. Test Method

### Testing of c-Jun Transcription Factor Binding Activity to DNA

The binding ability of DNA to transcription factor was tested using TransAM kits (Active Motif 46096). The transcription factor was c-Jun/AP-1. Drugs at final concentrations of 500 µM, 250 µM, 125 µM, 62.5 µM, and 31.25 µM, along with 1 µL of cell extract containing the transcription factor, were sequentially added to a 96-well plate pre-coated with double-stranded DNA oligonucleotides. After incubation for 1 hour, the plate was incubated with an antibody against the c-JUN transcription factor. The absorbance at 450 nm was measured, with absorbance at 655 nm used as background. The binding activity of the dsDNA sequence to the transcription factor was measured. Lower binding activity indicates stronger inhibitory activity of compound.

### 2.2 Test Results

The results are shown in the table below and Figure 4.

### DNA & JUN Binding Activity

| Compound ID | DNA Binding Activity (%) |
|---|---|
| **T1** | 49.8 |
| **T2** | 42.9 |
| **T3** | 14.8 |
| **T4** | 20.8 |
| **T5** | 4.5 |
| **T6** | 81.0 |
| **T7** | 103.4 |
| **T-5224** | 10.0 |

From the above results, it can be seen that, except for **T7,** the other six compounds all showed activity in inhibiting JUN binding to DNA. Among them, **T3, T4,** and **T5** were superior or comparable to the positive control T-5224. Thus, the compounds of the present invention (especially compounds of Formulas I-1, I-1', and I-2 series) possess activity in inhibiting JUN binding to DNA.

### 3. Test for Inhibition of Transcriptional Activity

### 3.1 Test Method

### Dual-Luciferase Reporter System Assay for Inhibition of DNA Binding Activity

293T cells were passaged at a ratio of 1:3, plated, and incubated for 24 hours. The medium was replaced with antibiotic-free medium Opti-MEM (Gibco, 11058021). The 293T cells were transiently transfected with the luciferase reporter plasmid pGL4.44[luc2P/AP1 RE/Hygro] (Promega E411) and cultured for 24 hours. The cells were incubated for 1 hour in 1% PS/10% FBS/DMEM (Gibco, 11965092) containing the drugs at final concentrations of 50 µM, 25 µM, 12.5 µM, and 6.25 µM. Subsequently, the cells were stimulated with PMA (final concentration 10 ng/ml) (Sigma, 79346-5MG) and cultured for 34 hours. Lysates were measured using the Dual-Luciferase Reporter Assay System (Promega). Both the drugs and PMA were prepared as stock solutions in DMSO, diluted with culture medium, and added to the culture.

### 3.2 Test Results

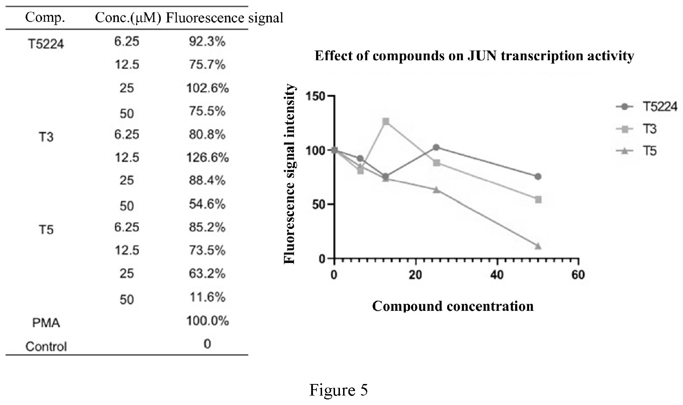

The results are shown in the table below and Figure 5.

| Com. | Conc.(µM) | Luc |
|---|---|---|
| **T-5224** | 6.25 | 92.3% |
| | 12.5 | 75.7% |
| | 25 | 102.6% |
| | 50 | 75.5% |
| **T3** | 6.25 | 80.8% |
| | 12.5 | 126.6% |
| | 25 | 88.4% |
| | 50 | 54.6% |
| **T5** | 6.25 | 85.2% |
| | 12.5 | 73.5% |
| | 25 | 63.2% |
| | 50 | 11.6% |
| PMA | | 100.0% |
| Control | | 0 |

From the above results, **T3** and **T5** further showed activity in inhibiting JUN transcription. It can be seen that the compounds of the present invention (especially compounds of Formulas I-1, I-1', and I-2 series) possess activity in inhibiting JUN transcription.

### 4. Test of Therapeutic Efficacy in Animal Model

The materials and reagents, animal experiment guidelines, and induction of heart failure with preserved ejection fraction model were the same as in Example 1.

The administration method was as follows:
After successful induction of the animal model, the model treatment group was treated with the compounds of the present invention (e.g., compounds **T3, T5, T8** to **T16**) or **T-5224.** The model control group was treated with the drug-free vehicle as a control. Mice fed normally with food and water throughout the induction period served as the negative control. After the mice were raised to 5 to 8 weeks of age, drug treatment was initiated following echocardiography and Doppler imaging confirmation of successful model establishment. For the treatment groups, **T3, T5, T8** to **T16,** and **T-5224** were administered at a dose of 12 mg/kg per administration based on mouse body weight. Specifically, the compounds were administered every other day. Each time, 0.54 mg of the compound was dissolved in 100 µL of 0.5% PVP solution (in practice, the drug solution was prepared based on the number of mice. For example, for 14 mice, 8 mg of the compound was dissolved in 1.5 mL of solution, and each mouse received 100 µL of the solution). Drug treatment was initiated after successful model induction. The control group received an equal volume of 0.5% PVP solution, with all other treatments being the same.

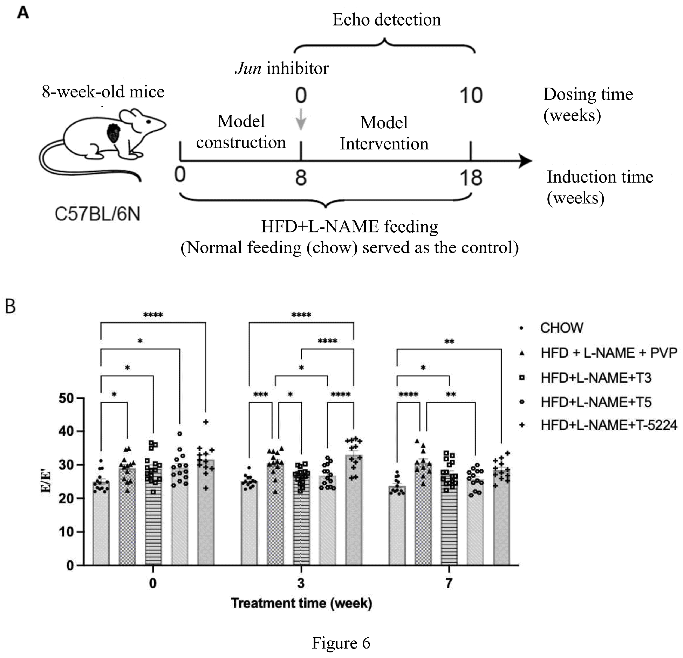

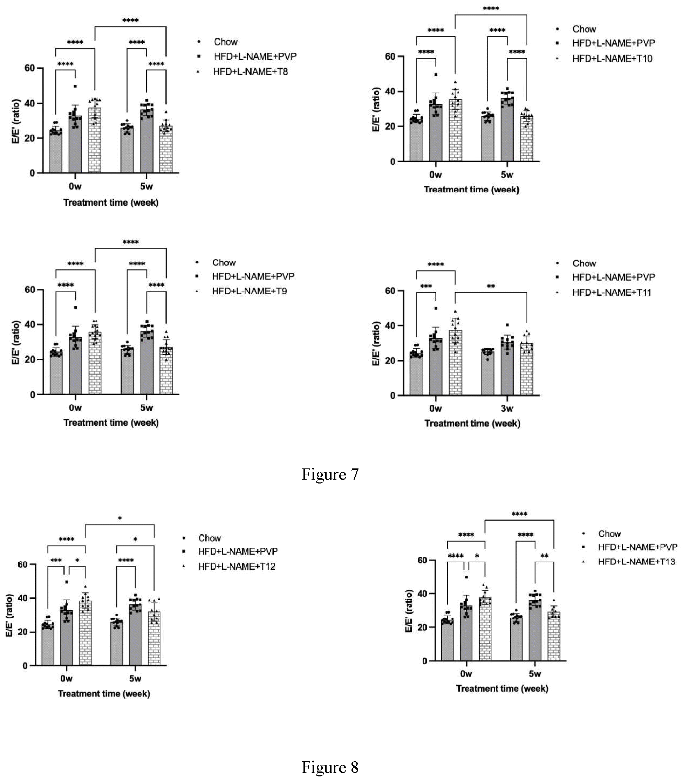

The experimental results are shown in Figures 6 to 9 (wherein, week 0 on the horizontal axis represents the day when administration started after successful model construction, i.e., the starting point of treatment). It is evident from Figure 6 that, at the 3-week point after the administration, the positive control **T-5224** had not yet shown any inhibitory effect on disease progression. In contrast, the compounds **T3** and **T5** of the present invention had already significantly inhibited disease progression at the same time point, demonstrating a clear advantage. Thus, compared to the positive control **T-5224,** the compounds **T3** and **T5** of the present invention could inhibit disease progression earlier, showing a significant advantage. Similarly, it can be observed that compounds **T8** to **T16** of the present invention also exhibited a significant therapeutic effect on HFpEF, with a rapid onset of action. Therefore, the compounds of the present invention (particularly those of Formulas I-1, I-1', and I-2 series) demonstrated a significant therapeutic effect on HFpEF, with a rapid onset of action.

In conclusion, the inventors of the present application have found that the compounds of the present invention possess activity in inhibiting JUN binding to DNA, exhibit activity in inhibiting JUN transcription, can produce a therapeutic effect on HFpEF, have significant advantages in the treatment of HFpEF, and hold broad application prospects.

## Claims

1. Use of a compound represented by Formula I, or a stereoisomer, prodrug, crystal form, pharmaceutically acceptable salt, pharmaceutically acceptable ester, or pharmaceutically acceptable solvate thereof in the manufacture of a medicament for treating and/or preventing heart failure with preserved ejection fraction, 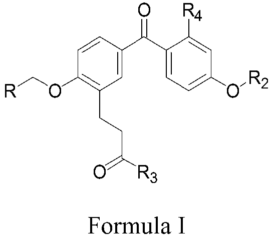 wherein:
R is selected from the group consisting of C6-C10 aryl, and 6- to 10-membered heteroaryl, wherein the C6-C10 aryl, and 6- to 10-membered heteroaryl are each independently optionally substituted with R¹ and/or R⁵;
preferably, R is selected from the group consisting of C6-C10 aryl, and 6- to 10-membered heteroaryl, wherein the C6-C10 aryl, and 6- to 10-membered heteroaryl are each independently optionally substituted with R¹ or R¹ and R⁵;
more preferably, R is selected from the group consisting of C6-C10 aryl, and 6- to 10-membered heteroaryl, wherein the C6-C10 aryl, and 6- to 10-membered heteroaryl are each independently optionally substituted with R¹;
R¹ is selected from the group consisting of hydrogen, C1-C6 alkyl, -COOH, -C(=O)O-C1-C6 alkyl, -(CH₂)ₘ-O-C1-C6 alkyl, and -NR^{c}R^{d};
R^{c}, and R^{d} are each independently selected from the group consisting of hydrogen, C1-C6 alkyl, and -(CH₂)ₘ-O-C1-C6 alkyl;
m is selected from the group consisting of 0, 1, 2, 3, 4, 5, and 6;
preferably, m is selected from the group consisting of 0, and 1;
more preferably, m is 1;
R² is selected from the group consisting of -O-(CH₂)ₙ-R^{2'};
n is selected from the group consisting of 0, 1, 2, 3, 4, 5, and 6;
preferably, n is selected from the group consisting of 0, and 1;
R^{2'} is selected from the group consisting of C1-C6 alkyl, C3-C8 cycloalkyl, C6-C10 aryl, and 5- to 6-membered heteroaryl;
R³ is selected from the group consisting of hydroxy, and -O-C1-C6 alkyl, wherein the C1-C6 alkyl is optionally substituted with 5- to 6-membered heterocyclyl;
preferably, R³ is selected from the group consisting of hydroxy, and -O-C1-C6 alkyl, wherein the C1-C6 alkyl is optionally substituted with 6-membered heterocyclyl;
more preferably, R³ is selected from the group consisting of hydroxy, and -O-C1-C6 alkyl, wherein the C1-C6 alkyl is optionally substituted with morpholinyl;
most preferably, R³ is selected from the group consisting of hydroxy, and -O-C1-C6 alkyl;
R⁴ is selected from the group consisting of hydroxy, cyano, nitro, carboxyl, mercapto, halogen, C1-C6 alkyl, -NR^{a}R^{b}, -CONR^{a}R^{b}, C6-C10 aryl, -O-C6-C10 aryl, -C(=O)-C1-C6 alkyl, -CH₂-C6-C10 aryl, C1-C6 alkoxy, C2-C6 alkenyl, C3-C8 cycloalkyl, -C(=O)O-C1-C6 alkyl, -C(=O)O-C6-C10 aryl, C1-C6 alkylthio, -S(=O)-C1-C6 alkyl, -S(=O)₂-C1-C6 alkyl, and 5- to 6-membered heteroaryl;
R^{a}, and R^{b} are each independently selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
R⁵ is selected from the group consisting of hydrogen, C1-C6 alkyl, C1-C6 alkoxy, and C1-C6 alkylthio;
and, the compound represented by Formula I does not include

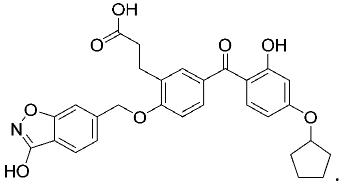

2. The use according to claim 1, wherein R is selected from the group consisting of phenyl, benzimidazolyl, and 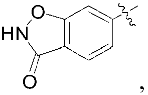 wherein the phenyl, benzimidazolyl, and 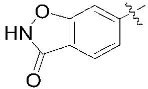 are each independently optionally substituted with R¹ and/or R⁵;
preferably, R is selected from the group consisting of phenyl, benzimidazolyl, and 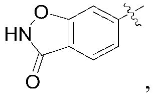 wherein the phenyl, benzimidazolyl, and 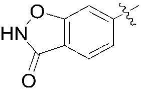 are each independently optionally substituted with R¹ or R¹ and R⁵;
more preferably, R is selected from the group consisting of phenyl, benzimidazolyl, and 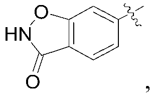 wherein the phenyl, benzimidazolyl, and 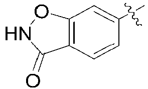 are each independently optionally substituted with R¹;
or preferably, R is selected from the group consisting of phenyl, 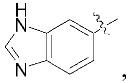 and 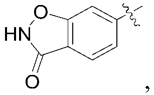 wherein the phenyl, 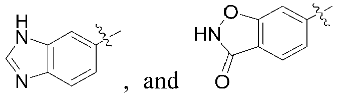 are each independently optionally substituted with R¹ and/or R⁵;
or more preferably, R is selected from the group consisting of phenyl, 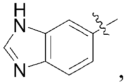 and 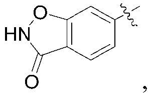 wherein the phenyl, 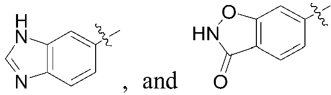 are each independently optionally substituted with R¹ or R¹ and R⁵;
or further preferably, R is selected from the group consisting of phenyl, 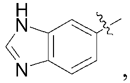 and 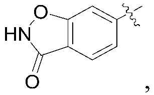 wherein the phenyl, 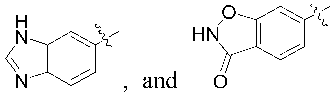 are each independently optionally substituted with R¹;
or preferably, R is selected from the group consisting of 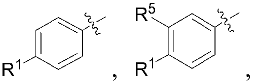 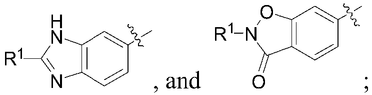
or more preferably, R is selected from the group consisting of 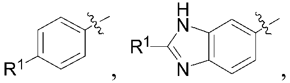 and 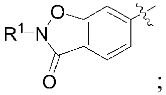
preferably, R¹ is selected from the group consisting of hydrogen, -COOH, -C(=O)O-C1-C6 alkyl, -(CH₂)ₘ-O-C1-C6 alkyl, and -NR^{c}R^{d};
more preferably, R¹ is selected from the group consisting of hydrogen, -COOH, -C(=O)O-C1-C6 alkyl, and -(CH₂)ₘ-O-C1-C6 alkyl;
preferably, R^{c}, and R^{d} are each independently selected from the group consisting of hydrogen, and -(CH₂)ₘ-O-C1-C6 alkyl;
more preferably, one of R^{c} and R^{d} is hydrogen, and the other is selected from the group consisting of -(CH₂)ₘ-O-C1-C6 alkyl;
further preferably, R¹ is selected from the group consisting of hydrogen, -COOH, -COOCH₃, -COOCH₂CH₃, -COO(CH₂)₂CH₃, -COO(CH₂)₃CH₃, -COO(CH₂)₄CH₃, -COO(CH₂)₅CH₃, and 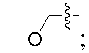
still further preferably, R¹ is selected from the group consisting of hydrogen, -COOH, -COOCH₃, -COOCH₂CH₃, and 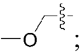
most preferably, R¹ is selected from the group consisting of hydrogen, -COOH, -COOCH₂CH₃, and 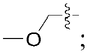
preferably, R⁵ is selected from the group consisting of hydrogen, C1-C6 alkyl, and C1-C6 alkoxy;
more preferably, R⁵ is selected from the group consisting of C1-C6 alkoxy;
most preferably, R⁵ is methoxy.

3. The use according to any one of claims 1 to 2, wherein R^{2'} is selected from the group consisting of C1-C6 alkyl, C3-C8 cycloalkyl, phenyl, pyrazinyl (e.g., 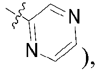 and furanyl (e.g., 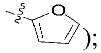
preferably, R^{2'} is selected from the group consisting of cyclopropyl, cyclobutyl, cyclopentyl, cyclohexyl, cycloheptyl, 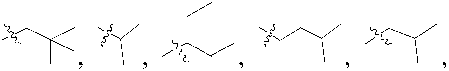 phenyl, pyrazinyl (e.g., 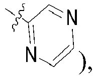
and furanyl (e.g., 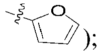
or, R^{2'} is selected from the group consisting of C1-C6 alkyl, C3-C8 cycloalkyl, and C6-C10 aryl;
or preferably, R^{2'} is selected from the group consisting of C1-C6 alkyl, C3-C8 cycloalkyl, and phenyl;
or more preferably, R^{2'} is selected from the group consisting of cyclopentyl, cyclohexyl, 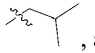 and phenyl;
or more preferably, R^{2'} is selected from the group consisting of cyclopentyl, 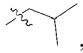 and phenyl;
preferably, R² is selected from the group consisting of -O-C1-C6 alkyl, -O-C3-C8 cycloalkyl, -O-CH₂-C6-C10 aryl, and -O-CH₂-5- to 6-membered heteroaryl;
more preferably, R² is selected from the group consisting of -O-C1-C6 alkyl, -O-C3-C8 cycloalkyl, -O-CH₂-phenyl, and -O-CH₂-5- to 6-membered heteroaryl, wherein the 5- to 6-membered heteroaryl is selected from the group consisting of pyrazinyl (e.g., 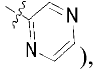 and furanyl (e.g., 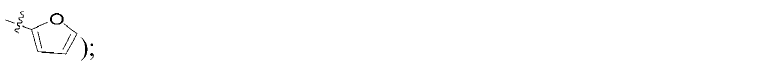
most preferably, R² is selected from the group consisting of 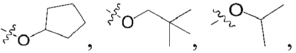 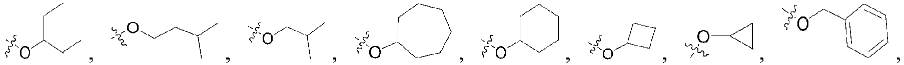 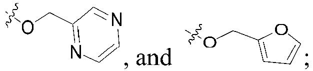
or preferably, R² is selected from the group consisting of -O-C1-C6 alkyl, -O-C3-C8 cycloalkyl, and -O-CH₂-C6-C10 aryl;
or more preferably, R² is selected from the group consisting of -O-C1-C6 alkyl, -O-C3-C8 cycloalkyl, and -O-CH₂-phenyl;
or further preferably, R² is selected from the group consisting of 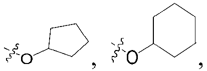 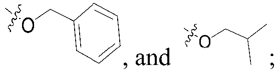
or most preferably, R² is selected from the group consisting of 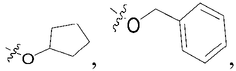
and

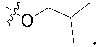

4. The use according to any one of claims 1 to 3, wherein R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, -O(CH₂)₅CH₃, and 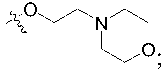
preferably, R³ is selected from the group consisting of hydroxy, -OCH₃, -OCH₂CH₃, -OCH(CH₃)CH₃, -O(CH₂)₂CH₃, and 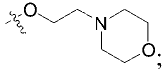
or preferably, R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, and -O(CH₂)₅CH₃;
or more preferably, R³ is selected from the group consisting of hydroxy, -OCH₃, -OCH₂CH₃, -O(CH₂)₂CH₃, -O(CH₂)₃CH₃, -O(CH₂)₄CH₃, and -O(CH₂)₅CH₃;
or further preferably, R³ is selected from the group consisting of hydroxy, -OCH₃, -OCH₂CH₃, -OCH(CH₃)CH₃, and -O(CH₂)₂CH₃;
or most preferably, R³ is selected from the group consisting of hydroxy, -OCH₃, and -OCH₂CH₃.

5. The use according to any one of claims 1 to 4, wherein R⁴ is selected from the group consisting of hydroxy, cyano, nitro, carboxyl, mercapto, halogen, C1-C6 alkyl, -NR^{a}R^{b}, -CONR^{a}R^{b}, phenyl, phenoxy, -C(=O)-C1-C6 alkyl, benzyl, C1-C6 alkoxy, C2-C6 alkenyl, C3-C8 cycloalkyl, -C(=O)O-C1-C6 alkyl, -C(=O)O-phenyl, C1-C6 alkylthio, -S(=O)-C1-C6 alkyl, -S(=O)₂-C1-C6 alkyl, and thienyl (e.g., 2-thienyl);
preferably, R^{a}, and R^{b} are each independently selected from the group consisting of 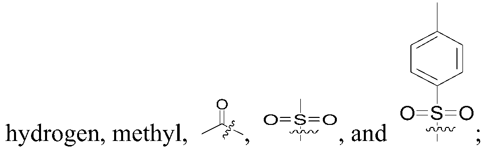
or preferably, one of R^{a} and R^{b} is selected from the group consisting of hydrogen, and C1-C6 alkyl, and the other is selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
or more preferably, one of R^{a} and R^{b} is selected from the group consisting of hydrogen, and methyl, and the other is selected from the group consisting of hydrogen, methyl, 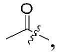 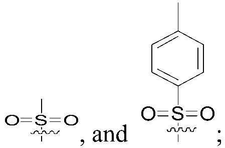
preferably, R⁴ is selected from the group consisting of hydroxy, cyano, nitro, carboxyl, -NH₂, -SH, -CONH₂, methyl, phenyl, benzyl, methoxy, phenoxy, acetyl, -F, -Cl, -Br, -CH=CH₂, cyclopentyl, -COOMe, -COOPh, -SMe, -SOMe, -SO₂Me, -NMe₂, -NHAc, -NHMs, -NHTs, and 2-thienyl;
more preferably, R⁴ is hydroxy.

6. The use according to any one of claims 1 to 5, wherein the structural formula of the compound is as shown in Formula I-1 or Formula I-1', preferably, the structural formula of the compound is as shown in Formula I-1, 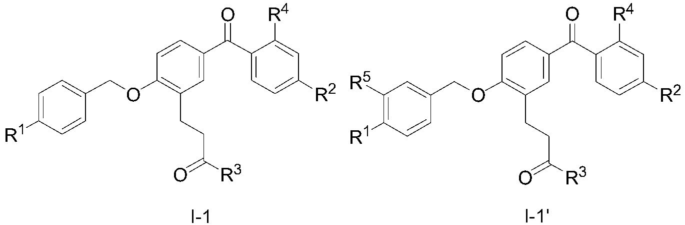 wherein:
R¹ is selected from the group consisting of -COOH, and -C(=O)O-C1-C6 alkyl;
preferably, R¹ is selected from the group consisting of -COOH, -COOCH₃, -COOCH₂CH₃, -COO(CH₂)₂CH₃, -COO(CH₂)₃CH₃, -COO(CH₂)₄CH₃, and -COO(CH₂)₅CH₃;
more preferably, R¹ is selected from the group consisting of -COOH, -COOCH₃, and -COOCH₂CH₃;
most preferably, R¹ is selected from the group consisting of -COOH, and -COOCH₂CH₃;
R² is selected from the group consisting of -O-(CH₂)ₙ-R^{2'};
n is selected from the group consisting of 0, 1, 2, 3, 4, 5, and 6;
preferably, n is selected from the group consisting of 0, and 1;
R^{2'} is selected from the group consisting of C1-C6 alkyl, C3-C8 cycloalkyl, C6-C10 aryl, and 5- to 6-membered heteroaryl;
preferably, R^{2'} is selected from the group consisting of C1-C6 alkyl, C3-C8 cycloalkyl, phenyl, pyrazinyl (e.g., 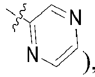 and furanyl (e.g., 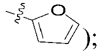
more preferably, R^{2'} is selected from the group consisting of cyclopropyl, cyclobutyl, cyclopentyl, cyclohexyl, cycloheptyl, 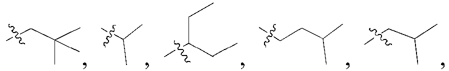 phenyl, pyrazinyl (e.g., 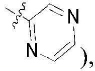 and furanyl (e.g., 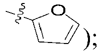
or preferably, R^{2'} is selected from the group consisting of C1-C6 alkyl, C3-C8 cycloalkyl, and C6-C10 aryl;
or more preferably, R^{2'} is selected from the group consisting of C1-C6 alkyl, C3-C8 cycloalkyl, and phenyl;
or most preferably, R^{2'} is selected from the group consisting of cyclopentyl, 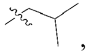 and phenyl;
preferably, R² is selected from the group consisting of -O-C1-C6 alkyl, -O-C3-C8 cycloalkyl, -O-CH₂-C6-C10 aryl, and -O-CH₂-5- to 6-membered heteroaryl;
more preferably, R² is selected from the group consisting of -O-C1-C6 alkyl, -O-C3-C8 cycloalkyl, -O-CH₂-phenyl, and -O-CH₂-5- to 6-membered heteroaryl, wherein the 5- to 6-membered heteroaryl is selected from the group consisting of pyrazinyl (e.g., 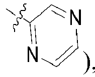 and furanyl (e.g., 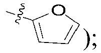
most preferably, R² is selected from the group consisting of 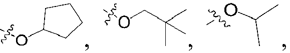 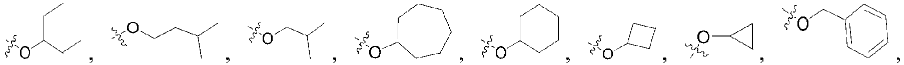 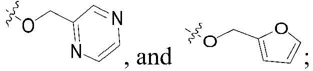
or preferably, R² is selected from the group consisting of -O-C1-C6 alkyl, -O-C3-C8 cycloalkyl, and -O-CH₂-C6-C10 aryl;
or more preferably, R² is selected from the group consisting of -O-C1-C6 alkyl, -O-C3-C8 cycloalkyl, and -O-CH₂-phenyl;
or most preferably, R² is selected from the group consisting of 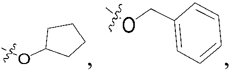 and 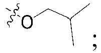
R³ is selected from the group consisting of hydroxy, and -O-C1-C6 alkyl;
preferably, R³ is selected from the group consisting of hydroxy, -OCH₃, -OCH₂CH₃, and -O(CH₂)₂CH₃;
more preferably, R³ is hydroxy;
R⁴ is selected from the group consisting of hydroxy, cyano, nitro, carboxyl, mercapto, halogen, C1-C6 alkyl, -NR^{a}R^{b}, -CONR^{a}R^{b}, C6-C10 aryl, -O-C6-C10 aryl, -C(=O)-C1-C6 alkyl, -CH₂-C6-C10 aryl, C1-C6 alkoxy, C2-C6 alkenyl, C3-C8 cycloalkyl, -C(=O)O-C1-C6 alkyl, -C(=O)O-C6-C10 aryl, C1-C6 alkylthio, -S(=O)-C1-C6 alkyl, -S(=O)₂-C1-C6 alkyl, and 5- to 6-membered heteroaryl;
preferably, R⁴ is selected from the group consisting of hydroxy, cyano, nitro, carboxyl, mercapto, halogen, C1-C6 alkyl, -NR^{a}R^{b}, -CONR^{a}R^{b}, phenyl, phenoxy, -C(=O)-C1-C6 alkyl, benzyl, C1-C6 alkoxy, C2-C6 alkenyl, C3-C8 cycloalkyl, -C(=O)O-C1-C6 alkyl, -C(=O)O-phenyl, C1-C6 alkylthio, -S(=O)-C1-C6 alkyl, -S(=O)₂-C1-C6 alkyl, and thienyl (e.g., 2-thienyl);
R^{a}, and R^{b} are each independently selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
preferably, R^{a}, and R^{b} are each independently selected from the group consisting of hydrogen, methyl, 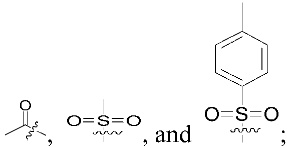
or preferably, one of R^{a} and R^{b} is selected from the group consisting of hydrogen, and C1-C6 alkyl, and the other is selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
or more preferably, one of R^{a} and R^{b} is selected from the group consisting of hydrogen, and methyl, and the other is selected from the group consisting of hydrogen, methyl, 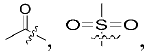 and 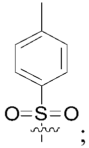
more preferably, R⁴ is selected from the group consisting of hydroxy, cyano, nitro, carboxyl, -NH₂, -SH, -CONH₂, methyl, phenyl, benzyl, methoxy, phenoxy, acetyl, -F, -Cl, -Br, -CH=CH₂, cyclopentyl, -COOMe, -COOPh, -SMe, -SOMe, -SO₂Me, -NMe₂, -NHAc, -NHMs, -NHTs, and 2-thienyl;
most preferably, R⁴ is hydroxy;
R⁵ is selected from the group consisting of hydrogen, C1-C6 alkyl, C1-C6 alkoxy, and C1-C6 alkylthio;
preferably, R⁵ is selected from the group consisting of hydrogen, C1-C6 alkyl, and C1-C6 alkoxy;
more preferably, R⁵ is selected from the group consisting of C1-C6 alkoxy;
most preferably, R⁵ is methoxy.

7. The use according to any one of claims 1 to 5, wherein the structural formula of the compound is as shown in Formula I-2, 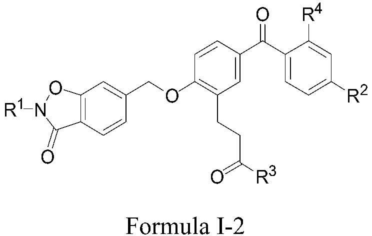 wherein:
R¹ is selected from the group consisting of hydrogen, -(CH₂)ₘ-O-C1-C6 alkyl, and -NR^{c}R^{d};
preferably, R¹ is selected from the group consisting of hydrogen, and -(CH₂)ₘ-O-C1-C6 alkyl;
R^{c}, and R^{d} are each independently selected from the group consisting of hydrogen, C1-C6 alkyl, and -(CH₂)ₘ-O-C1-C6 alkyl;
preferably, R^{c}, and R^{d} are each independently selected from the group consisting of hydrogen, and -(CH₂)ₘ-O-C1-C6 alkyl;
more preferably, one of R^{c} and R^{d} is hydrogen, and the other is selected from the group consisting of -(CH₂)ₘ-O-C1-C6 alkyl;
m is selected from the group consisting of 0, 1, 2, 3, 4, 5, and 6;
preferably, m is selected from the group consisting of 0, and 1;
more preferably, m is 1;
more preferably, R¹ is selected from the group consisting of hydrogen, and 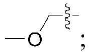
R² is selected from the group consisting of -O-(CH₂)ₙ-R^{2'};
n is selected from the group consisting of 0, 1, 2, 3, 4, 5, and 6;
preferably, n is selected from the group consisting of 0, and 1;
R^{2'} is selected from the group consisting of C1-C6 alkyl, C3-C8 cycloalkyl, C6-C10 aryl, and 5- to 6-membered heteroaryl;
preferably, R^{2'} is selected from the group consisting of C1-C6 alkyl, C3-C8 cycloalkyl, phenyl, pyrazinyl (e.g., 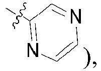 and furanyl (e.g., 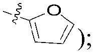
more preferably, R^{2'} is selected from the group consisting of cyclopropyl, cyclobutyl, cyclopentyl, cyclohexyl, cycloheptyl, 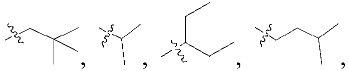 phenyl, pyrazinyl (e.g., 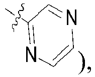 and furanyl (e.g., 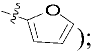
or preferably, R^{2'} is selected from the group consisting of C3-C8 cycloalkyl;
or more preferably, R^{2'} is selected from the group consisting of cyclopentyl, and cyclohexyl;
or most preferably, R^{2'} is cyclopentyl;
preferably, R² is selected from the group consisting of -O-C1-C6 alkyl, -O-C3-C8 cycloalkyl, -O-CH₂-C6-C10 aryl, and -O-CH₂-5- to 6-membered heteroaryl;
more preferably, R² is selected from the group consisting of -O-C1-C6 alkyl, -O-C3-C8 cycloalkyl, -O-CH₂-phenyl, and -O-CH₂-5- to 6-membered heteroaryl, wherein the 5- to 6-membered heteroaryl is selected from the group consisting of pyrazinyl (e.g., 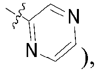 and furanyl (e.g., 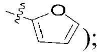
most preferably, R² is selected from the group consisting of 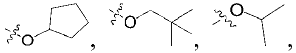 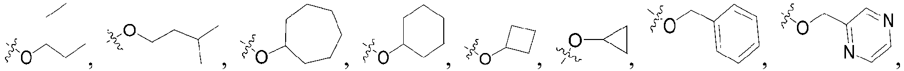 and 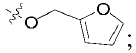
or preferably, R² is selected from the group consisting of -O-C3-C8 cycloalkyl;
or more preferably, R² is selected from the group consisting of 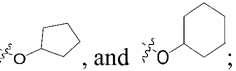
or most preferably, R² is 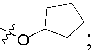
R³ is selected from the group consisting of hydroxy, and -O-C1-C6 alkyl, wherein the C1-C6 alkyl is optionally substituted with 5- to 6-membered heterocyclyl;
preferably, R³ is selected from the group consisting of hydroxy, and -O-C1-C6 alkyl, wherein the C1-C6 alkyl is optionally substituted with 6-membered heterocyclyl;
more preferably, R³ is selected from the group consisting of hydroxy, and -O-C1-C6 alkyl, wherein the C1-C6 alkyl is optionally substituted with morpholinyl;
further preferably, R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, -O(CH₂)₅CH₃, and 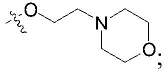
still further preferably, R³ is selected from the group consisting of -OCH₃, -OCH₂CH₃, -OCH(CH₃)CH₃, -O(CH₂)₂CH₃, and 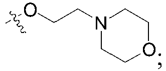
or preferably, R³ is selected from the group consisting of hydroxy, and -O-C1-C6 alkyl;
or more preferably, R³ is selected from the group consisting of hydroxy, -OCH₃, -OCH₂CH₃, -OCH(CH₃)CH₃, -O(CH₂)₂CH₃, -O(CH₂)₃CH₃, -O(CH₂)₄CH₃, and -O(CH₂)₅CH₃;
or further preferably, R³ is selected from the group consisting of hydroxy, -OCH₃, -OCH₂CH₃, -O(CH₂)₂CH₃, -O(CH₂)₃CH₃, -O(CH₂)₄CH₃, and -O(CH₂)₅CH₃;
or preferably, R³ is selected from the group consisting of -O-C1-C6 alkyl;
or more preferably, R³ is selected from the group consisting of -OCH₃, -OCH₂CH₃, -OCH(CH₃)CH₃, and -O(CH₂)₂CH₃;
or most preferably, R³ is selected from the group consisting of -OCH₃, and -OCH₂CH₃;
R⁴ is selected from the group consisting of hydroxy, cyano, nitro, carboxyl, mercapto, halogen, C1-C6 alkyl, -NR^{a}R^{b}, -CONR^{a}R^{b}, C6-C10 aryl, -O-C6-C10 aryl, -C(=O)-C1-C6 alkyl, -CH₂-C6-C10 aryl, C1-C6 alkoxy, C2-C6 alkenyl, C3-C8 cycloalkyl, -C(=O)O-C1-C6 alkyl, -C(=O)O-C6-C10 aryl, C1-C6 alkylthio, -S(=O)-C1-C6 alkyl, -S(=O)₂-C1-C6 alkyl, and 5- to 6-membered heteroaryl;
preferably, R⁴ is selected from the group consisting of hydroxy, cyano, nitro, carboxyl, mercapto, halogen, C1-C6 alkyl, -NR^{a}R^{b}, -CONR^{a}R^{b}, phenyl, phenoxy, -C(=O)-C1-C6 alkyl, benzyl, C1-C6 alkoxy, C2-C6 alkenyl, C3-C8 cycloalkyl, -C(=O)O-C1-C6 alkyl, -C(=O)O-phenyl, C1-C6 alkylthio, -S(=O)-C1-C6 alkyl, -S(=O)₂-C1-C6 alkyl, and thienyl (e.g., 2-thienyl);
R^{a}, and R^{b} are each independently selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
preferably, R^{a}, and R^{b} are each independently selected from the group consisting of hydrogen, methyl, 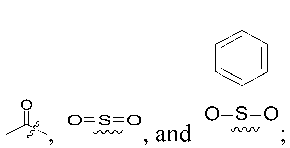
or preferably, one of R^{a} and R^{b} is selected from the group consisting of hydrogen, and C1-C6 alkyl, and the other is selected from the group consisting of hydrogen, C1-C6 alkyl, -C(=O)-C1-C6 alkyl, -S(=O)₂-C1-C6 alkyl, and -S(=O)₂-phenyl, wherein the phenyl is optionally substituted with C1-C6 alkyl;
or more preferably, one of R^{a} and R^{b} is selected from the group consisting of hydrogen, and methyl, and the other is selected from the group consisting of hydrogen, methyl, 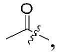 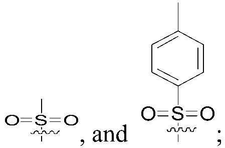
more preferably, R⁴ is selected from the group consisting of hydroxy, cyano, nitro, carboxyl, -NH₂, -SH, -CONH₂, methyl, phenyl, benzyl, methoxy, phenoxy, acetyl, -F, -Cl, -Br, -CH=CH₂, cyclopentyl, -COOMe, -COOPh, -SMe, -SOMe, -SO₂Me, -NMe₂, -NHAc, -NHMs, -NHTs, and 2-thienyl;
most preferably, R⁴ is hydroxy;
and, the compound represented by Formula I-2 does not include

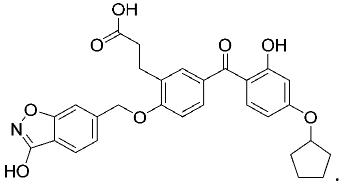

8. The use according to any one of claims 1 to 5, wherein the structural formula of the compound is as shown in Formula I-3, 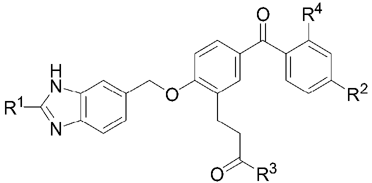 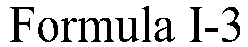 wherein:
R¹ is selected from the group consisting of hydrogen, and C1-C6 alkyl;
preferably, R¹ is hydrogen;
R² is selected from the group consisting of -O-C3-C8 cycloalkyl;
preferably, R² is 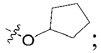
R³ is selected from the group consisting of -O-C 1-C6 alkyl;
preferably, R³ is -OCH₂CH₃;
R⁴ is hydroxy.

9. The use according to any one of claims 1 to 8, wherein the compound is selected from the group consisting of: 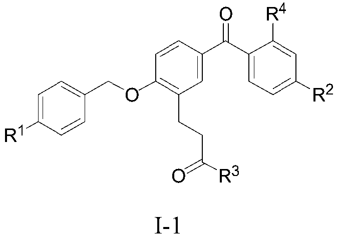
| | R¹ | R² | R³ | R⁴ |
|---|---|---|---|---|
| | -COOCH₃ | 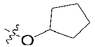 | -OH | -OH |
| | -COOCH₂CH₃ | 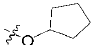 | -OH | -OH |
| | -COO(CH₂)₂CH₃ | 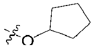 | -OH | -OH |
| | -COO(CH₂)₃CH₃ | 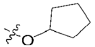 | -OH | -OH |
| | -COO(CH₂)₄CH₃ | 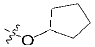 | -OH | -OH |
| | -COO(CH₂)₅CH₃ | 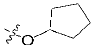 | -OH | -OH |
| | -COOH | 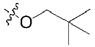 | -OH | -OH |
| | -COOH | 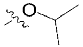 | -OH | -OH |
| | -COOH | 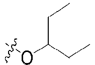 | -OH | -OH |
| | -COOH | 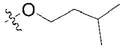 | -OH | -OH |
| **T1** | -COOH | 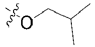 | -OH | -OH |
| | -COOH | 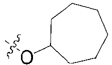 | -OH | -OH |
| | -COOH | 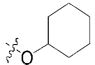 | -OH | -OH |
| | -COOH | 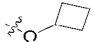 | -OH | -OH |
| | -COOH | 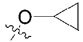 | -OH | -OH |
| **T4** | -COOH | 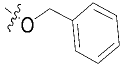 | -OH | -OH |
| | -COOH | 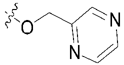 | -OH | -OH |
| | -COOH | 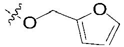 | -OH | -OH |
| | -COOH | 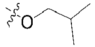 | -OCH₃ | -OH |
| | -COOH | 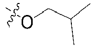 | -OCH₂CH₃ | -OH |
| | -COOH | 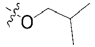 | -O(CH₂)₂CH₃ | -OH |
| | -COOH | 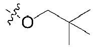 | -OCH₃ | -OH |
| | -COOH | 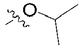 | -OCH₃ | -OH |
| | -COOH | 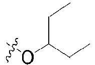 | -OCH₃ | -OH |
| | -COOH | 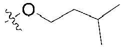 | -OCH₃ | -OH |
| | -COOH | 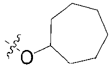 | -OCH₃ | -OH |
| | -COOH | 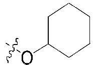 | -OCH₃ | -OH |
| **T12** | -COOH | 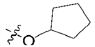 | -OCH₃ | -OH |
| | -COOH | 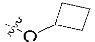 | -OCH₃ | -OH |
| | -COOH | 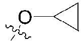 | -OCH₃ | -OH |
| | -COOH | 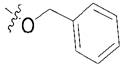 | -OCH₃ | -OH |
| | -COOH | 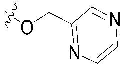 | -OCH₃ | -OH |
| | -COOH | 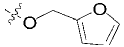 | -OCH₃ | -OH |
| | -COOH | 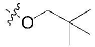 | -OCH₂CH₃ | -OH |
| | -COOH | 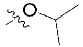 | -OCH₂CH₃ | -OH |
| | -COOH | 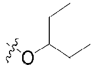 | -OCH₂CH₃ | -OH |
| | -COOH | 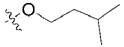 | -OCH₂CH₃ | -OH |
| | -COOH | 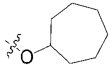 | -OCH₂CH₃ | -OH |
| | -COOH | 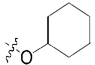 | -OCH₂CH₃ | -OH |
| | -COOH | 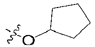 | -OCH₂CH₃ | -OH |
| | -COOH | 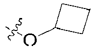 | -OCH₂CH₃ | -OH |
| | -COOH | 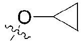 | -OCH₂CH₃ | -OH |
| | -COOH | 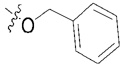 | -OCH₂CH₃ | -OH |
| | -COOH | 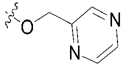 | -OCH₂CH₃ | -OH |
| | -COOH | 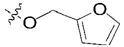 | -OCH₂CH₃ | -OH |
| | -COOCH₃ | 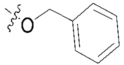 | -OH | -OH |
| | -COOCH₂CH₃ | 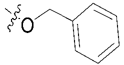 | -OH | -OH |
| | -COO(CH₂)₂CH₃ | 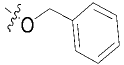 | -OH | -OH |
| | -COO(CH₂)₃CH₃ | 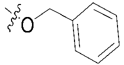 | -OH | -OH |
| | -COO(CH₂)₄CH₃ | 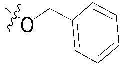 | -OH | -OH |
| | -COO(CH₂)₅CH₃ | 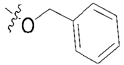 | -OH | -OH |
| | -COOCH₃ | 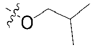 | -OH | -OH |
| **T2** | -COOCH₂CH₃ | 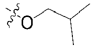 | -OH | -OH |
| | -COO(CH₂)₂CH₃ | 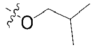 | -OH | -OH |
| | -COO(CH₂)₃CH₃ | 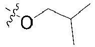 | -OH | -OH |
| | -COO(CH₂)₄CH₃ | 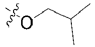 | -OH | -OH |
| | -COO(CH₂)₅CH₃ | 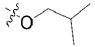 | -OH | -OH |
| | -COOH | 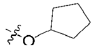 | -OCH₃ | -OH |
| **T13** | -COOCH₃ | 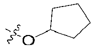 | -OCH₃ | -OH |
| | -COOCH₂CH₃ | 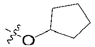 | -OCH₃ | -OH |
| | -COO(CH₂)₂CH₃ | 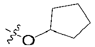 | -OCH₃ | -OH |
| | -COO(CH₂)₃CH₃ | 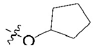 | -OCH₃ | -OH |
| | -COO(CH₂)₄CH₃ | 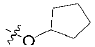 | -OCH₃ | -OH |
| | -COO(CH₂)₅CH₃ | 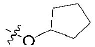 | -OCH₃ | -OH |
| | -COOH | 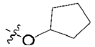 | -OCH₂CH₃ | -OH |
| | -COOCH₃ | 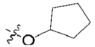 | -OCH₂CH₃ | -OH |
| | -COOCH₂CH₃ | 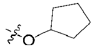 | -OCH₂CH₃ | -OH |
| | -COO(CH₂)₂CH₃ | 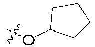 | -OCH₂CH₃ | -OH |
| | -COO(CH₂)₃CH₃ | 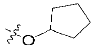 | -OCH₂CH₃ | -OH |
| | -COO(CH₂)₄CH₃ | 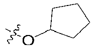 | -OCH₂CH₃ | -OH |
| | -COO(CH₂)₅CH₃ | 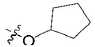 | -OCH₂CH₃ | -OH |
| | -COOH | 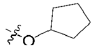 | -O(CH₂)₂CH₃ | -OH |
| | -COOCH₃ | 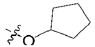 | -O(CH₂)₂CH₃ | -OH |
| | -COOCH₂CH₃ | 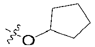 | -O(CH₂)₂CH₃ | -OH |
| | -COO(CH₂)₂CH₃ | 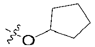 | -O(CH₂)₂CH₃ | -OH |
| | -COO(CH₂)₃CH₃ | 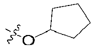 | -O(CH₂)₂CH₃ | -OH |
| | -COO(CH₂)₄CH₃ | 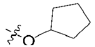 | -O(CH₂)₂CH₃ | -OH |
| | -COO(CH₂)₅CH₃ | 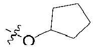 | -O(CH₂)₂CH₃ | -OH |
| | -COOH | 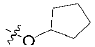 | -OH | -CN |
| | -COOH | 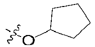 | -OH | -NO₂ |
| | -COOH | 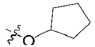 | -OH | -COOH |
| | -COOH | 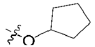 | -OH | -NH₂ |
| | -COOH | 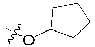 | -OH | -SH |
| | -COOH | 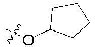 | -OH | -CONH₂ |
| | -COOH | 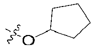 | -OH | -Me |
| | -COOH | 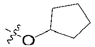 | -OH | -Ph |
| | -COOH | 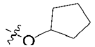 | -OH | -Bn |
| | -COOH | 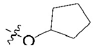 | -OH | -OMe |
| | -COOH | 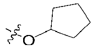 | -OH | -OPh |
| | -COOH | 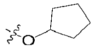 | -OH | -Ac |
| | -COOH | 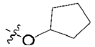 | -OH | -F |
| | -COOH | 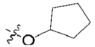 | -OH | -Cl |
| | -COOH | 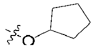 | -OH | -Br |
| | -COOH | 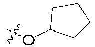 | -OH | -CH=CH2 |
| | -COOH | 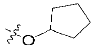 | -OH | 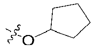 |
| | -COOH | 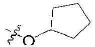 | -OH | -COOMe |
| | -COOH | 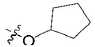 | -OH | -COOPh |
| | -COOH | 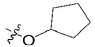 | -OH | -SMe |
| | -COOH | 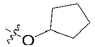 | -OH | -SOMe |
| | -COOH | 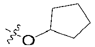 | -OH | -SO₂Me |
| | -COOH | 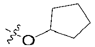 | -OH | -NMe₂ |
| | -COOH | 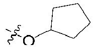 | -OH | -NHAc |
| | -COOH | 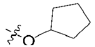 | -OH | -NHMs |
| | -COOH | 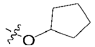 | -OH | -NHTs |
| | -COOH | 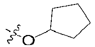 | -OH | 2-thienyl |
or, 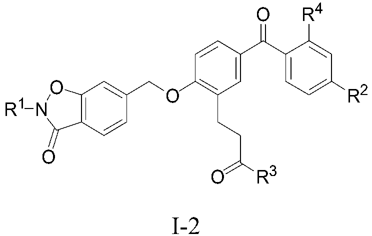
| | R¹ | R² | R³ | R⁴ |
|---|---|---|---|---|
| **T8** | H | 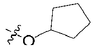 | -OCH₃ | -OH |
| **T9** | H | 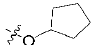 | -OCH(CH₃)CH₃ | -OH |
| **T16** | H | 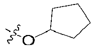 | -O(CH₂)₂CH₃ | -OH |
| | H | 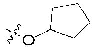 | -O(CH₂)₃CH₃ | -OH |
| | H | 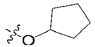 | -O(CH₂)₄CH₃ | -OH |
| | H | 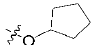 | -O(CH₂)₅CH₃ | -OH |
| | H | 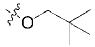 | -OH | -OH |
| | H | 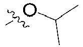 | -OH | -OH |
| | H | 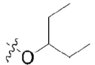 | -OH | -OH |
| | H | 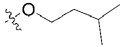 | -OH | -OH |
| | H | 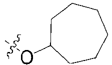 | -OH | -OH |
| | H | 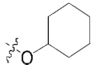 | -OH | -OH |
| | H | 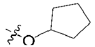 | -OH | -OH |
| | H | 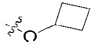 | -OH | -OH |
| | H | 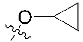 | -OH | -OH |
| | H | 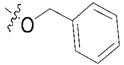 | -OH | -OH |
| | H | 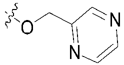 | -OH | -OH |
| | H | 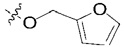 | -OH | -OH |
| | H | 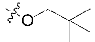 | -OCH₃ | -OH |
| | H | 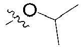 | -OCH₃ | -OH |
| | H | 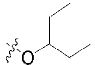 | -OCH₃ | -OH |
| | H | 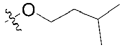 | -OCH₃ | -OH |
| | H | 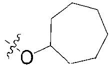 | -OCH₃ | -OH |
| **T10** | H | 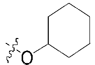 | -OCH₃ | -OH |
| | H | 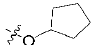 | -OCH₃ | -OH |
| | H | 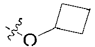 | -OCH₃ | -OH |
| | H | 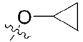 | -OCH₃ | -OH |
| | H | 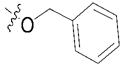 | -OCH₃ | -OH |
| | H | 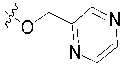 | -OCH₃ | -OH |
| | H | 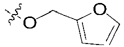 | -OCH₃ | -OH |
| | H | 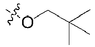 | -OCH₂CH₃ | -OH |
| | H | 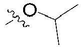 | -OCH₂CH₃ | -OH |
| | H | 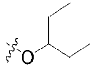 | -OCH₂CH₃ | -OH |
| | H | 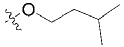 | -OCH₂CH₃ | -OH |
| | H | 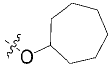 | -OCH₂CH₃ | -OH |
| **T11** | H | 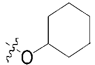 | -OCH₂CH₃ | -OH |
| | H | 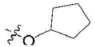 | -OCH₂CH₃ | -OH |
| | H | 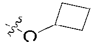 | -OCH₂CH₃ | -OH |
| | H | 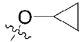 | -OCH₂CH₃ | -OH |
| | H | 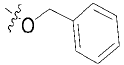 | -OCH₂CH₃ | -OH |
| | H | 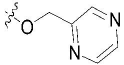 | -OCH₂CH₃ | -OH |
| | H | 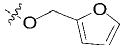 | -OCH₂CH₃ | -OH |
| | | 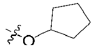 | -OH | -OH |
| **T6** | a | 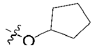 | -OCH₃ | -OH |
| | | 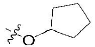 | -OCH₂CH₃ | -OH |
| | H | 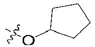 | -OH | -CN |
| | H | 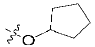 | -OH | -NO₂ |
| | H | 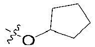 | -OH | -COOH |
| | H | 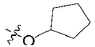 | -OH | -NH₂ |
| | H | 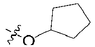 | -OH | -SH |
| | H | 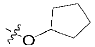 | -OH | -CONH₂ |
| | H | 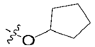 | -OH | -Me |
| | H | 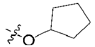 | -OH | -Ph |
| | H | 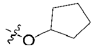 | -OH | -Bn |
| | H | 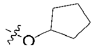 | -OH | -OMe |
| | H | 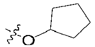 | -OH | -OPh |
| | H | 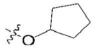 | -OH | -Ac |
| | H | 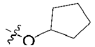 | -OH | -F |
| | H | 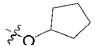 | -OH | -Cl |
| | H | 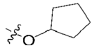 | -OH | -Br |
| | H | 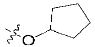 | -OH | -CH=CH₂ |
| | H | 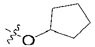 | -OH | 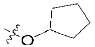 |
| | H | 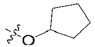 | -OH | -COOMe |
| | H | 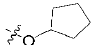 | -OH | -COOPh |
| | H | 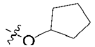 | -OH | -SMe |
| | H | 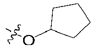 | -OH | -SOMe |
| | H | 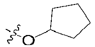 | -OH | -SO₂Me |
| | H | 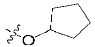 | -OH | -NMe₂ |
| | H | 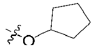 | -OH | -NHAc |
| | H | 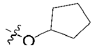 | -OH | -NHMs |
| | H | 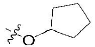 | -OH | -NHTs |
| | H | 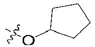 | -OH | 2-thienyl |
| **T14** | H | 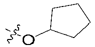 | 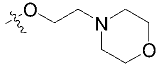 | -OH |
or,

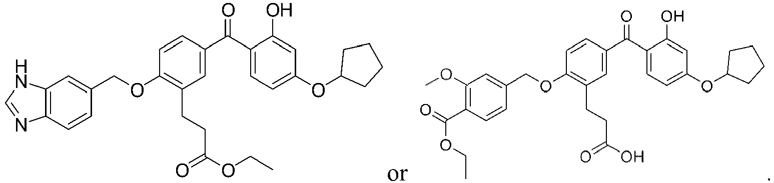

10. Use of a pharmaceutical composition in the manufacture of a medicament for treating and/or preventing heart failure with preserved ejection fraction, wherein the pharmaceutical composition comprises a compound represented by Formula I, or a stereoisomer, prodrug, crystal form, pharmaceutically acceptable salt, pharmaceutically acceptable ester, or pharmaceutically acceptable solvate thereof, 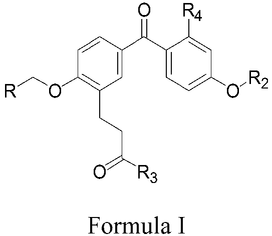
wherein, R, R², R³, R⁴ are each independently as defined in any one of claims 1 to 5;
preferably, the structural formula of the compound is as shown in Formula I-1, Formula I-1', Formula I-2 or Formula I-3, wherein the compound represented by Formula I-1 or Formula I-1' is as defined in claim 6, the compound represented by Formula I-2 is as defined in claim 7, and the compound represented by Formula I-3 is as defined in claim 8;
more preferably, the structural formula of the compound is as shown in Formula I-1, Formula I-2 or Formula I-3, wherein the compound represented by Formula I-1 is as defined in claim 6, the compound represented by Formula I-2 is as defined in claim 7, and the compound represented by Formula I-3 is as defined in claim 8;
most preferably, the compound is as defined in claim 9.
